(19) Europäisches Patentamt
European Patent Office
Office européen des brevets

(11) EP 4 339 187 A1

(12) # EUROPEAN PATENT APPLICATION
published in accordance with Art. 153(4) EPC

(43) Date of publication:
**20.03.2024 Bulletin 2024/12**

(21) Application number: **22807497.7**

(22) Date of filing: **11.05.2022**

(51) International Patent Classification (IPC):
***C07D 213/85*** (2006.01) ***A01N 37/34*** (2006.01)
***A01N 41/10*** (2006.01) ***A01N 43/40*** (2006.01)
***A01N 43/80*** (2006.01) ***A01N 43/836*** (2006.01)
***A01N 47/02*** (2006.01) ***A01P 7/02*** (2006.01)
***A01P 7/04*** (2006.01) ***C07C 317/28*** (2006.01)
***C07C 323/25*** (2006.01) ***C07D 213/83*** (2006.01)
***C07D 401/12*** (2006.01) ***C07D 413/12*** (2006.01)
***C07D 417/12*** (2006.01)

(52) Cooperative Patent Classification (CPC):
**A01N 37/34; A01N 41/10; A01N 43/40; A01N 43/80; A01N 43/82; A01N 47/02; A01P 7/02; A01P 7/04; C07C 317/28; C07C 323/25; C07D 213/83; C07D 213/85; C07D 401/12; C07D 413/12; C07D 417/12**

(86) International application number:
**PCT/JP2022/019936**

(87) International publication number:
**WO 2022/239798 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.05.2021 JP 2021082497**

(71) Applicant: **Nippon Soda Co., Ltd.**
**Tokyo 100-7010 (JP)**

(72) Inventors:
- **MAKINO Satoshi**
**Odawara-shi, Kanagawa 250-0280 (JP)**
- **NISHIO Fumiya**
**Odawara-shi, Kanagawa 250-0280 (JP)**
- **FURUKAWA Hironori**
**Odawara-shi, Kanagawa 250-0280 (JP)**
- **KAWAGUCHI Masahiro**
**Odawara-shi, Kanagawa 250-0280 (JP)**

(74) Representative: **Mewburn Ellis LLP**
**Aurora Building**
**Counterslip**
**Bristol BS1 6BX (GB)**

(54) **(HETERO)ARYLAMIDE COMPOUND AND PEST CONTROL AGENT**

(57) A compound represented by Formula (I) or a salt thereof. In Formula (I), Ar represents a substituted or unsubstituted C6 to C10 aryl group or the like, $L^1$ represents a single bond or the like, $L^2$ represents a single bond or the like, $X^1$ represents a cyano group or the like, $X^2$ represents a hydrogen atom or the like, $X^3$ represents a halogeno group or the like, A represents a nitrogen atom or the like, $R^1$ represents a hydrogen atom or the like, $R^{2a}$ represents a substituted or unsubstituted C1 to C6 alkyl group and $R^{2b}$ represents a hydrogen atom or the like, where $R^{2a}$ and $R^{2b}$ may be combined with each other to form a substituted or unsubstituted C2 to C5 alkylene group, and Y represents a substituted or unsubstituted C1 to C6 alkyl group or the like.

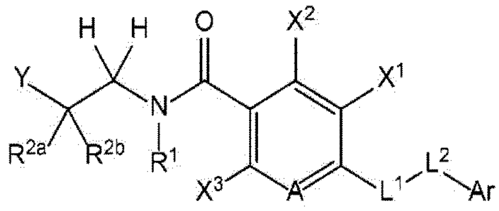

(I)

EP 4 339 187 A1

## Description

[Technical Field]

The present invention relates to a (hetero)arylamide compound and a pest control agent. More specifically, the present invention relates to a (hetero)arylamide compound which has excellent insecticidal activity and/or acaricidal activity, and excellent safety, and can be synthesized industrially advantageously; and a pest control agent containing the (hetero)arylamide compound as an active ingredient.

Priority is claimed on Japanese Patent Application No. 2021-082497, filed May 14, 2021, the content of which is incorporated herein by reference.

[Background Art]

Patent Document 1 discloses a compound represented by Formula (A) or (B) as a compound which is structurally related to the (hetero)arylamide compound of the present invention. These compounds have been shown to be used for an insecticidal or acaricidal agent.

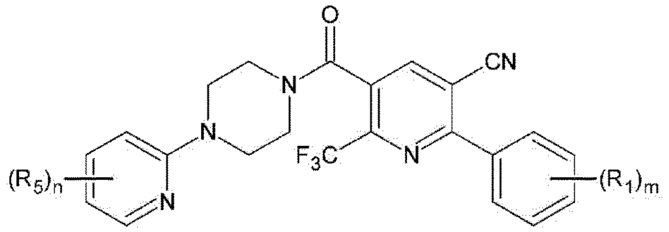

(A)

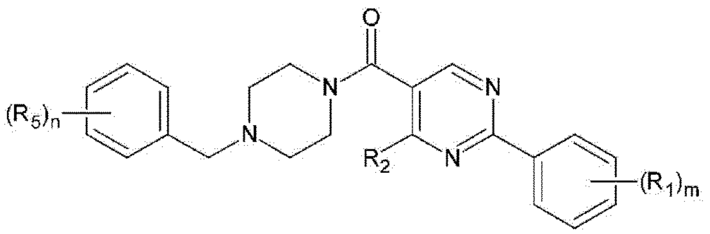

(B)

[Citation List]

[Patent Document]

[Patent Document 1]
PCT International Publication No. WO 2015/032280

[Summary of Invention]

[Technical Problem]

An object of the present invention is to provide a (hetero)arylamide compound having an excellent pest control activity, in particular, excellent insecticidal activity and/or acaricidal activity, and excellent safety, and can be synthesized industrially advantageously; and a pest control agent containing the (hetero)arylamide compound as an active ingredient.

[Solution to Problem]

As a result of studies to solve the problems, the present invention including the following aspects has been completed.

(1) A compound represented by Formula (I) or a salt thereof.

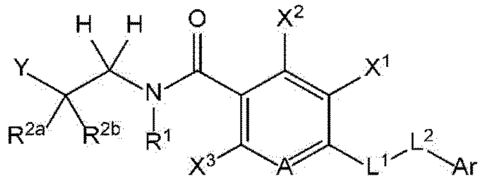

(I)

In Formula (I),

Ar represents a substituted or unsubstituted C6 to C10 aryl group, or a substituted or unsubstituted 5- or 6-membered heteroaryl group,
$L^1$ represents a single bond, an oxygen atom, or a sulfur atom,
$L^2$ represents a single bond, or a substituted or unsubstituted C1 or C2 alkylene group,
$X^1$ represents a cyano group, or a substituted or unsubstituted thiocarbamoyl group,
$X^2$ represents a hydrogen atom, a halogeno group, a substituted or unsubstituted C1 to C6 alkyl group, a hydroxyl group, or a substituted or unsubstituted C1 to C6 alkoxy group,
$X^3$ represents a halogeno group, a substituted or unsubstituted C1 to C6 alkyl group, a substituted or unsubstituted C1 to C6 alkoxy group, or a cyano group,
A represents a nitrogen atom or $CX^4$,
$X^4$ represents a hydrogen atom, a halogeno group, a substituted or unsubstituted C1 to C6 alkyl group, a substituted or unsubstituted C1 to C6 alkoxy group, or a cyano group,
$R^1$ represents a hydrogen atom, or a substituted or unsubstituted C1 to C6 alkyl group,
$R^{2a}$ represents a substituted or unsubstituted C1 to C6 alkyl group,
$R^{2b}$ represents a hydrogen atom, or a substituted or unsubstituted C1 to C6 alkyl group,
where $R^{2a}$ and $R^{2b}$ may be combined with each other to form a substituted or unsubstituted C2 to C5 alkylene group, and
Y represents a substituted or unsubstituted C1 to C6 alkyl group, a substituted or unsubstituted C6 to C10 aryl group, or a substituted or unsubstituted 5- or 6-membered heteroaryl group.

(2) A pest control agent containing at least one selected from the group consisting of the compound according to (1) and a salt thereof as an active ingredient.
(3) An insecticidal or acaricidal agent containing at least one selected from the group consisting of the compound according to (1) and a salt thereof as an active ingredient.
(4) An ectoparasite control or repellent agent containing at least one selected from the group consisting of the compound according to (1) and a salt thereof as an active ingredient.

[Advantageous Effects of Invention]

The (hetero)arylamide compound of the present invention can control pests that cause problems in terms of agricultural crops and hygiene. In particular, the (hetero)arylamide compound can effectively control agricultural pests and acari at a lower concentration. Furthermore, the (hetero)arylamide compound can effectively control ectoparasites that harm humans and livestock.

[Description of Embodiments]

[(Hetero)arylamide Compound]

The (hetero)arylamide compound of the present invention is a compound represented by Formula (I) (hereinafter sometimes referred to as a compound (I)) or a salt of the compound (I).

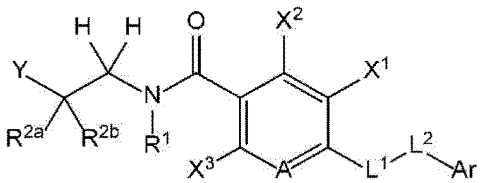

(I)

In the present invention, a term "unsubstituted" means only a group that serves as a mother nucleus. In a case where the group that serves as a mother nucleus is described only with the name without a description of "substituted", it means an "unsubstituted" group unless otherwise specified.

On the other hand, the term "substituted" means that any hydrogen atom in the group serving as a mother nucleus is substituted with a group (a substituent) having the same structure or a different structure as that of the mother nucleus. Accordingly, the "substituent" is another group bonded to the group serving as a mother nucleus. The number of the substituents may be one or two or more. Two or more substituents may be the same as or different from each other.

The term "Cl to C6" or the like indicates that the number of carbon atoms in the group serving as a mother nucleus is 1 to 6. The number of carbon atoms in the substituent is not included in the number of carbon atoms. For example, an ethoxybutyl group is classified into a C2 alkoxy C4 alkyl group since the group serving as a mother nucleus is a butyl group and the substituent is an ethoxy group.

The "substituent" is chemically acceptable and is not particularly limited as long as it has the effect of the present invention.

Specific examples of the group that can be the "substituent" include the following groups.

A halogeno group such as a fluoro group, a chloro group, a bromo group, and an iodo group;

a C1 to C6 alkyl group such as a methyl group, an ethyl group, an n-propyl group, an i-propyl group, an n-butyl group, an s-butyl group, an i-butyl group, a t-butyl group, an n-pentyl group, and an n-hexyl group;
a C2 to C6 alkenyl group such as a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group;
a C2 to C6 alkynyl group such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group;
a C3 to 8 cycloalkyl group such as a cyclopropyl group, a cyclobutyl group, a cyclopentyl group, a cyclohexyl group, a cycloheptyl group, and a cubanyl group;
a C3 to 8 cycloalkenyl group such as a 2-cyclopropenyl group, a 2-cyclopentenyl group, a 3-cyclohexenyl group, and a 4-cyclooctenyl group;
a C6 to C10 aryl group such as a phenyl group and a naphthyl group;
a 3- to 6-membered heterocyclyl group;
a hydroxyl group; an oxo group;
a C1 to C6 alkoxy group such as a methoxy group, an ethoxy group, an n-propoxy group, an i-propoxy group, an n-butoxy group, an s-butoxy group, an i-butoxy group, and a t-butoxy group;
a C2 to C6 alkenyloxy group such as a vinyloxy group and an allyloxy group;
a C2 to C6 alkynyloxy group such as an ethynyloxy group and a propargyloxy group;
a C6 to C10 aryloxy group such as a phenoxy group and a naphthoxy group;
a 3- to 6-membered heterocyclyloxy group;
a hydroxyC1 to 6 alkyl group such as a hydroxymethyl group and a hydroxyethyl group;
a C1 to C6 alkoxyalkyl group such as a methoxymethyl group and an ethoxymethyl group;
a C1 to C6 alkoxyC1 to C6 alkoxy group such as a methoxymethoxy group and an ethoxymethoxy group;
a carboxyl group;
a formyl group; a C1 to C6 alkylcarbonyl group such as an acetyl group and a propionyl group; a formyloxy group;
a C1 to C6 alkylcarbonyloxy group such as an acetyloxy group, a propionyloxy group, an n-propylcarbonyloxy group, and an i-propylcarbonyloxy group;
a C1 to C6 alkoxycarbonyl group such as a methoxycarbonyl group, an ethoxycarbonyl group, an n-propoxycarbonyl group, an i-propoxycarbonyl group, an n-butoxycarbonyl group, and a t-butoxycarbonyl group;
a C6 to C10 arylcarbonyl group such as benzoyl;
a C1 to C6 haloalkyl group such as a chloromethyl group, a chloroethyl group, a difluoromethyl group, a trifluoromethyl group, a 2,2,2-trifluoroethyl group, a perfluoroethyl group, a perfluoropropan-2-yl group, a 1-fluoro-n-butyl group, and a perfluoro-n-pentyl group;
a C1 to C6 alkoxyC1 to C6 haloalkyl group such as a 1,1,1,3,3,3-hexafluoro-2-methoxypropan-2-yl group;
a C2 to C6 haloalkenyl group such as a 2-chloro-1-propenyl group and a 2-fluoro-1-butenyl group;
a C2 to C6 haloalkynyl group such as a 4,4-dichloro-1-butynyl group, a 4-fluoro-1-pentynyl group, and a 5-bromo-2-pentynyl group;
a C3 to C6 halocycloalkyl group such as a 1,1-difluorocyclopropyl group and a 3,3-difluorocyclobutyl group;
a C1 to C6 haloalkoxy group such as a difluoromethoxy group, a trifluoromethoxy group, a 2,2,2-trifluoroethoxy

group, a 1,1,2,2-tetrafluoroethoxy group, a 2,2,3,3,3-pentafluoropropoxy group, a 4,4,4-trifluorobutoxy group, and a (1,1,1,3,3,3-hexafluoropropan-2-yl)oxy group;
a C2 to C6 haloalkenyloxy group such as a 2-chloropropenyloxy group and a 3-bromobutenyloxy group;
a C1 to C6 haloalkylcarbonyl group such as a chloroacetyl group, a trifluoroacetyl group, and a trichloroacetyl group;
a cyano group; a nitro group; an amino group;
a C1 to C6 alkylamino group such as a methylamino group, a dimethylamino group, and a diethylamino group;
a C6 to C10 arylamino group such as an anilino group and a naphthylamino group;
a formylamino group; a C1 to C6 alkylcarbonylamino group such as an acetylamino group, a propanoylamino group, a butyrylamino group, and an i-propylcarbonylamino group;
a C1 to C6 alkoxycarbonylamino group such as a methoxycarbonylamino group, an ethoxycarbonylamino group, an n-propoxycarbonylamino group, and an i-propoxycarbonylamino group;
a C1 to C6 alkylsulfoximino group such as an S,S-dimethylsulfoximino group;
a carbamoyl group; an N'-hydroxycarbamimidoyl group;
a C1 to C6 alkylaminocarbonyl group such as a methylaminocarbonyl group, a dimethylaminocarbonyl group, an ethylaminocarbonyl group, and an i-propylaminocarbonyl group;
an iminoC1 to C6 alkyl group such as an iminomethyl group, a (1-imino) ethyl group, and a (1-imino)-n-propyl group;
a hydroxyiminoC1 to C6 alkyl group such as a hydroxyiminomethyl group, a (1-hydroxyimino)ethyl group, and a (1-hydroxyimino)propyl group;
a (C1 to C6 alkoxyimino)C1 to C6 alkyl group such as a methoxyiminomethyl group and a (1-methoxyimino)ethyl group;
a (C1 to C6 alkylcarbonyloxyimino)C1 to C6 alkyl group such as an acetoxyiminomethyl group;
a mercapto group;
a C1 to C6 alkylthio group such as a methylthio group, an ethylthio group, an n-propylthio group, an i-propylthio group, an n-butylthio group, an i-butylthio group, an s-butylthio group, and a t-butylthio group;
a C1 to C6 haloalkylthio group such as a trifluoromethylthio group and a 2,2,2-trifluoroethylthio group;
a C2 to C6 alkenylthio group such as a vinylthio group and an allylthio group;
a C2 to C6 alkynylthio group such as an ethynylthio group and a propargylthio group;
a C1 to C6 alkylsulfinyl group such as a methylsulfinyl group, an ethylsulfinyl group, and a t-butylsulfinyl group;
a C1 to C6 haloalkylsulfinyl group such as a trifluoromethylsulfinyl group and a 2,2,2-trifluoroethylsulfinyl group;
a C2 to C6 alkenylsulfinyl group such as an allylsulfinyl group;
a C2 to C6 alkynylsulfinyl group such as a propargylsulfinyl group;
a C1 to C6 alkylsulfonyl group such as a methylsulfonyl group, an ethylsulfonyl group, and a t-butylsulfonyl group;
a C1 to C6 haloalkylsulfonyl group such as a trifluoromethylsulfonyl group and a 2,2,2-trifluoroethylsulfonyl group;
a C2 to C6 alkenylsulfonyl group such as an allylsulfonyl group;
a C2 to C6 alkynylsulfonyl group such as a propargylsulfonyl group;
a thiocarbamoyl group;
an imino(Cl to C6 alkylthio)methyl group such as an imino(methylthio)methyl group;
a pentafluorosulfanyl group;
a triC1 to C6 alkylsilyl group such as a trimethylsilyl group, a triethylsilyl group, and a t-butyldimethylsilyl group; and
a triC6 to C10 arylsilyl group such as a triphenylsilyl group.

In addition, in these "substituents", any hydrogen atom in the substituent may be substituted with a group having a different structure.

Moreover, the "3- to 6-membered heterocyclyl group" is a group generated by removing one hydrogen atom from any ring atom of a 3- to 6-membered cyclyl compound including 1 to 4 hetero atoms selected from the group consisting of a nitrogen atom, an oxygen atom, and a sulfur atom as constituent atoms of the ring (which may also be referred to as ring member atoms), that is, a "3- to 6-membered heterocyclyl compound".

The "3- to 6-membered heterocyclyl group" may also be expressed as "a 3- to 6-membered heterocyclyl group including 1 to 4 heteroatoms selected from the group consisting of a nitrogen atom, an oxygen atom, and a sulfur atom as ring member atoms".

The heterocyclyl group may be either a monocycle or a polycycle. As long as the polycyclic heterocyclyl group includes a heterocyclic ring as at least one ring, the remaining rings may be any of a saturated alicyclic ring, an unsaturated alicyclic ring, or an aromatic ring. Examples of the "3- to 6-membered heterocyclyl group" include a 3-to 6-membered saturated heterocyclyl group, a 5- or 6-membered heteroaryl group, and a 5- or 6-membered partially unsaturated heterocyclyl group.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

[Ar]

In Formula (I), Ar represents a substituted or unsubstituted C6 to C10 aryl group, or a substituted or unsubstituted 5- or 6-membered heteroaryl group.

The "C6 to C10 aryl group" in Ar is a group formed by removing one hydrogen on the ring of the monocyclic or polycyclic aromatic hydrocarbon. Examples of the C6 to C10 aryl group include a phenyl group and a naphthyl group, and the phenyl group is preferable.

In addition, the "5- or 6-membered heteroaryl group" of Ar is a group generated by removing one hydrogen atom from any ring atom of a 5- or 6-membered cyclyl aromatic compound including 1 to 4 hetero atoms selected from the group consisting of a nitrogen atom, an oxygen atom, and a sulfur atom as constituent atoms of the ring (which may also be referred to as ring member atoms), that is, a "5- or 6-membered heteroaryl compound". The "5- or 6-membered heteroaryl group" may also be expressed as "a 5- or 6-membered heteroaryl group including 1 to 4 heteroatoms selected from the group consisting of a nitrogen atom, an oxygen atom, and a sulfur atom as ring member atoms".

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

Among these, the pyrazolyl group, the pyridyl group, or the pyridazinyl group is preferable.

Ar in Formula (I) is preferably a substituted or unsubstituted C6 to C10 aryl group.

Examples of the substituent (which may be referred to as $X^5$) on the "C6 to C10 aryl group" or the "5- or 6-membered heteroaryl group" in Ar include a halogeno group, a substituted or unsubstituted C1 to C6 alkyl group, a substituted or unsubstituted C2 to C6 alkenyl group, a substituted or unsubstituted C2 to C6 alkynyl group, a substituted or unsubstituted C1 to C6 alkoxy group, a substituted or unsubstituted C2 to C6 alkenyloxy group, a formyl group, a substituted or unsubstituted C1 to C6 alkylcarbonyl group, a substituted or unsubstituted C1 to C6 alkoxycarbonyl group, a substituted or unsubstituted C1 to C6 alkylthio group, a substituted or unsubstituted C1 to C6 alkylsulfinyl group, a substituted or unsubstituted C1 to C6 alkylsulfonyl group, a substituted or unsubstituted C3 to 8 cycloalkyl group, a substituted or unsubstituted C6 to C10 aryl group, a substituted or unsubstituted 5- or 6-membered heteroaryl group, a substituted or unsubstituted C6 to C10 aryloxy group, a substituted or unsubstituted 5- or 6-membered heteroaryloxy group, a group represented by $R^aR^bN$-CO-, a group represented by $R^cCONR^d$-, a group represented by $R^cON=C(R^d)$-, a hydroxyl group, a nitro group, or a cyano group.

Examples of the "halogeno group", which is one of $X^5$'s, include a fluoro group, a chloro group, a bromo group, and an iodo group.

The "Cl to C6 alkyl group", which is one of $X^5$'s, may be linear or branched. Examples of the alkyl group include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, and an i-hexyl group.

Examples of the "C2 to C6 alkenyl group", which is one of $X^5$'s, include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, 1-hexenyl group, a 2-hexenyl group, a 3-hexenyl group, a 4-hexenyl group, and 5-hexenyl group.

Examples of the "C2 to C6 alkynyl group", which is one of $X^5$'s, include an ethynyl group, a 1-propynyl group, a 2-propynyl group, a 1-butynyl group, a 2-butynyl group, a 3-butynyl group, a 1-methyl-2 -propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, 1-hexynyl group, and a 1,1-dimethyl-2-butynyl group.

Examples of the "Cl to C6 alkoxy group", which is one of $X^5$'s, include a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group, and an i-hexyloxy group.

Examples of the "C2 to C6 alkenyloxy group", which is one of $X^5$'s, include a vinyloxy group, an allyloxy group, a propenyloxy group, and a butenyloxy group.

Examples of the "Cl to C6 alkylcarbonyl group", which is one of $X^5$'s, include an acetyl group and a propionyl

group.

Examples of the "Cl to C6 alkoxycarbonyl group", which is one of $X^5$'s, include a methoxycarbonyl group, an ethoxycarbonyl group, an n-propoxycarbonyl group, an i-propoxycarbonyl group, and a t-butoxycarbonyl group.

Examples of the "Cl to C6 alkylthio group", which is one of $X^5$'s, include a methylthio group, an ethylthio group, an n-propylthio group, an n-butylthio group, an n-pentylthio group, an n-hexylthio group, and an i-propylthio group.

Examples of the "Cl to C6 alkylsulfinyl group", which is one of $X^5$'s, include a methylsulfinyl group, an ethylsulfinyl group, and a t-butylsulfinyl group.

Examples of the "Cl to C6 alkylsulfonyl group", which is one of $X^5$'s, include a methylsulfonyl group, an ethylsulfonyl group, and a t-butylsulfonyl group.

Preferred examples of the substituent on the "Cl to C6 alkyl group", the "C2 to C6 alkenyl group", the "C2 to C6 alkynyl group", the "Cl to C6 alkoxy group", the "C2 to C6 alkenyloxy group", the "Cl to C6 alkylcarbonyl group", the "Cl to C6 alkoxycarbonyl group", the "Cl to C6 alkylthio group", the "Cl to C6 alkylsulfinyl group", or the "Cl to C6 alkylsulfonyl group", which is $X^5$, include a halogeno group such as a fluoro group, a chloro group, a bromo group, and an iodo group; a C1 to C6 alkoxy group such as a methoxy group, an ethoxy group, an n-propoxy group, an i-propoxy group, an n-butoxy group, an s-butoxy group, an i-butoxy group, and a t-butoxy group; a C1 to C6 haloalkoxy group such as a 2-chloro-n-propoxy group, a 2,3-dichlorobutoxy group, and a trifluoromethoxy group; a C6 to C10 aryl group such as a phenyl group and a naphthyl group; and a cyano group.

Examples of the "C3 to 8 cycloalkyl group", which is one of the substituents on the "C6 to C10 aryl group" or the "5- or 6-membered heteroaryl group", include a cyclopropyl group, a cyclobutyl group, a cyclopentyl group, a cyclohexyl group, and a cycloheptyl group.

Examples of the "C6 to C10 aryl group", which is one of $X^5$'s, include a phenyl group and a naphthyl group.

Examples of the "5- or 6-membered heteroaryl group", which is one of $X^5$'s, include a 5-membered heteroaryl group such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group; and a 6-membered heteroaryl group such as a pyridyl group, a pyrazinyl group, a pyrimidinyl group, a pyridazinyl group, and a triazinyl group.

Examples of the "C6 to C10 aryloxy group", which is one of $X^5$'s, include a phenoxy group and a naphthoxy group.

Examples of the "5- or 6-membered heteroaryloxy group", which is one of $X^5$'s, include a 5- or 6-membered heteroaryloxy group such as a thiazolyloxy group and a pyridyloxy group.

Preferred examples of a substituent on the "C3 to 8 cycloalkyl group", the "C6 to C10 aryl group", the "5- or 6-membered heteroaryl group", the "C6 to C10 aryloxy group", or the "5- or 6-membered heteroaryloxy group", which is $X^5$, include a halogeno group such as a fluoro group, a chloro group, a bromo group, and an iodo group; a C1 to C6 alkyl group such as a methyl group, an ethyl group, an n-propyl group, an i-propyl group, an n-butyl group, an s-butyl group, an i-butyl group, a t-butyl group, an n-pentyl group, and an n-hexyl group; a C1 to C6 haloalkyl group such as a chloromethyl group, a chloroethyl group, a trifluoromethyl group, a 1,2-dichloro-n-propyl group, a 1-fluoro-n-butyl group, and a perfluoro-n-pentyl group; a C1 to C6 alkoxy group such as a methoxy group, an ethoxy group, an n-propoxy group, an i-propoxy group, an n-butoxy group, an s-butoxy group, an i-butoxy group, and a t-butoxy group; a C1 to C6 haloalkoxy group such as a 2-chloro-n-propoxy group, a 2,3-dichlorobutoxy group, and a trifluoromethoxy group; and a cyano group.

In the "group represented by $R^aR^bN$-CO-", which is one of $X^5$'s, $R^a$ represents a hydrogen atom, a C1 to C6 alkyl group, or a C1 to C6 alkoxy group, $R^b$ represents a hydrogen atom or a C1 to C6 alkyl group, and $R^a$ and $R^b$ may be combined with each other to form a C3 to C6 alkylene group.

Examples of the "Cl to C6 alkyl group" in $R^a$ and $R^b$ include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, and an i-hexyl group.

Examples of the "Cl to C6 alkoxy group" include a methoxy group, an ethoxy group, an n-propoxy group, an i-propoxy group, an n-butoxy group, an s-butoxy group, an i-butoxy group, and a t-butoxy group.

Examples of the "C3 to C6 alkylene group" formed by the combination of $R^a$ and $R^b$ include a trimethylene group, a tetramethylene group, and a pentamethylene group.

Examples of the group represented by $R^aR^bN$-CO- include a carbamoyl group, an N-methylaminocarbonyl group, an N,N-dimethylaminocarbonyl group, an N-ethyl-N-methylaminocarbonyl group, an N-methoxy-N-methylaminocarbonyl group, and a pyrrolidine-1-carbonyl group.

In the "group represented by $R^cCONR^d$-", which is one of $X^5$'s, $R^c$ represents a C1 to C6 alkyl group, and $R^d$ represents a hydrogen atom or a C1 to C6 alkyl group.

Examples of the "Cl to C6 alkyl group" in $R^c$ and $R^d$ include the same groups as those provided as exemplary examples of $R^a$ and $R^b$.

Examples of the group represented by $R^cCONR^d$- include an acetamide group and an N-methylacetamide group.

In the "group represented by $R^cON=C(R^d)$-", which is one of $X^5$'s, $R^c$ represents a C1 to C6 alkyl group. $R^d$ represents a hydrogen atom or a C1 to C6 alkyl group, and preferably represents the hydrogen atom.

Examples of the "Cl to C6 alkyl group" in $R^c$ and $R^d$ include the same groups as those provided as exemplary examples of $R^a$ and $R^b$.
Among these, as $X^5$, the halogeno group, the substituted or unsubstituted C1 to C6 alkyl group (more preferably the C1 to C6 alkyl group which may be substituted with a halogeno group), the substituted or unsubstituted C1 to C6 alkoxy group (more preferably the C1 to C6 alkoxy group which may be substituted with a halogeno group), the substituted or unsubstituted C1 to C6 alkylthio group (more preferably the C1 to C6 alkylthio group which may be substituted with a halogeno group), the group represented by $R^cON=C(R^d)$-, or the cyano group is preferable, and more preferred examples thereof include the halogeno group, the C1-6 alkyl group which may be substituted with a halogeno group, the C1-6 alkoxy group which may be substituted with a halogeno group, or the group represented by $R^cON=C(R^d)$-.

[$L^1$ and $L^2$]

$L^1$ in Formula (I) represents a single bond, an oxygen atom, or a sulfur atom.
$L^2$ in Formula (I) represents a single bond, or a substituted or unsubstituted C1 or C2 alkylene group.
In Formula (I), in a case where $L^1$ is the single bond and $L^2$ is the substituted or unsubstituted C1 or C2 alkylene group, $L^2$ is directly bonded to an aromatic ring including A.
In Formula (I), in a case where $L^1$ is the oxygen atom or the sulfur atom and $L^2$ is the single bond, Ar is directly bonded to $L^1$.
In Formula (I), in a case where both $L^1$ and $L^2$ are the single bonds, Ar is directly bonded to an aromatic ring including A.
Examples of the "Cl or C2 alkylene group" in $L^2$ include a methylene group and an ethylene group.
Examples of the substituent on the "Cl or C2 alkylene group" in $L^2$ include a halogeno group such as a fluoro group, a chloro group, a bromo group, and an iodo group; a C1 to C6 alkyl group such as a methyl group, an ethyl group, an n-propyl group, an i-propyl group, an n-butyl group, an s-butyl group, an i-butyl group, a t-butyl group, an n-pentyl group, and an n-hexyl group.
$L^1$ in Formula (I) is preferably the single bond or the oxygen atom, and more preferably the single bond.
$L^2$ in Formula (I) is preferably the single bond or the unsubstituted C1 or C2 alkylene group, and more preferably the single bond.

[$X^1$, $X^2$, and $X^3$]

$X^1$ in Formula (I) represents a cyano group, or a substituted or unsubstituted thiocarbamoyl group. $X^2$ in Formula (I) represents a hydrogen atom, a halogeno group, a substituted or unsubstituted C1 to C6 alkyl group, a hydroxyl group, or a substituted or unsubstituted C1 to C6 alkoxy group.
$X^3$ in Formula (I) represents a halogeno group, a substituted or unsubstituted C1 to C6 alkyl group, a substituted or unsubstituted C1 to C6 alkoxy group, or a cyano group.
Examples of the "substituted thiocarbamoyl group" in $X^1$ include a mono- or di-C1 to C6 alkyl-substituted thiocarbamoyl group such as a methylthiocarbamoyl group, a dimethylthiocarbamoyl group, and an ethyl(methyl)thiocarbamoyl group.
Examples of the "halogeno group", the "substituted or unsubstituted C1 to C6 alkyl group", and the "substituted or unsubstituted C1 to C6 alkoxy group" in $X^2$ and $X^3$ include the same groups as those provided as exemplary examples of Ar.
$X^1$ in Formula (I) is preferably a cyano group or an unsubstituted thiocarbamoyl group, and more preferably the cyano group.
$X^2$ in Formula (I) is preferably a hydrogen atom.
$X^3$ in Formula (I) is preferably a C1 to C6 alkyl group which may be substituted with a halogeno group or a C1 to C6 alkoxy group; or an unsubstituted C1 to C6 alkyl group, and more preferably the unsubstituted C1 to C6 alkyl group.

[A]

A in Formula (I) represents a nitrogen atom or $CX^4$.
In "$CX^4$", which is one of A's, $X^4$ represents a hydrogen atom, a halogeno group, a substituted or unsubstituted C1 to C6 alkyl group, a substituted or unsubstituted C1 to C6 alkoxy group, or a cyano group.
In "$CX^4$", which is one of A's, examples of the "halogeno group", the "substituted or unsubstituted C1 to C6 alkyl group", and the "substituted or unsubstituted C1 to C6 alkoxy group" in $X^4$ include the same groups as those provided as exemplary examples of Ar.
A in Formula (I) is preferably a nitrogen atom.
In "$CX^4$", which is one of A's, $X^4$ is preferably a hydrogen atom.

[$R^1$, $R^{2a}$, and $R^{2b}$]

$R^1$ in Formula (I) represents a hydrogen atom, or a substituted or unsubstituted C1 to C6 alkyl group.

$R^{2a}$ in Formula (I) represents a substituted or unsubstituted C1 to C6 alkyl group.

$R^{2b}$ in Formula (I) represents a hydrogen atom, or a substituted or unsubstituted C1 to C6 alkyl group.

Examples of the "substituted or unsubstituted C1 to C6 alkyl group" in $R^1$, $R^{2a}$, and $R^{2b}$ include the same groups as those provided as exemplary examples of Ar.

$R^{2a}$ and $R^{2b}$ in Formula (I) may be combined with each other to form a substituted or unsubstituted C2 to C5 alkylene group.

Examples of the "C2 to C5 alkylene group" formed by the combination of $R^{2a}$ and $R^{2b}$ include an ethylene group, a trimethylene group, a tetramethylene group, and a pentamethylene group. Examples of the substituent on the "C2 to C5 alkylene group" formed by the combination of $R^{2a}$ and $R^{2b}$ include a halogeno group such as a fluoro group, a chloro group, a bromo group, and an iodo group; and a C1 to C6 alkyl group such as a methyl group, an ethyl group, an n-propyl group, an i-propyl group, an n-butyl group, an s-butyl group, an i-butyl group, a t-butyl group, an n-pentyl group, and an n-hexyl group.

$R^1$ in Formula (I) is preferably a hydrogen atom or an unsubstituted C1 to C6 alkyl group, and more preferably the hydrogen atom.

$R^{2a}$ in Formula (I) is preferably an unsubstituted C1 to C6 alkyl group.

$R^{2b}$ in Formula (I) is preferably an unsubstituted C1 to C6 alkyl group.

[Y]

Y in Formula (I) represents a substituted or unsubstituted C1 to C6 alkyl group, a substituted or unsubstituted C6 to C10 aryl group, or a substituted or unsubstituted 5- or 6-membered heteroaryl group.

(1) The "Cl to C6 alkyl group" in Y may be linear or branched. Examples of the alkyl group include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, and an i-hexyl group.

Examples of the preferred substituent on the "Cl to C6 alkyl group" in Y include a halogeno group, a hydroxyl group, a C1 to C6 alkoxy group, a C1 to C6 haloalkoxy group, a C1 to C6 alkylthio group, a C1 to C6 alkylsulfinyl group, a C1 to C6 alkylsulfonyl group, a C1 to C6 haloalkylthio group, a C1 to C6 haloalkylsulfinyl group, a C1 to C6 haloalkylsulfonyl group, a substituted or unsubstituted C6 to C10 aryl group, a substituted or unsubstituted C6 to C10 aryloxy group, a substituted or unsubstituted 5- or 6-membered heteroaryl group, a substituted or unsubstituted 5- or 6-membered heteroaryloxy group, a C1 to C6 alkylcarbonyl group, a C1 to C6 alkoxycarbonyl group, a group represented by $R^aR^bN$-, a group represented by $R^aR^bN$-CO-, a group represented by $R^aR^bN$-$SO_2$-, a group represented by $R^cCONR^d$-, a group represented by $R^cSO_2NR^d$-, a group represented by $R^eO$-N=$CR^f$-, or a cyano group.

Specific examples thereof include a halogeno group such as a fluoro group, a chloro group, a bromo group, and an iodo group; a hydroxyl group; a C1 to C6 alkoxy group such as a methoxy group, an ethoxy group, an n-propoxy group, an i-propoxy group, an n-butoxy group, an s-butoxy group, an i-butoxy group, and a t-butoxy group; a C1 to C6 haloalkoxy group such as a 2-chloro-n-propoxy group, a 2,3-dichlorobutoxy group, and a trifluoromethoxy group; a C1 to C6 alkylthio group such as a methylthio group and an ethylthio group; a C1 to C6 alkylsulfinyl group such as a methylsulfinyl group and an ethylsulfinyl group; a C1 to C6 alkylsulfonyl group such as a methylsulfonyl group and an ethylsulfonyl group; a trifluoromethylthio group, a C1 to C6 haloalkylthio group such as a 2,2,2-trifluoroethylthio group; a C1 to C6 haloalkylsulfinyl group such as a trifluoromethylsulfinyl group and a 2,2,2-trifluoroethylsulfinyl group; a C1 to C6 haloalkylsulfonyl group such as a trifluoromethylsulfonyl group and a 2,2,2-trifluoroethylsulfonyl group; a C6 to C10 aryl group which is substituted with a halo or with trifluoromethyl, or unsubstituted, such as a phenyl group, a 4-chlorophenyl group, and a 4-trifluoromethylphenyl group; a C6 to C10 aryloxy group which is substituted with a halo or with trifluoromethyl, or unsubstituted, such as a phenyloxy group, a 4-chlorophenyloxy group, and a 4-trifluoromethylphenyloxy group; a 5- or 6-membered heteroaryl group (preferably an oxadiazolyl group) which is substituted with a halo, with a C1 to C6 alkyl, or with trifluoromethyl, or unsubstituted; a 5- or 6-membered heteroaryloxy group (preferably a pyridyloxy group) which is substituted with a halo, with a C1 to C6 alkyl, or with trifluoromethyl, or unsubstituted; a C1 to C6 alkylcarbonyl group such as an acetyl group and a propionyl group; a C1 to C6 alkoxycarbonyl group such as a methoxycarbonyl group, an ethoxycarbonyl group, an n-propoxycarbonyl group, an i-propoxycarbonyl group, and a t-butoxycarbonyl group; and a cyano group.

In the "group represented by $R^aR^bN$-", which is one of the preferred substituents on the "Cl to C6 alkyl group", $R^a$ represents a hydrogen atom, a C1 to C6 alkyl group, or a C1 to C6 alkoxy group, $R^b$ represents a hydrogen atom or a C1 to C6 alkyl group, and $R^a$ and $R^b$ may be combined with each other to form a C3 to C6 alkylene group.

Examples of the "Cl to C6 alkyl group" in $R^a$ and $R^b$ include a methyl group, an ethyl group, an n-propyl group,

an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, and an i-hexyl group.

Examples of the "Cl to C6 alkoxy group" include a methoxy group, an ethoxy group, an n-propoxy group, an i-propoxy group, an n-butoxy group, an s-butoxy group, an i-butoxy group, and a t-butoxy group.

Examples of the "C3 to C6 alkylene group" formed by the combination of $R^a$ and $R^b$ include a trimethylene group, a tetramethylene group, and a pentamethylene group.

Examples of the group represented by $R^aR^bN$- include an amino group, an N-methylamino group, an N,N-dimethylamino group, an N-ethyl-N-methylamino group, an N-methoxy-N-methylamino group, and a pyrrolidin-1-yl group.

In the "group represented by $R^aR^bN$-CO-", which is one of the preferred substituents on the "Cl to C6 alkyl group", $R^a$ and $R^b$ have the same meanings as described above.

Examples of the group represented by $R^aR^bN$-CO- include a carbamoyl group, an N-methylaminocarbonyl group, an N,N-dimethylaminocarbonyl group, an N-ethyl-N-methylaminocarbonyl group, an N-methoxy-N-methylaminocarbonyl group, and a pyrrolidine-1-carbonyl group.

In the "group represented by $R^aR^bN$-$SO_2$-", which is one of the preferred substituents on the "Cl to C6 alkyl group", $R^a$ and $R^b$ have the same meanings as described above.

Examples of the group represented by $R^aR^bN$-$SO_2$- include a sulfamoyl group, an N-methylaminosulfonyl group, an N,N-dimethylaminosulfonyl group, an N-ethyl-N-methylaminosulfonyl group, an N-methoxy-N-methylaminosulfonyl group, and a pyrrolidin-1-ylsulfonyl group.

In the "group represented by $R^cCONR^d$-", which is one of the preferred substituents on the "Cl to C6 alkyl group", $R^c$ represents a C1 to C6 alkyl group, and $R^d$ represents a hydrogen atom or a C1 to C6 alkyl group.

Examples of the "Cl to C6 alkyl group" in $R^c$ and $R^d$ include the same groups as those provided as exemplary examples of $R^a$ and $R^b$.

Examples of the group represented by $R^cCONR^d$- include an acetamide group and an N-methylacetamide group.

In the "group represented by $R^cSO_2NR^d$-", which is one of the preferred substituents on the "Cl to C6 alkyl group", $R^c$ and $R^d$ have the same meanings as described above.

Examples of the group represented by $R^cSO_2NR^d$- include a methylsulfonamide group and an N-methylmethylsulfonamide group.

In "group represented by $R^eO$-N=$CR^f$-, " which is one of preferred substituents on "Cl to C6 alkyl group", $R^e$ represents a C1 to C6 alkyl group, and $R^f$ represents a hydrogen atom or a C1 to C6 alkyl group.

Examples of the "Cl to C6 alkyl group" in $R^e$ and $R^f$ include the same groups as those provided as exemplary examples of $R^a$ and $R^b$.

Examples of the group represented by $R^eO$-N=$CR^f$- include a (methoxyimino)methyl group, an (ethoxyimino)methyl group, a (methoxyimino)ethyl group, and an (ethoxyimino)ethyl group.

Among these, more preferred examples of the substituent on the "Cl to C6 alkyl group" in Y include the C1 to C6 alkylthio group, the C1 to C6 alkylsulfinyl group, the C1 to C6 alkylsulfonyl group, or the group represented by $R^aR^bN$-CO- (preferably an N-methylaminocarbonyl group).

(2) The "C6 to C10 aryl group" in Y is a group formed by removing one hydrogen on the ring of the monocyclic or polycyclic aromatic hydrocarbon. Examples of the C6 to C10 aryl group include a phenyl group and a naphthyl group, and the phenyl group is preferable.

Examples of the substituent on the C6 to C10 aryl group include a halogeno group, a substituted or unsubstituted C1 to C6 alkyl group, a substituted or unsubstituted C2 to C6 alkenyl group, a substituted or unsubstituted C2 to C6 alkynyl group, a substituted or unsubstituted C1 to C6 alkoxy group, a substituted or unsubstituted C2 to C6 alkenyloxy group, a formyl group, a substituted or unsubstituted C1 to C6 alkylcarbonyl group, a substituted or unsubstituted C1 to C6 alkoxycarbonyl group, a substituted or unsubstituted C1 to C6 alkylthio group, a substituted or unsubstituted C1 to C6 alkylsulfinyl group, a substituted or unsubstituted C1 to C6 alkylsulfonyl group, a substituted or unsubstituted C3 to 8 cycloalkyl group, a substituted or unsubstituted C6 to C10 aryl group, a substituted or unsubstituted 5- or 6-membered heteroaryl group, a substituted or unsubstituted C6 to C10 aryloxy group, a substituted or unsubstituted 5- or 6-membered heteroaryloxy group, a hydroxyl group, a nitro group, or a cyano group.

Specific examples thereof include the same substituents as those provided as exemplary examples of Ar.

(3) The "5- or 6-membered heteroaryl group" in Y is a group generated by removing one hydrogen atom from any ring atom of a 5- or 6-membered cyclyl aromatic compound including 1 to 4 hetero atoms selected from the group consisting of a nitrogen atom, an oxygen atom, and a sulfur atom as constituent atoms of the ring (which may also be referred to as ring member atoms), that is, a "5- or 6-membered heteroaryl compound".

The "5- or 6-membered heteroaryl group" may also be expressed as "a 5- or 6-membered heteroaryl group including 1 to 4 heteroatoms selected from the group consisting of a nitrogen atom, an oxygen atom, and a sulfur atom as ring member atoms".

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a

triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

Examples of the substituent on the 5- or 6-membered heteroaryl group include the same substituents as those on the C6 to C10 aryl group described in (2), and preferably include the C1 to C6 alkyl group or the halogeno group.

Among these, Y is preferably the substituted or unsubstituted C1 to C6 alkyl group or the substituted or unsubstituted 5- or 6-membered heteroaryl group (preferably a triazolyl group, an oxadiazolyl group, or a pyrimidinyl group), and more preferably the substituted or unsubstituted C1 to C6 alkyl group, the 5-membered heteroaryl group (preferably a triazolyl group or an oxadiazolyl group) which may be substituted with a C1 to C6 alkyl group, or the 6-membered heteroaryl group (preferably a pyrimidinyl group) which may be substituted with a halogeno group.

The compound represented by Formula (I) is preferably represented by Formula (I-1), (I-2), or (I-3).

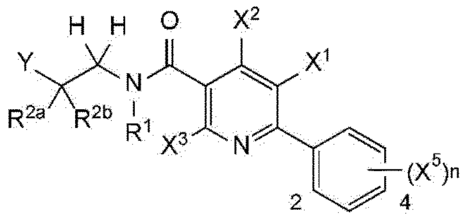

(I−1)

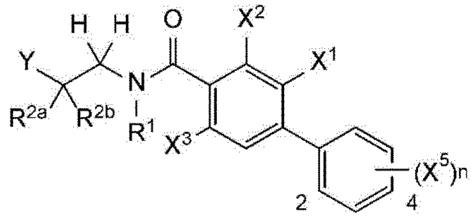

(I−2)

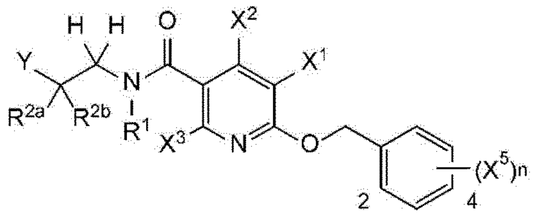

(I−3)

In the formula, $R^1$, $R^{2a}$, $R^{2b}$, Y, $X^1$, $X^2$, $X^3$, and $X^5$ are as described above, and n represents the number of the substituents $X^5$'s on the phenyl group, and represents 1 or 2.

The salt of the compound (I) is not particularly limited as long as it is an agriculturally and horticulturally acceptable salt. Examples thereof include salts of inorganic acids such as hydrochloric acid and sulfuric acid; salts of organic acids such as acetic acid and lactic acid; salts of alkali metals such as lithium, sodium, and potassium; salts of alkaline earth metals such as calcium and magnesium; salts of transition metals such as iron and copper; and salts of organic bases such as ammonia, triethylamine, tributylamine, pyridine, and hydrazine.

The compound (I) or the salt of the compound (I) is not particularly limited by a production method thereof. In addition, the salt of the compound (I) can be obtained from the compound (I) by a known method. For example, the compound (I) or the salt of the compound (I) of the present invention can be obtained by the following production method, specifically, by a known production method described in Examples and the like.

(Production Method 1)

A hydrochloride represented by Formula (I-1a) and a carboxylic acid represented by Formula (I-1b) are reacted with each other in the presence of a suitable base such as triethylamine to obtain a compound represented by Formula (I-1). In the following formula, $R^1$, $R^{2a}$, $R^{2b}$, Y, $X^1$, $X^2$, $X^3$, $X^5$, and n are as described above.

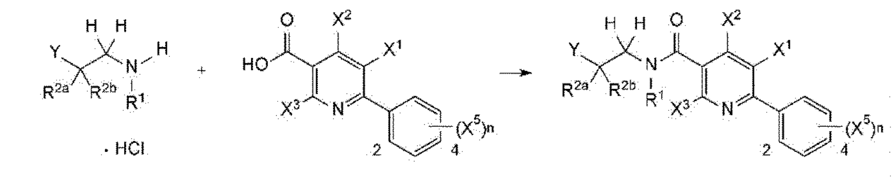

(I-1a) (I-1b) (I-1)

The (hetero)arylamide compound) of the present invention (which may be hereinafter referred to as "the compound of the present invention") has an excellent control effect on various pests such as agricultural pests affecting the growth of plants, and acari.

In addition, the compound of the present invention has high safety since it does not have phytotoxicity against plants and has low toxicity against fish or warm-blooded animals. Therefore, the compound of the present invention is useful as an active ingredient of an insecticidal or acaricidal agent.

Furthermore, in recent years, many pests such as diamondback moths, planthoppers, leafhoppers, and aphids have developed resistance to various existing drugs, causing a problem of insufficient efficacy of the drugs. Thus, there is a demand for drugs that are also effective for resistant strains of pests. The compound of the present invention exhibits an excellent control effect not only on sensitive strains of pests but also on various resistant strains of pests and acaricidal agent-resistant strains of acari.

The compound of the present invention has an excellent control effect on ectoparasites that harm humans and animals. In addition, the compound of the present invention has high safety since it has low toxicity against fish or warm-blooded animals. Therefore, the compound of the present invention is useful as an active ingredient of an ectoparasite control agent.

Moreover, the compound of the present invention exhibits efficacy at all developmental stages of an organism to be controlled, and exhibits an excellent control effect on, for example, eggs, nymph, larvae, puses, and adults of mites, insects, and the like.

[Pest Control Agent, or Insecticidal or Acaricidal Agent]

The pest control agent, or the insecticidal or acaricidal agent of the present invention contains at least one selected from the (hetero)arylamide compounds of the present invention as an active ingredient. The amount of the compound of the present invention included in the pest control agent, or the insecticidal or acaricidal agent of the present invention is not particularly limited as long as it exhibits a pest control effect.

The pest control agent, or the insecticidal or acaricidal agent of the present invention are preferably used for plants such as grains; vegetables; root vegetables; potatoes; flowers; trees such as fruit trees, foliage plants, tea, coffee, cacao, or the like; grasses for pastures; grasses for lawns; and cotton.

In the application to the plants, the pest control agent, or the insecticidal or acaricidal agent of the present invention may be applied on any one part of the plants, such as leaf, stem, stalk, flower, bud, fruit, seed, sprout, root, tuber, tuberous root, shoot, cutting, and the like. In addition, although the plant varieties for which the pest control agent, or the insecticidal or acaricidal agent of the present invention is applicable are not particularly limited, examples of the plant varieties include the originals, varieties, improved varieties, cultivated varieties, mutant plants, hybrid plants, and genetically-modified plants (GMO).

The pest control agent of the present invention can be used for seed treatment, foliage spraying, soil application, water surface application, and the like in order to control various agricultural pests and acari.

Specific examples of the various agricultural pests and acari which can be controlled by the pest control agent of the present invention are shown below.

(1) Butterflies or moths of Lepidoptera order

(a) Moths belonging to the Arctiidae family, for example, Hyphantria cunea and Lemyra imparilis;
(b) moths belonging to the Bucculatricidae family, for example, Bucculatrix pyrivorella;
(c) moths belonging to the Carposinidae family, for example, Carposina sasakii;
(d) moths belonging to the Crambidae family, for example, Diaphania indica and Diaphania nitidalis of Diaphania spp.; for example, Ostrinia furnacalis, Ostrinia nubilalis and Ostrinia scapulalis of Ostrinia spp.; and others such as Chilo suppressalis, Cnaphalocrocis medinalis, Conogethes punctiferalis, Diatraea grandiosella, Glyphodes pyloalis, Hellula undalis, and Parapediasia teterrella;

(e) moths belonging to the Gelechiidae family, for example, Helcystogramma triannulella, Pectinophora gossypiella, Phthorimaea operculella, and Sitotroga cerealella;
(f) moths belonging to the Geometridae family, for example, Ascotis selenaria;
(g) moths belonging to the Gracillariidae family, for example, Caloptilia theivora, Phyllocnistis citrella, and Phyllonorycter ringoniella;
(h) butterflies belonging to the Hesperiidae family, for example, Parnara guttata;
(i) moths belonging to the Lasiocampidae family, for example, Malacosoma neustria;
(j) moths belonging to the Lymantriidae family, for example, Lymantria dispar and Lymantria monacha of Lymantria spp.; and others such as Euproctis pseudoconspersa and Orgyia thyellina;
(k) moths belonging to the Lyonetiidae family, for example, Lyonetia clerkella and Lyonetia prunifoliella malinella of Lyonetia spp.;
(l) moths belonging to the Noctuidae family, for example, Spodoptera depravata, Spodoptera eridania, Spodoptera exigua, Spodoptera frugiperda, Spodoptera littoralis and Spodoptera litura of Spodoptera spp.; for example, Autographa gamma and Autographa nigrisigna of Autographa spp.; for example, Agrotis ipsilon and Agrotis segetum of Agrotis spp.; for example, Helicoverpa armigera, Helicoverpa assulta and Helicoverpa zea of Helicoverpa spp.; for example, Heliothis armigera and Heliothis virescens of Heliothis spp.; and others such as Aedia leucomelas, Ctenoplusia agnata, Eudocima tyrannus, Mamestra brassicae, Mythimna separata, Naranga aenescens, Panolis japonica, Peridroma saucia, Pseudoplusia includens, and Trichoplusia ni;
(m) moths belonging to the Nolidae family, for example, Earias insulana;
(n) butterflies belonging to the Pieridae family, for example, Pieris brassicae and Pieris rapae crucivora of Pieris spp.;
(o) moths belonging to the Plutellidae family, for example, Acrolepiopsis sapporensis and Acrolepiopsis suzukiella of Acrolepiopsis spp.; and others such as Plutella xylostella;
(p) moths belonging to the Pyralidae family, for example, Cadra cautella, Elasmopalpus lignosellus, Etiella zinckenella, and Galleria mellonella;
(q) moths belonging to the Sphingidae, for example, Manduca quinquemaculata and Manduca sexta of Manduca spp.;
(r) moths belonging to the Stathmopodidae family, for example, Stathmopoda masinissa;
(s) moths belonging to the Tineidae family, for example, Tinea translucens;
(t) moths belonging to the Tortricidae family, for example, Adoxophyes honmai and Adoxophyes orana of Adoxophyes spp.; for example, Archips breviplicanus and Archips fuscocupreanus Archips spp.; and others such as Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana, and Sparganothis pilleriana; and
(u) moths belonging to the Yponomeutidae family, for example, Argyresthia conjugella.

(2) Pests of Thysanoptera order

(a) Pests belonging to the Phlaeothripidae family, for example, Ponticulothrips diospyrosi; and
(b) pests belonging to the Thripidae family, for example, Frankliniella intonsa and Frankliniella occidentalis of Frankliniella spp.; for example, Thrips palmi and Thrips tabaci of Thrips spp.; and others such as Heliothrips haemorrhoidalis and Scirtothrips dorsalis.

(3) Pests of Hemiptera order

(A) Archaeorrhyncha suborder

(a) Pests belonging to the Delphacidae family, for example, Laodelphax striatella, Nilaparvata lugens, Perkinsiella saccharicida, and Sogatella furcifera.

(B) Clypeorrhyncha suborder

(a) Pests belonging to the Cicadellidae family, for example, Empoasca fabae, Empoasca nipponica, Empoasca onukii and Empoasca sakaii of Empoasca spp.; and others such as Arboridia apicalis, Balclutha saltuella, Epiacanthus stramineus, Macrosteles striifrons, and Nephotettix cinctinceps.

(C) Heteroptera suborder

(a) Pests belonging to the Alydidae family, for example, Riptortus clavatus;
(b) pests belonging to the Coreidae family, for example, Cletus punctiger and Leptocorisa chinensis;
(c) pests belonging to the Lygaeidae family, for example, Blissus leucopterus, Cavelerius saccharivorus, and Togo hemipterus;
(d) pests belonging to the Miridae family, for example, Halticus insularis, Lygus lineolaris, Psuedatomoscelis seriatus, Stenodema sibiricum, Stenotus rubrovittatus, and Trigonotylus caelestialium;
(e) pests belonging to the Pentatomidae family, for example, Nezara antennata and Nezara viridula of Nezara spp.; for example, Eysarcoris aeneus, Eysarcoris lewisi and Eysarcoris ventralis of Eysarcoris spp.; and others such as Dolycoris baccarum, Eurydema rugosum, Glaucias subpunctatus, Halyomorpha halys, Piezodorus hybneri, Plautia crossota, and Scotinophora lurida;
(f) pests belonging to the Pyrrhocoridae family, for example, Dysdercus cingulatus;
(g) pests belonging to the Rhopalidae family, for example, Rhopalus msculatus;
(h) pests belonging to the Scutelleridae family, for example, Eurygaster integriceps; and
(i) pests belonging to the Tingidae family, for example, Stephanitis nashi.

(D) Stemorrhyncha suborder

(a) Pests belonging to the Adelgidae family, for example, Adelges laricis;
(b) pests belonging to the Aleyrodidae family, for example, Bemisia argentifolii, Bemisia tabaci of Bemisia spp.; and others such as Aleurocanthus spiniferus, Dialeurodes citri, and Trialeurodes vaporariorum;
(c) pests belonging to the Aphididae family, for example, Aphis craccivora, Aphis fabae, Aphis forbesi, Aphis gossypii, Aphis pomi, Aphis sambuci and Aphis spiraecola of Aphis spp.; for example, Rhopalosiphum maidis and Rhopalosiphum padi of Rhopalosiphum spp.; for example, Dysaphis plantaginea and Dysaphis radicola of Dysaphis spp.; for example, Macrosiphum avenae and Macrosiphum euphorbiae of Macrosiphum spp.; for example, Myzus cerasi, Myzus persicae and Myzus varians of Myzus spp.; and others such as Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion avenae, and Toxoptera aurantii;
(d) pest belong to the Coccidae family, for example, Ceroplastes ceriferus and Ceroplastes rubens of Ceroplastes spp.;
(e) pests belonging to the Diaspididae family, for example, Pseudaulacaspis pentagona and Pseudaulacaspis prunicola of Pseudaulacaspis spp.; for example, Unaspis euonymi and Unaspis yanonensis of Unaspis spp.; and others such as Aonidiella aurantii, Comstockaspis pemiciosa, Fiorinia theae, and Pseudaonidia paeoniae;
(f) pests belonging to the Margarodidae family, for example, Drosicha corpulenta and Icerya purchasi;
(g) pests belonging to the Phylloxeridae family, for example, Viteus vitifolii;
(h) pests belonging to the Pseudococcidae family, for example, Planococcus citri and Planococcus kuraunhiae of Planococcus spp.; and others such as Phenacoccus solani and Pseudococcus comstocki; and
(i) pests belonging to the Psyllidae family, for example, Psylla mali and Psylla pyrisuga of Psylla spp.; and others such as Diaphorina citri.

(4) Pests of Polyphaga suborder

(a) Pests belonging to the Anobiidae family, for example, Lasioderma serricorne;
(b) pests belonging to the Attelabidae family, for example, Byctiscus betulae and Rhynchites heros;
(c) pests belonging to the Bostrichidae family, for example, Lyctus brunneus;
(d) pests belonging to the Brentidae family, for example, Cylas formicarius;
(e) pests belonging to the Buprestidae family, for example, Agrilus sinuatus;
(f) pests belonging to the Cerambycidae family, for example, Anoplophora malasiaca, Monochamus alternatus, Psacothea hilaris, and Xylotrechus pyrrhoderus;
(g) pests belonging to the Chrysomelidae family, for example, Bruchus pisorum and Bruchus rufimanus of Bruchus spp.; for example, Diabrotica barberi, Diabrotica undecimpunctata and Diabrotica virgifera of Diabrotica spp.; for example, Phyllotreta nemorum and Phyllotreta striolata of Phyllotreta spp.; and others such as Aulacophora femoralis, Callosobruchus chinensis, Cassida nebulosa, Chaetocnema concinna, Leptinotarsa decemlineata, Oulema oryzae, and Psylliodes angusticollis;
(h) pests belonging to the Coccinellidae family, for example, Epilachna varivestis and Epilachna vigintioctopunctata of Epilachna spp.;

(i) pests belonging to the Curculionidae family, for example, Anthonomus grandis and Anthonomus pomorum of Anthonomus spp.; for example, Sitophilus granarius and Sitophilus zeamais of Sitophilus spp.; and others such as Echinocnemus squameus, Euscepes postfasciatus, Hylobius abietis, Hypera postica, Lissohoptrus oryzophilus, Otiorhynchus sulcatus, Sitona lineatus, and Sphenophorus venatus;
(j) pests belonging to the Elateridae family, for example, Melanotus fortnumi and Melanotus tamsuyensis of Melanotus spp.;
(k) pests belonging to the Nitidulidae family, for example, Epuraea domina;
(l) pests belonging to the Scarabaeidae family, for example, Anomala cuprea and Anomala rufocuprea of Anomala spp.; and others such as Cetonia aurata, Gametis jucunda, Heptophylla picea, Melolontha melolontha, and Popillia japonica;
(m) pests belonging to the Scolytidae family, for example, Ips typographus;
(n) pests belonging to the Staphylinidae family, for example, Paederus fuscipes;
(o) pests belonging to the Tenebrionidae family, for example, Tenebrio molitor and Tribolium castaneum; and
(p) pests belonging to the Trogossitidae family, for example, Tenebroides mauritanicus.

(5) Pests of Diptera order

(A) Brachycera suborder

(a) Pests belonging to the Agromyzidae family, for example, Liriomyza bryoniae, Liriomyza chinensis, Liriomyza sativae and Liriomyza trifolii of Liriomyza spp.; and others such as Chromatomyia horticola and Agromyza oryzae;
(b) pests belonging to the Anthomyiidae family, for example, Delia platura, Delia radicum of Delia spp.; and others such as Pegomya cunicularia;
(c) pests belonging to the Drosophilidae family, for example, Drosophila melanogaster and Drosophila suzukii of Drosophila spp.;
(d) pests belonging to the Ephydridae family, for example, Hydrellia griseola;
(e) pests belonging to the Psilidae family, for example, Psila rosae; and
(f) pests belonging to the Tephritidae family, for example, Bactrocera cucurbitae and Bactrocera dorsalis of Bactrocera spp.; for example, Rhagoletis cerasi and Rhagoletis pomonella of Rhagoletis spp.; and others such as Ceratitis capitata and Dacus oleae.

(B) Nematocera suborder

(a) Pests belonging to the Cecidomyiidae family, for example, Asphondylia yushimai, Contarinia sorghicola, Mayetiola destructor, and Sitodiplosis mosellana.

(6) Pests of Orthoptera order

(a) Pests belonging to the Acrididae family, for example, Schistocerca Americana and Schistocerca gregaria of Schistocerca spp.; and others such as Chortoicetes terminifera, Dociostaurus maroccanus, Locusta migratoria, Locustana pardalina, Nomadacris septemfasciata, and Oxya yezoensis;
(b) pests belonging to the Gryllidae family, for example, Acheta domestica and Teleogryllus emma;
(c) pests belonging to the Gryllotalpidae family, for example, Gryllotalpa orientalis; and
(d) pests belonging to the Tettigoniidae family, for example, Tachycines asynamorus.

(7) Acari

(A) Acaridida of Astigmata order

(a) Mites belonging to the Acaridae family, for example, Rhizoglyphus echinopus and Rhizoglyphus robini of Rhizoglyphus spp.; Tyrophagus neiswanderi, Tyrophagus pemiciosus, Tyrophagus putrescentiae and Tyrophagus similis of Tyrophagus spp.; and others such as Acarus siro, Aleuroglyphus ovatus, and Mycetoglyphus fungivorus.

(B) Actinedida of Prostigmata order

(a) Mites belonging to the Tetranychidae family, for example, Bryobia praetiosa and Bryobia rubrioculus of

Bryobia spp.; for example, Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis and Eotetranychus uncatus of Eotetranychus spp.; for example, Oligonychus hondoensis, Oligonychus ilicis, Oligonychus karamatus, Oligonychus mangiferus, Oligonychus orthius, Oligonychus perseae, Oligonychus pustulosus, Oligonychus shinkajii and Oligonychus ununguis of Oligonychus spp.; for example, Panonychus citri, Panonychus mori and Panonychus ulmi of Panonychus spp.; for example, Tetranychus cinnabarinus, Tetranychus evansi, Tetranychus kanzawai, Tetranychus ludeni, Tetranychus quercivorus, Tetranychus phaselus, Tetranychus urticae and Tetranychus viennensis of Tetranychus spp.; for example, Aponychus corpuzae and Aponychus firmianae of Aponychus spp.; for example, Sasanychus akitanus and Sasanychus pusillus of Sasanychus spp.; for example, Shizotetranychus celarius, Shizotetranychus longus, Shizotetranychus miscanthi, Shizotetranychus recki and Shizotetranychus schizopus of Shizotetranychus spp.; and others such as Tetranychina harti, Tuckerella pavoniformis, and Yezonychus sapporensis;

(b) mites belonging to the Tenuipalpidae family, for example, Brevipalpus lewisi, Brevipalpus obovatus, Brevipalpus phoenicis, Brevipalpus russulus, and Brevipalpus californicus of Brevipalpus spp.; for example, Tenuipalpus pacificus and Tenuipalpus zhizhilashviliae of Tenuipalpus spp.; and others such as Dolichotetranychus floridanus;

(c) mites belonging to the Eriophyidae family, for example, Aceria diospyri, Aceria ficus, Aceria japonica, Aceria kuko, Aceria paradianthi, Aceria tiyingi, Aceria tulipae and Aceria zoysiea of Aceria spp.; for example, Eriophyes chibaensis and Eriophyes emarginatae of Eriophyes spp.; for example, Aculops lycopersici and Aculops pelekassi of Aculops spp.; for example, Aculus fockeui, Aculus schlechtendali, which belong Aculus spp.; and others such as Acaphylla theavagrans, Calacarus carinatus, Colomerus vitis, Calepitrimerus vitis, Epitrimerus pyri, Paraphytoptus kikus, Paracalacarus podocarpi, and Phyllocotruta citri;

(d) mites belonging to the Transonemidae family, for example, Tarsonemus bilobatus and Tarsonemus waitei of Tarsonemus spp.; and others such as Phytonemus pallidus and Polyphagotarsonemus latus; and

(e) mites belonging to the Penthaleidae family, for example, Penthaleus erythrocephalus and Penthaleus major of Penthaleus spp.

The pest control agent, or the insecticidal or acaricidal agent of the present invention may contain components other than the compound of the present invention. Examples of other components include a known carrier used for making preparations. In addition, examples of such other components include known fungicides, insecticidal or acaricidal agents, nematocides, soil pesticides, plant growth regulators, herbicides, synergists, fertilizers, soil improvers, and animal feeds. By containing these other components, synergistic effects can be exhibited.

Specific examples of the insecticidal or acaricidal agents, the nematocides, the soil pesticides, the anthelmintic agents, and the like, which can be mixed or used in combination with the pest control agent, or the insecticidal or acaricidal agent of the present invention, are shown below.

(1A) (Carbamate-based) Acetylcholine esterase (AChE) inhibitors

Alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, and xylycarb. Aldoxycarb, allyxycarb, aminocarb, bufencarb, cloethocarb, phenothiocarb, and promecarb.

(1B) (Organophosphorus-based) Acetylcholine esterase (AChE) inhibitors

acephate, azamethiphos, azinphos-ethyl, azinphos-methyl, cadusafos, chlorethoxyfos, chlorfenvinphos, chlormephos, chlorpyriphos, chlorpyriphos-methyl, coumaphos, cyanophos, demeton-S-methyl, diazinon, dichlorvos, dicrotophos, dimethoate, dimethylvinfos, disulfoton, EPN, ethion, ethoprophos, famphur, fenamiphos, fenitrothion, fenthion, fosthiazete, heptenophos, imicyafos, isofenphos, isopropyl=O-(methoxyaminothiophosphoryl)salicylate, isoxathion, malathion, mecarbam, methamidophos, methidathion, mevinphos, monocrotophos, naled, omethoate, oxydemeton-metyl, parathion, parathion-methyl, phenthoate, phorate, phosalone, phosmet, phosphamidon, phoxim, pirimiphos-methyl, profenofos, propetamphos, prothiofos, pyraclofos, pyridafenthion, quinalphos, sulfotep, tebupirimfos, temephos, terbufos, tetrachlorvinphos, thiomethon, triazophos, trichlorfon, and vamidothion.

Bromophos-ethyl, cyanofenphos, demeton-S-methyl sulfone, dialifos, dichlofenthion, dioxabenzofos, etrimfos, fensulfothion, fonofos, formothion, iodofenphos, isazofos, isocarbphos, methacrifos, phosphocarb, pyrimiphos-ethyl, propaphos, protoate, and sulprofos.

(2) GABA-gated chloride ion (chlorine ion) channel blockers

Chlordane and endosulfan; and ethiprole and fipronil.
Acetoprole, camphechlor, dienochlor, heptachlor, pyrafluprole, and pyriprole; and flufiprole.

(3A) (Pyrethroid-based) Sodium channel modulators

Acrinathrin, allethrin, d-cis-trans-allethrin, d-trans-allethrin, bifenthrin, bioallethrin, a bioallethrin-S-cyclopentenyl-isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ζ-cypermethrin, cyphenothrin [(1R)-trans isomer], delta-methrin, empenthrin [(EZ)-(1R)-isomer], esfenvalerate, ethofenprox, fenpropathrin, fenvalerate, flucythrinate, flumethrin, τ-fluvalinate, halfenprox, imiprothrin, kadethrin, permethrin, phenothrin [(1R)-trans isomer], prallethrin, pyrethrum, resmethrin, silafluofen, tefluthrin, tetramethrin, tetramethrin [(1R)-isomer], tralomethrin, and transfluthrin.
κ-Bifenthrin, biopermethrin, chloroprallethrin, dimefluthrin, fenfluthrin, fenpyritrin, flufenprox, heptafluthrin, meperfluthrin, ε-metofluthrin, momfluorothrin, ε-momfluorothrin, trans-permethrin, profluthrin, protrifenbuto, κ-tefluthrin, tellallethrin, and tetramethylfluthrin; and bioethanomethrin.

(3B) Sodium channel modulators (DDTs)

DDT and methoxychlor.

(4) Nicotinic acetylcholine receptor (nAChR) competitive modulators

acetamiprid, clothianidine, dinotefuran, imidacloprid, nitenpyram, and thiacloprid, thiamethoxam; nicotine; sulfoxaflor; flupyradifurone; and triflumezopyrim.
Nithiazine; and dichloromezothiaz and flupyrimin.

(5) Nicotinic acetylcholine receptor (nAChR) allosteric modulators

Spinetoram and spinosad.

(6) Glutamate-gated chloride ion channel (GluCl) allosteric modulators

Abamectin, emamectin, emamectin benzoate, lepimectin, and milbemectin. Doramectin, eprinomectin, ivermectin, moxidectin, and seramectin.

(7) Juvenile hormone analogues

Hydroprene, kinoprene, and methoprene; fenoxycarb; and pyriproxyfen. Diofenolan, epofenonane, and triprene.

(8) Other non-specific (multi-site) inhibitors

Methyl bromide and alkyl halides; chloropicrin; sodium aluminum fluoride and sulphuryl fluoride; borax, boric acid, disodium octaborate, sodium borate, and sodium metaborate; potassium antimony tartrate; and dazomet, metam, a metam-potassium salt, and a metam-sodium salt.

(9) Homoptera TRPV channel modulators

Pymetrozine and pyrifluquinazon; and aphidopyropene.

(10) Acari growth inhibitors

Clofentezine, diflovidazin, and hexythiazox; and etoxazole.

(11) Microorganism-derived insect midgut inner membrane distruptors

B. t. subsp. israelensis, B. t. subsp. aizawai, B. t. subsp. kurstaki, and B. t. subsp. tenebrionis; proteins included in B. t. crop: Cry1Ab, CrylAc, CrylFa, CrylA.105, Cry2Ab, Vip3A, mCry3A, Cry3Ab, Cry3Bb, and Cry34Ab1/Cry35Ab1; and Bacillus sphaericus.

(12) Mitochondria ATP synthetase inhibitors

Diafenthiuron; azocyclotin, cyhexatin, and fenbutatin oxide; propargite; and tetradifon.

(13) Oxidative phosphorylation uncouplers that disrupt proton gradient

Chlorfenapyr, DNOC, and sulfluramid.
Binapacryl, dinobuton, and dinocap.

(14) Nicotinic acetylcholine receptor (nAChR) channel blockers

Bensultap, cartap hydrochloride, thiocyclarm, and a thiosultap-sodium salt.

(15) Chitin bioynthesis inhibitors, Type 0

Bistrifluron, chlorfluazuron, diflubenzuron, flucycloxuron, flufenoxuron, hexaflumuron, lufenuron, novaluron, nobifumuron, teflubenzuron, and triflumuron.
Fluazuron.

(16) Chitin biosynthesis inhibitors, Type 1

Buprofezin.

(17) Molting inhibitors

Cyromazine.

(18) Molting hormone (ecdysone) receptor agonists

Chromafenozide, halofenozide, methoxyfenozide, and tebufenozide.

(19) Octopamine receptor agonists

Amitraz.
Chlordimeform.

(20) Mitochondrial electron transport system complex III inhibitors

Hydramethylnon; acequinocyl; fluacrypyrim; and bifenazate.

(21) Mitochondrial electron transport system complex I inhibitors (METI)

Fenazaquin, fenpyroximate, pyridaben, pyrimidifen, tebufenpyrad, and tolfenpyrad; and rotenone.

(22) Voltage-dependent sodium channel blockers

Indoxacarb; and metaflumizone.

(23) Acetyl-CoA carboxylase inhibitors

Spirodiclofen, spiromesifen, and spirotetramat.
Spiropidion.

(24) Mitochondrial electron transport system complex IV Inhibitors

Aluminum phosphide, calcium phosphide, zinc phosphide (Zn-phosphide), and phosphine; and calcium cyanide, sodium cyanide, and potassium cyanide.

(25) Mitochondrial electron transport system complex II Inhibitors

Cyenopyrafen and cyflumetofen; and pyflubumide.

(28) Rianodine receptor modulators

Chlorantraniliprole, cyantraniliprole, cyclaniliprole, and flubendiamide. Cyhalodiamide, tetrachlorantraniliprole, and tetraniliprole.

(29) Chordotonal organ modulators, target site unidentified Flonicamid.

(30) GABA-gated chloride ion channel allosteric modulators Broflanilide and fluxametamide.
Isocycloseram; afoxolaner, fluralaner, lotilaner, and sarolanar.

(31) Other insecticidal and acaricidal agents

Azadirachtin, benzoximate, bromopropylate, chinomethionate, dicofol, a lime/sulfur mixture, mancozeb, pyridalyl, and sulfur.
Acynonapyr, amidoflumet, benzomate, benzpyrimoxan, chlorobenzilate, dicyclanil, fenoxacrim, fentrifanil, flometoquin, flubenzimine, fluphenazine, fluhexafon, fluopyram, metaflumizone, methoxadiazone, oxazosulfil, tetrasul, triarathene, and tyclopyrazoflor.

(32) Anthelmintic agents:

(a) benzimidazole-based anthelmintics: fenbendazole, albendazole, triclabendazole, oxibendazole, mebendazole, oxfendazole, parbendazole, flubendazole, febantel, netobimin, thiophanate, thiabendazole, and cambendazole;
(b) salicylanilide-based anthelmintics: closantel, oxyclozanide, rafoxanide, and niclosamide;
(c) substituted phenol-based anthelmintics: nitroxinil and nitroscanate;
(d) pyrimidine-based anthelmintics: pyrantel and morantel;
(e) imidazothiazole-based anthelmintics: levamisole and tetramisole;
(f) tetrahydropyrimidine-based anthelmintics: praziquantel and epsiprantel; and
(g) other anthelmintics: cyclodien, ryania, clorsulon, metronidazole, demiditraz, piperazine, diethylcarbamazine, dichlorophen, monepantel, tribendimidine, amidantel, thiacetarsamide, and melarsomine, arsenamide.

Specific examples of the fungicide that can be mixed with or used in combination with the pest control agent, or the insecticidal or acaricidal agent of the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, and ofurace;
(b) adenosine deaminase inhibitors: bupirimate, dimethirimol, and ethirimol;
(c) DNA/RNA synthesis inhibitors: hymexazol and octhilinone; and
(d) DNA topoisomerase II inhibitors: oxolinic acid.

(2) Mitotic inhibitors and cell division inhibitors:

(a) β-Tubulin polymerization inhibitors: benomyl, carbendazim, chlorfenazole, fuberidazole, thiabendazole, thiophanate, thiophanate-methyl, diethofencarb, zoxamide, and ethaboxam;
(b) cell division inhibitors: pencycuron; and
(c) delocalization inhibitors of spectrin-like proteins: fluopicolide.

(3) Respiration inhibitors:

(a) Complex I NADH oxidoreductase inhibitors: diflumetorim and tolfenpyrad;
(b) complex II succinic acid dehydrogenase inhibitors: benodanil, flutolanil, mepronil, isofetamido, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxan, boscalid, and pyraziflumid;
(c) complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, and mandestrobin;
(d) complex III ubiquinol reductase Qi inhibitors: cyazofamid and amisulbrom;
(e) oxidative phosphorylation uncouplers: binapacryl, meptyldinocap, dinocap, fluazinam, and ferimzone;
(f) oxidative phosphorylation inhibitors (ATP synthase inhibitors): fenthin acetate, fentin chloride, and fentin hydroxide;
(g) ATP production inhibitors: silthiofam; and
(h) complex III: Qx (unknown) inhibitors of cytochrome bc1 (ubiquinone reductase): ametoctradin.

(4) Amino acid and protein synthesis inhibitors

(a) Methionine biosynthesis inhibitors: andoprim, cyprodinil, mepanipyrim, and pyrimethanil; and
(b) protein synthesis inhibitors: blasticidin-S, kasugamycin, kasugamycin hydrochloride, streptomycin, and oxytetracycline.

(5) Signal transduction inhibitors:

(a) signal transduction inhibitors: quinoxyfen and proquinazid; and
(b) MAP/histidine kinase inhibitors in osmotic pressure signal transduction: fenpiclonil, fludioxonil, chlozolinate, iprodione, procymidone, and vinclozolin.

(6) Lipid and cell membrane synthesis inhibitors:

(a) phospholipid biosynthesis and methyltransferase inhibitors: edifenphos, iprobenfos, pyrazophos, and isoprothiolane;
(b) peroxidizing agents for lipid: biphenyl, chloroneb, dichloran, quintozene, tecnazene, tolclofos-methyl, and etridiazole;
(c) agents acting on cell membranes: iodocarb, propamocarb, propamocarb-hydrochloride, propamocarb-fosetylate, and prothiocarb;
(d) microorganisms disturbing pathogen cell membranes: Bacillus subtilis, Bacillus subtilis strain QST713, Bacillus subtilis strain FZB24, Bacillus subtilis strain MBI600, and Bacillus subtilis strain D747; and
(e) agents disturbing cell membranes: a melaleuca alternifolia (tea tree) extract.

(7) Cell membrane sterol biosynthesis inhibitors:

(a) C14 position demethylation inhibitors in sterol biosynthesis: triforine, pyrifenox, pyrisoxazole, fenarimol, flurprimidol, nuarimol, imazalil, imazalil-sulfate, oxpoconazole, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, simeconazole, tebuconazole, tetraconazole, triadimefon, triadimenol, triticonazole, prothioconazole, voriconazole, and mefentrifluconazole;
(b) $\Delta$14 reductase and $\Delta 8 \rightarrow \Delta 7$-isomerase inhibitors in sterol biosynthesis: aldimorph, dodemorph, dodemorph acetate, fenpropimorph, tridemorph, fenpropidin, piperalin, and spiroxamine;
(c) 3-keto reductase inhibitors in C4-position demethylation in a sterol biosynthesis system: fenhexamid and fenpyrazamine; and
(d) squalene epoxidase inhibitors in a sterol biosynthesis system: pyributicarb, naftifene, and terbinafine.

(8) Cell wall synthesis inhibitors

(a) Trehalase inhibitors: validamycin;
(b) chitin synthetase inhibitors: polyoxins and polyoxorim; and
(c) cellulose synthetase inhibitors: dimethomorph, flumorph, pyrimorph, benthiavalicarb, iprovalicarb, tolprocarb, valifenalate, and mandipropamide.

(9) Melanin biosynthesis inhibitors

(a) Reductase inhibitors in melanin biosynthesis: fthalide, pyroquilon, and tricyclazole; and
(b) anhydrase inhibitors in melanin biosynthesis: carpropamid, diclocymet, and fenoxanil.

(10) Host plant resistance-inducing agents:

(a) agents acting on salicylic acid synthesis pathway: acibenzolar-S-methyl; and
(b) others: probenazole, tiadinil, isotianil, laminarin, and reynoutria sachalinensis extract liquid.

(11) Agents for which the mode of activity is unclear: cymoxanil, fosetyl aluminum, phosphoric acid (phosphate), tecloftalam, triazoxide, flusulfamide, diclomezine, methasulfocarb, cyflufenamid, metrafenone, pyriofenone, dodine, a dodine free base, and flutianil.
(12) Agents having multiple activities: copper (copper salts), a bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine acetate, iminoctadine triacetate, iminoctadine trialbesilate, anilazine, dithianon, quinomethionate, and fluoroimide.
(13) Other agents: DBEDC, fluoro folpet, bis(8-quinolinolato) copper(II), propamidine, chloropicrin, cyprofuram, agrobacterium, bethoxazin, diphenylamine, methyl isothiocyanate (MITC), mildiomycin, capsaicin, curfraneb, cyprosulfamide, dazomet, debacarb, dichlorophen, difenzoquat, difenzoquat methyl sulfonate, flumetover, fosetyl-calcium, fosetyl-sodium, irumamycin, natamycin, nitrothal-isopropyl, oxamocarb, pyrrolnitrin, tebufloquin, tolnifanide, zarilamide, algophase, amicarthiazol, oxathiapiprolin, metiram zinc, benthiazole, trichlamide, uniconazole, oxyfenthiin, picarbutrazox, fenpicoxamid, dichlobentiazox, and quinofumelin.

Specific examples of the plant growth regulator that can be mixed or used in combination with the pest control agent, or the insecticidal or acaricidal agent of the present invention are shown below.
1-Methylcyclopropene, 2,3,5-triiodobenzoic acid, IAA, IBA, MCPA, MCPB, 4-CPA, 5-aminolevulinic acid hydrochloride, 6-benzylaminopurine, abscisic acid, aviglycine hydrochloride, ancymidol, butralin, calcium carbonate, calcium chloride, calcium formate, calcium peroxide, lime sulfur, calcium sulfate, chlormequat chloride, chlorpropham, choline chloride, chloroprop, cyanamide, cyclanilide, daminozide, decyl alcohol, dichlorprop, dikegulac, dimethipin, diquat, ethephon, ethychlozate, flumetralin, flurprimidol, forchlorfenuron, gibberellin A, gibberellin A3, hymexazol, inabenfide, isoprothiolane, kinetin, maleic hydrazide, mefluidide, mepiquat chloride, oxidized glutathione, paclobutrazol, pendimethalin, prohexadione-calcium, prohydrojasmon, pyraflufen-ethyl, sintofen, sodium 1-naphthaleneacetate, sodium cyanate, streptomycin, thidiazuron, triapenthenol, tribufos, trinexapac-ethyl, uniconazole P, 1-naphthylacetamide.

[Ectoparasite Control or Repellent Agent]

The ectoparasite control or repellent agent of the present invention contains at least one selected from the compounds of the present invention as an active ingredient. The ectoparasite control or repellent agent of the present invention has an excellent effect of controlling the ectoparasites that harm humans and animals.
The ectoparasites are parasitic in the body and skin of host animals, particularly warm-blooded animals and fish. Specifically, the ectoparasites are parasitic in the back, armpits, underbelly, inner thigh, and the like of the host animals, and obtain nutritional sources such as blood, dandruff from the animals to live. Examples of the ectoparasites include acari, lice, fleas, mosquitoes, stable flies, flesh flies, and Japanese fishlouse.
Examples of the host animal to be treated with the ectoparasite control or repellent agent of the present invention include warm-blooded animals such as pet animals such as dogs and cats; pet birds; domestic animals such as cattle, horses, pigs, and sheep; and domestic fowl. Other examples of the host animal include fish such as salmon, trout, puffer fish, carp, and goldfish; and insects such as honey bees, stag beetles, and rhinoceros beetles.
The ectoparasites are parasitic in and on a host animal, particularly a warm-blooded animal. Specifically, the ectoparasites are parasitic in the back, armpits, underbelly, inner thigh, and the like of the host animals, and obtain nutritional sources such as blood, dandruff from the animals to live.
The ectoparasite control or repellent agent of the present invention can be applied by a known veterinary

method (topical, oral, parenteral, or subcutaneous administration). Examples of the method include a method of orally administering tablets, capsules, mixed feeds, or the like to the animals; a method of administering to the animals by using an immersion liquid, a suppository, an injection (intramuscular, subcutaneous, intravenous, intraperitoneal, or the like), or the like; a method of topically administering by spraying, pouring-on, or spotting-on an oily or aqueous liquid preparation; a method of kneading an ectoparasite control agent in a resin, molding the kneaded product into an appropriate shape such as a collar and ear tag, and attaching and topically administering the resultant to the animals.

Specific examples of the ectoparasites that can be controlled or repelled by the ectoparasite control or repellent agent of the present invention are shown below.

(1) Acari

Mites belonging to the Dermanyssidae family, mites belonging to the Macronyssidae family, mites belonging to the Laelapidae family, mites belonging to the Varroidae family, mites belonging to the Argasidae family, mites belonging to the Ixodidae family, mites belonging to the Psoroptidae family, mites belonging to the Sarcoptidae family, mites belonging to the Knemidokoptidae family, mites belonging to the Demodixidae family, mites belonging to the Trombiculidae family, and insect-parasitic mites such as Coleopterophagus berlesei.

(2) Phthiraptera Order

Lice belonging to the Haematopinidae family, lice belonging to the Linognathidae family, chewing lice belonging to the Menoponidae family, chewing lice belonging to the Philopteridae family, and chewing lice belonging to the Trichodectidae family.

(3) Siphonaptera Order

Flea belonging to the Pulicidae family, for example, Ctenocephalides canis and Ctenocephalides felis of Ctenocephalides spp.; and
flea belonging to the Tungidae family, flea belonging to the Ceratophyllidae family, and flea belonging to the Leptopsyllidae family.

(4) Hemiptera Order

(5) Pests of Diptera order

Mosquitoes belonging to the Culicidae family, black flies belonging to the Simuliidae family, biting midges belonging to the Ceratopogonidae family, horseflies belonging to the Tabanidae family, flies belonging to the Muscidae family, and tsetse flies belonging to the Glossinidae family; flesh flies belonging to the Sarcophagidae family, flies belonging to the Hippoboscidae family, flies belonging to the Calliphoridae family, and flies belonging to the Oestridae family.

[Control Agents for Other Pests]

In addition, the control agent exhibits an excellent effect of controlling other pests that have a sting or venom that can harm humans and animals, pests carrying various pathogens and pathogenic bacteria, and pests giving unpleasant feelings to humans (toxic pests, hygiene pests, unpleasant pests, and the like).

Specific examples thereof will be shown below.

(1) Pests of Hymenoptera order

Bees belonging to the Argidae family, bees belonging to the Cynipidae family, bees belonging to the Diprionidae family, alis belonging to the Formicidae family, bees belonging to the Mutillidae family, bees belonging to the Vespidae family.

(2) Other pests

Blattodea, termites, Araneae, centipedes, millipedes, crustacea and Cimex lectularius.

[Formulation of Preparations]

Some Preparation Examples for the pest control agent, the insecticidal agent, the acaricidal agent, and the ectoparasite control or repellent agent of the present invention are shown below. However, the present invention is not limited to these Preparation Examples. "Parts" and "%" in Preparation Examples are based on mass.

The formulations of preparations for agricultural and horticultural use and for paddy rice are shown below.

(Preparation Example 1: Wettable Powder)

40 parts of the compound of the present invention, 53 parts of diatom earth, 4 parts of a fatty alcohol sulfuric acid ester, and 3 parts of an alkylnaphthalene sulfonate are uniformly mixed and finely pulverized to obtain a wettable powder containing 40% of an active ingredient.

(Preparation Example 2: Emulsion)

30 parts of the compound of the present invention, 33 parts of xylene, 30 parts of dimethylformamide, and 7 parts of a polyoxyethylene alkyl allyl ether are mixed and dissolved to obtain an emulsion containing 30% of an active ingredient.

(Preparation Example 3: Granules)

5 parts of the compound of the present invention, 40 parts of talc, 38 parts of clay, 10 parts of bentonite, and 7 parts of a sodium alkylsulfate are uniformly mixed, finely pulverized, and then granulated into a granular form having a diameter of 0.5 to 1.0 mm to obtain granules containing 5% of an active ingredient.

(Preparation Example 4: Granules)

5 parts of the compound of the present invention, 73 parts of clay, 20 parts of bentonite, 1 part of sodium dioctyl sulfosuccinate, and 1 part of potassium phosphate were mixed and pulverized, water was added thereto, and the mixture was thoroughly kneaded, followed by granulation and drying, to obtain granules containing 5% of an active ingredient.

(Preparation Example 5: Suspension)

10 parts of the compound of the present invention, 4 parts of a polyoxyethylene alkyl allyl ether, 2 parts of a sodium polycarboxylate, 10 parts of glycerin, 0.2 parts of xanthan gum, and 73.8 parts of water are mixed and wet-ground until the particle size reached 3 μm or less to obtain a suspension containing 10% of an active ingredient.

The formulations of the prescriptions of the ectoparasite control agent are shown below.

(Preparation Example 6: Granular Agent)

5 parts of the compound of the present invention are dissolved in an organic solvent to obtain a solution, the solution is sprayed onto 94 parts of kaolin and 1 part of white carbon, and then the solvent is evaporated under reduced pressure to obtain a granular agent. This type of granular agent can be mixed with animal feed and then used.

(Preparation Example 7: Injection)

0.1 to 1 part of the compound of the present invention and 99 to 99.9 parts of peanut oil are uniformly mixed and then sterilized by filtration through a sterilizing filter to obtain an injection.

(Preparation Example 8: Pour-on Agent)

5 parts of the compound of the present invention, 10 parts of a myristic acid ester, and 85 parts of isopropanol are uniformly mixed to obtain a pour-on agent.

(Preparation Example 9: Spot-on Agent)

10 to 15 parts of the compound of the present invention, 10 parts of a palmitic acid ester, and 75 to 80 parts of isopropanol are uniformly mixed to obtain a spot-on agent.

(Preparation Example 10: Spraying Agent)

1 part of the compound of the present invention, 10 parts of propylene glycol, and 89 parts of isopropanol are uniformly mixed to obtain a spraying agent.

[Examples]

(Synthesis Example for Compounds)

Next, the compound of the present invention will be described more specifically by showing Examples. However, the present invention is not limited by the following Examples.

(Example 1)

Production of 5-Carbamothioyl-N-[3-(ethylthio)-2,2-dimethylpropyl]-N,2-dimethyl-6-[4-(trifluorometh yl)phenyl]nicotinamide

(Step 1)

Synthesis of Ethyl 5-cyano-6-hydroxy-2-methylnicotinate

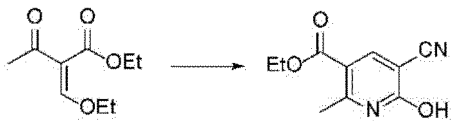

2.8 g of sodium was dissolved in 110 ml of ethanol, 10.4 g of 2-cyanoacetamide was added thereto under ice-cooling, and then 25.0 g of ethyl 2-(ethoxymethylene)-3-oxobutanoate was added to the mixture. The reaction solution was stirred overnight at room temperature, dilute hydrochloric acid was added thereto to adjust the pH to 1, and the precipitated crystals were filtered and dried to obtain 23.2 g of a target compound. Yield: 100%

$^1$H-NMR ($CD_3OD$, δppm) 8.47 (1H, s), 4.30 (2H, q), 2.67 (3H, s), 1.32 (3H, t)

(Step 2)

Synthesis of Ethyl 6-chloro-5-cyano-2-methylnicotinate

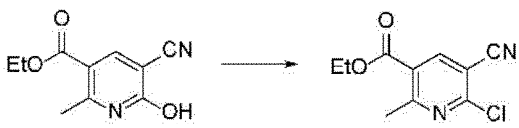

23.2 g of ethyl 5-cyano-6-hydroxy-2-methylnicotinate was suspended in 200 ml of dichloromethane, and 2.2 ml of N,N-dimethylformamide and 48 ml of oxalyl chloride were added thereto under ice-cooling, followed by heating and refluxing for 5 hours. The reaction solution was concentrated under reduced pressure, extracted with ethyl acetate, and washed with a saturated aqueous sodium hydrogen carbonate solution and saturated saline, and the organic layer was dried over anhydrous magnesium sulfate. The solvent was distilled off under reduced pressure and the obtained residue was purified by silica gel column chromatography (developing solvent: n-hexane/ethyl acetate) to obtain 8.21 g of a target compound. Yield: 32%.

$^1$H-NMR ($CDCl_3$, δppm) 8.48 (1H, s), 4.41 (2H, q), 2.89 (3H, s), 1.41 (3H, t)

(Step 3)

Synthesis of Ethyl 5-cyano-2-methyl-6-[4-(trifluoromethyl)phenyl]nicotinate

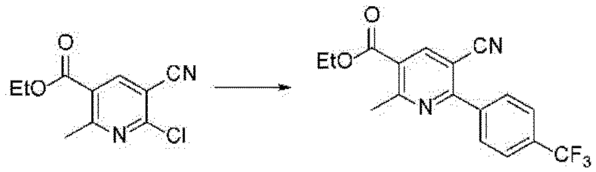

6.34 g of ethyl 6-chloro-5-cyano-2-methyl-nicotinate and 8.04 g of 4-trifluoromethylphenylboronic acid were dissolved in 140 ml of toluene and 14 ml of water, and 9.0 g of potassium carbonate and 1.0 g of dichlorobis[di-t-butyl(p-dimethylaminophenyl)phosphino]palladium (II) were added thereto, followed by heating and refluxing overnight in a nitrogen atmosphere. The reaction solution was cooled to room temperature, poured into water, and extracted with ethyl acetate. The organic layer was washed with saturated saline and then dried over anhydrous magnesium sulfate. The solvent was distilled off under reduced pressure and the obtained residue was purified by silica gel column chromatography (developing solvent: n-hexane/ethyl acetate) to obtain 8.55 g of a target compound. Yield: 91%.

$^1$H-NMR ($CDCl_3$, $\delta$ppm) 8.60 (1H, s), 8.10 (2H, d), 7.79 (2H, d), 4.43 (2H, q), 2.97 (3H, s), 1.44 (3H, t)

(Step 4)

Synthesis of 5-Cyano-2-methyl-6-[4-(trifluoromethyl)phenyl]nicotinic acid

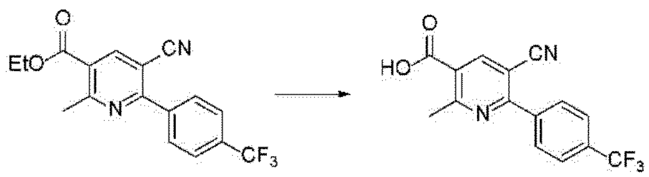

8.55 g of ethyl 5-cyano-2-methyl-6-[4-(trifluoromethyl)phenyl]nicotinate was dissolved in 43 ml of methanol, 43 ml of tetrahydrofuran, and 43 ml of water, and 2.1 g of sodium hydroxide was added thereto, followed by stirring at room temperature for 3 hours. The reaction solution was concentrated under reduced pressure, poured into dilute hydrochloric acid, and extracted with ethyl acetate. The organic layer was washed with saturated saline and then dried over anhydrous magnesium sulfate. The solvent was distilled off under reduced pressure to obtain 7.83 g of a target compound. Yield: 100%. $^1$H-NMR ($CDCl_3$, $\delta$ppm) 8.73 (1H, s), 8.13 (2H, d), 7.81 (2H, d), 3.03 (3H, s)

(Step 5)

Synthesis of tert-Butyl (3-hydroxy-2,2-dimethylpropyl)carbamate

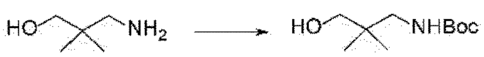

5.1 g of 3-amino-2,2-dimethylpropanol was dissolved in 49 ml of tetrahydrofuran, and 12.5 ml of di-tert-butyl dicarbonate and 49 ml of a 0.5 M aqueous sodium hydroxide solution were added thereto, followed by stirring overnight at room temperature. Thereafter, the reaction solution was added with dilute hydrochloric acid and extracted with ethyl acetate. The organic layer was washed with saturated saline and then dried over anhydrous magnesium sulfate. The solvent was distilled off under reduced pressure to obtain 10.05 g of a target compound. Yield: 100%.

$^1$H-NMR ($CDCl_3$, $\delta$ppm) 4.85 (1H, br), 3.71 (1H, t), 3.20 (2H, d), 2.97 (2H, d), 1.46 (9H, s), 0.86 (6H, s)

(Step 6)

Synthesis of 3-[(tert-Butoxycarbonyl)amino]-2,2-dimethylpropylmethanesulfonate

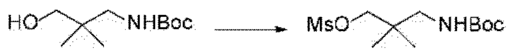

10.05 g of tertiary butyl-(3-hydroxy-2,2-dimethylpropyl)carbamate was dissolved in 100 ml of tetrahydrofuran, followed by cooling to 0°C, 10.3 ml of triethylamine and 4.6 ml of methanesulfonyl chloride were added thereto, followed by stirring at room temperature for 1 hour. The reaction solution was diluted with water and extracted with ethyl acetate. The organic layer was washed with saturated saline and then dried over anhydrous magnesium sulfate. The solvent was distilled off under reduced pressure to obtain 13.91 g of a target compound. Yield: 100%.

$^1$H-NMR ($CDCl_3$, δppm) 3.92 (2H, s), 3.06 (2H, d), 3.03 (3H, s), 1.46 (9H, s), 0.95 (6H, s)

(Step 7)

Synthesis of tert-Butyl [3-(ethylthio)-2,2-dimethylpropyl]carbamate

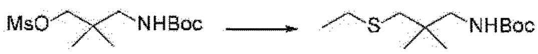

13.91 g of

3-[(tertiary-butoxycarbonyl)amino]-2,2-dimethylpropylmethanesulfonate was dissolved in 100 ml of N,N-dimethylformamide, and 12.5 g of sodium ethanethiolate was added thereto, followed by stirring overnight at room temperature. The reaction solution was diluted with water and extracted with ethyl acetate. The organic layer was washed with saturated saline and then dried over anhydrous magnesium sulfate. The solvent was distilled off under reduced pressure and the obtained residue was purified by silica gel column chromatography (developing solvent: n-hexane/ethyl acetate) to obtain 10.14 g of a target compound. Yield: 83%.

$^1$H-NMR ($CDCl_3$, δppm) 3.03 (2H, d), 2.51 (2H, q), 2.45 (2H, s), 1.46 (9H, s), 1.25 (3H, q), 0.94 (6H, s)

(Step 8)

Synthesis of 3-(Ethylthio)-2,2-dimethylpropan-1-amine hydrochloride

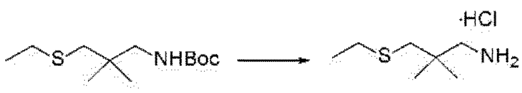

10.14 g of tertiary butyl-[3-(ethylthio)-2,2-dimethylpropyl]carbamate was dissolved in 100 ml of 1,4-dioxane, and 50 ml of a 4 M hydrogen chloride 1,4-dioxane solution was added thereto, followed by stirring overnight at room temperature. Thereafter, the reaction solution was distilled off under reduced pressure to obtain 6.04 g of a target compound. Yield: 100%.

$^1$H-NMR ($CDCl_3$, δppm) 8.40 (2H, br), 2.94 (2H, s), 2.63 (2H, s), 2.55 (2H, q), 1.25 (3H, q), 1.14 (6H, s)

(Step 9)

Synthesis of

5-Cyano-N-[3-(ethylthio)-2,2-dimethylpropyl]-2-methyl-6-[4-(trifluoromethyl)phenyl]ni cotinamide

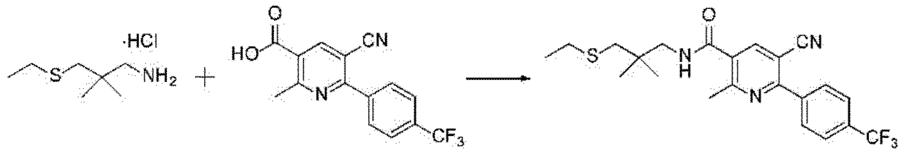

0.3 g of 5-cyano-2-methyl-6-[4-(trifluoromethyl)phenyl]nicotinic acid was dissolved in 10 ml of dichloromethane, and 0.24g of 1-(3-dimethylaminopropyl)-3-ethylcarbodiimide, 0.17g of 1-hydroxybenzotriazole, 0.19 g of 3-(ethylthio)-2,2-dimethylpropan-1-amine hydrochloride, and 0.55 ml of triethylamine were added thereto, followed by stirring overnight at room temperature. Thereafter, the reaction solution was distilled off under reduced pressure and the obtained residue was purified by silica gel column chromatography (developing solvent: n-hexane/ethyl acetate) to obtain 0.20 g of a target compound. Yield: 47%.

$^1$H-NMR ($CDCl_3$, δppm) 8.10 (1H, m), 8.07 (2H, d), 7.80 (2H, d), 6.35 (1H, t), 3.42 (2H, d), 2.80 (3H, s), 2.60-2.51 (4H, m), 1.25 (3H, t), 1.10 (6H, s)

(Step 10)

Synthesis of 5-Cyano-N-[3-(ethylthio)-2,2-dimethylpropyl]-N,2-dimethyl-6-[4-(trifluoromethyl)pheny l]nicotinamide

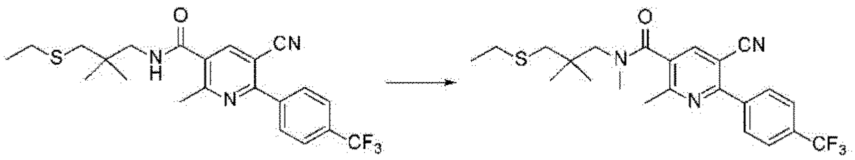

0.2 g of 5-cyano-N-[3-(ethylthio)-2,2-dimethylpropyl]-2-methyl-6-[4-(trifluoromethyl)phenyl]nic otinamide was dissolved in 5 ml of N,N-dimethylformamide, followed by cooling to 0°C. Thereafter, 20 mg of sodium hydride was added thereto, followed by stirring at 0°C for 30 minutes, and then 0.08 ml of methyl iodide was added thereto, followed by stirring at room temperature for 6 hours. Thereafter, the reaction solution was diluted with water and extracted with ethyl acetate. The organic layer was washed with saturated saline and then dried over anhydrous magnesium sulfate. The solvent was distilled off under reduced pressure and the obtained residue was purified by silica gel column chromatography (developing solvent: n-hexane/ethyl acetate) to obtain 38 mg of a target compound. Yield: 19%.

$^1$H-NMR ($CDCl_3$, δppm) 8.05 (2H, d), 7.89 (1H, s), 7.80 (2H, d), 3.57-2.35 (12H, m), 1.25 (3H, t), 1.13 (6H, s)

(Step 11)

Synthesis of

5-Carbamothioyl-N-[3-(ethylthio)-2,2-dimethylpropyl]-N,2-dimethyl-6-[4-(trifluorometh yl)phenyl]nicotinamide

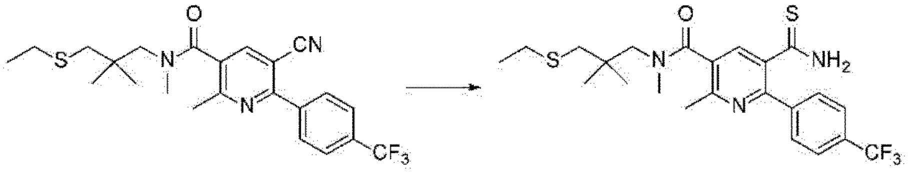

Synthesis of 5-Cyano-N-[3-(ethylthio)-2,2-dimethylpropyl]-N,2-dimethyl-6-[4-(trifluoromethyl)pheny l]nicotinamide

65 mg of 5-cyano-N-[3-(ethylthio)-2,2-dimethylpropyl]-N,2-dimethyl-6-[4-(trifluoromethyl)pheny l]nicotinamide was dissolved in 1.4 ml of N,N-dimethylformamide, and 88 mg of magnesium chloride hexahydrate and 35 mg of sodium hydrosulfide were added thereto, followed by stirring at room temperature for 1 hour. Thereafter, a saturated aqueous ammonium chloride solution was added to the reaction solution, the mixture was extracted with ethyl acetate and washed

with saturated saline, and the organic layer was dried over anhydrous magnesium sulfate. The solvent was distilled off under reduced pressure and the obtained residue was purified by silica gel column chromatography (developing solvent: n-hexane/ethyl acetate) to obtain 52 mg of a target compound. Yield: 74%.

$^1$H-NMR ($CDCl_3$, δppm) 7.81 (2H, d), 7.80 (1H, s), 7.70 (2H, d), 7.54 (1H, br), 6.81 (1H, br), 3.53-2.35 (12H, m), 1.25 (3H, t), 1.13 (6H, s)

(Example 2)

Production of 5-Cyano-N-[2,2-diethyl-4-(ethylthio)butyl]-2-methyl-6-[4-(trifluoromethyl)phenyl]nicoti namide

(Step 1)

Synthesis of 4-(Ethylthio)butanenitrile

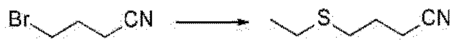

5.0 g of 4-bromobutanenitrile was dissolved in 67 ml of tetrahydrofuran, and 4.3 g of sodium ethanethiolate was added thereto, followed by stirring at room temperature overnight. Thereafter, the reaction solution was filtered and the filtrate was distilled off under reduced pressure to obtain 4.13 g of a target compound. Yield: 95%.

$^1$H-NMR ($CDCl_3$, δppm) 2.65 (2H, t), 2.55-2.48 (4H, m), 1.97-1.90 (2H, m), 1.25 (3H, t)

(Step 2)

Synthesis of 2,2-Diethyl-4-(ethylthio)butanenitrile

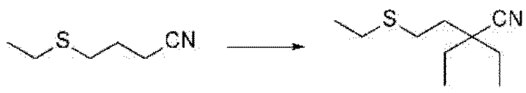

8 ml of tetrahydrofuran was added to 1.9 ml of diisopropylamine, followed by cooling to -78°C, and then 8.5 ml of a 1.6 M n-butyl lithium hexane solution was added thereto, followed by stirring at -78°C for 1 hour. Subsequently, 0.5 g of 4-(ethylthio)-butanenitrile was added thereto, followed by further stirring -78°C for 1 hour, and 1.8 ml of ethyl iodide was added thereto, followed by stirring overnight at room temperature. Thereafter, a saturated aqueous ammonium chloride solution was added to the reaction solution, the mixture was extracted with ethyl acetate and washed with saturated saline, and the organic layer was dried over anhydrous magnesium sulfate. The solvent was distilled off under reduced pressure and the obtained residue was purified by silica gel column chromatography (developing solvent: n-hexane/ethyl acetate) to obtain 0.72 g of a target compound. Yield: 100%.

$^1$H-NMR ($CDCl_3$, δppm) 2.63-2.55 (4H, m), 1.87-1.80 (2H, m), 1.65 (4H, q), 1.48 (3H, t), 1.03 (6H, t)

(Step 3)

Synthesis of 2,2-Diethyl-4-(ethylthio)butan-1-amine

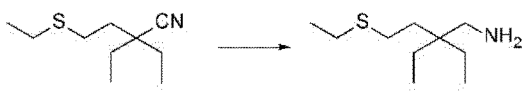

0.33 g of 2,2-diethyl-4-(ethylthio)butanenitrile was dissolved in 4 ml of tetrahydrofuran, followed by cooling to 0°C. Thereafter, 4.4 ml of a borane-tetrahydrofuran complex was added thereto, followed by stirring overnight at room temperature. A 2 M aqueous sodium hydroxide solution was added to the reaction solution, the mixture was extracted with ethyl acetate and washed with saturated saline, and the organic layer was dried over anhydrous magnesium sulfate.

Thereafter, the solvent was distilled off under reduced pressure and the crude product was used in the next step as it was.

(Step 4)

Synthesis of 5-Cyano-N-(2,2-diethyl-4-(ethylthio)butyl)-2-methyl-6-(4-(trifluoromethyl)phenyl)nicoti namide

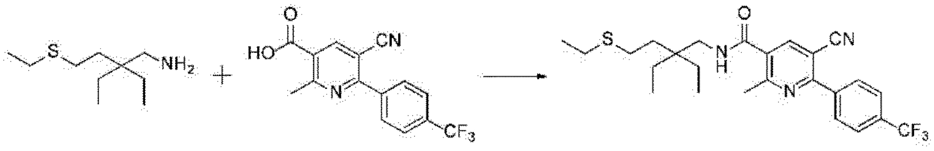

0.3 g of 5-cyano-2-methyl-6-[4-(trifluoromethyl)phenyl]nicotinic acid was dissolved in 7 ml of dichloromethane, and 0.32g of 1-(3-dimethylaminopropyl)-3-ethylcarbodiimide, 0.23 g of 1-hydroxybenzotriazole, 0.32 g of 2,2-diethyl-4-(ethylthio)butanamine, and 0.72 ml of triethylamine were added thereto, followed by stirring overnight at room temperature. Thereafter, the reaction solution was distilled off under reduced pressure and the obtained residue was purified by silica gel column chromatography (developing solvent: n-hexane/ethyl acetate) to obtain 0.29 g of a target compound. Yield: 47%.

$^1$H-NMR ($CDCl_3$, δppm) 8.10 (1H, s), 8.08 (2H, d), 7.80 (2H, d), 5.92 (1H, t), 3.37 (2H, d), 2.80 (3H, s), 2.60-2.49 (4H, m), 1.60-1.50 (2H, m), 1.34-1.20 (7H, m), 0.88 (6H, t)

(Example 3)

Production of 2-Cyano-N-[2,2-diethyl-4-(ethylthio)butyl]-4',5-bis(trifluoromethyl)-(1,1'-biphenyl)-4-ca rboxamide

(Step 1)

Synthesis of Methyl 4-amino-2-(trifluoromethyl)benzoate

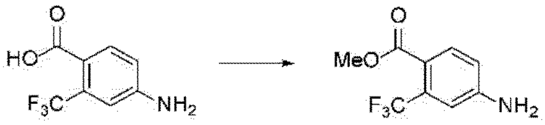

25.3 g of 4-amino-2-(trifluoromethyl)benzoic acid was dissolved in 180 ml of methanol, and 17.6 ml of thionyl chloride was added thereto under ice-cooling, followed by heating and refluxing overnight. Thereafter, the reaction solution was distilled off under reduced pressure, a saturated sodium bicarbonate solution was added thereto, the mixture was extracted with ethyl acetate, washed with saturated saline, and the organic layer was dried over anhydrous magnesium sulfate. The solvent was distilled off under reduced pressure to obtain 25.2 g of a target compound. Yield: 93%.

$^1$H-NMR ($CD_3OD$, δppm) 7.75 (1H, d), 6.97 (1H, d), 6.75 (1H, dd), 4.08-4.16 (2H, br), 3.86 (3H, s)

(Step 2)

Synthesis of Methyl 4-amino-5-iodo-2-(trifluoromethyl)benzoate

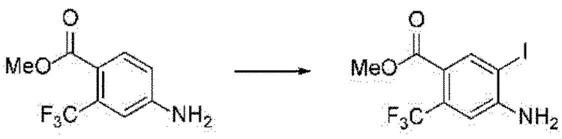

25.2 g of methyl 4-amino-2-(trifluoromethyl)benzoate was dissolved in 230 ml of acetic acid, and 25.9 g of N-iodosuccinimide was added thereto, followed by stirring at room temperature for 2 hours. Water was added to the reaction solution, and the precipitated crystals were washed with water and then dried under reduced pressure to obtain 37.1 g of a target compound. Yield: 93%.

$^1$H-NMR ($CD_3OD$, δppm) 8.24 (1H, s), 7.00 (1H, s), 4.65 (2H, s), 3.86 (3H, s)

(Step 3)

Synthesis of Methyl 4-amino-5-cyano-2-(trifluoromethyl)benzoate

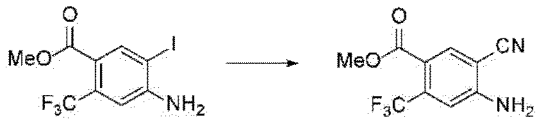

37.1 g of methyl 4-amino-5-iodo-2-(trifluoromethyl)benzoate was dissolved in 210 ml of NMP, and 11.6 g of copper (I) cyanide was added thereto, followed by stirring at 120°C for 3 hours. The reaction solution was cooled to room temperature, water was added thereto, and the precipitated crystals were washed with water and then dried under reduced pressure to obtain 23.5 g of a target compound. Yield: 89%.

$^1$H-NMR ($CD_3OD$, δppm) 8.05 (1H, s), 7.10 (1H, s), 5.02 (2H, s), 3.88 (3H, s)

(Step 4)

Synthesis of Methyl 4-bromo-5-cyano-2-(trifluoromethyl)benzoate

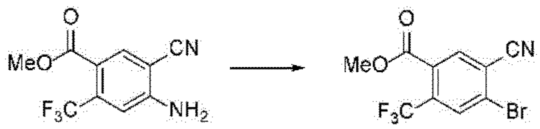

5.0 g of methyl 4-amino-5-cyano-2-(trifluoromethyl)benzoate was dissolved in 68 ml of hydrobromic acid, and 14 ml of water and 4.2 g of sodium nitrite were mixed thereto under ice-cooling, followed by stirring for 1 hour. Thereafter, 68 ml of water and 8.8 g of copper (I) bromide were mixed therewith, followed by stirring at room temperature overnight. Thereafter, aqueous ammonia was added to the reaction solution, the mixture was extracted with ethyl acetate and washed with saturated saline, and the organic layer was dried over anhydrous magnesium sulfate. The solvent was distilled off under reduced pressure and the precipitated crystals were washed with hexane to obtain 4.34 g of a target compound. Yield: 69%.

$^1$H-NMR ($CD_3OD$, δppm) 8.10 (1H, s), 8.07 (1H, s), 3.96 (3H, s)

(Step 5)

Synthesis of Methyl 2-cyano-4',5-bis(trifluoromethyl)-(1,1'-biphenyl)-4-carboxylate

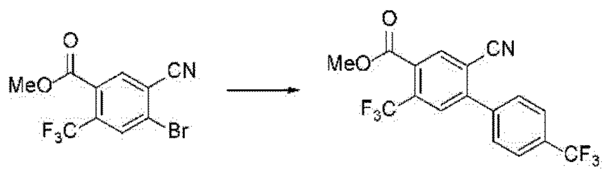

2.79 g of methyl 4-bromo-5-cyano-2-(trifluoromethyl)benzoate and 2.58 g of 4-trifluoromethylphenylboronic acid were dissolved in 30 ml of toluene and 3 ml of water, and 3.7 g of potassium carbonate and 0.32 g of dichlorobis[di-t-butyl(p-dimethylaminophenyl)phosphino]palladium (II) were added thereto, followed by heating and refluxing overnight in a nitrogen atmosphere. The reaction solution was cooled to room temperature, water was added thereto, the mixture was extracted with ethyl acetate and washed with saturated saline, and the organic layer was dried over anhydrous magnesium sulfate. The solvent was distilled off under reduced pressure and the obtained residue was purified by silica gel column chromatography (developing solvent: n-hexane/ethyl acetate) to obtain 2.58 g of a target compound. Yield: 76%.

$^1$H-NMR ($CD_3OD$, δppm) 8.25 (1H, s), 7.90 (1H, s), 7.81 (2H, d), 7.71 (2H, d), 4.00 (3H, s)

(Step 6)

Synthesis of 2-Cyano-4',5-bis(trifluoromethyl)-[1,1'-biphenyl]-4-carboxylic acid

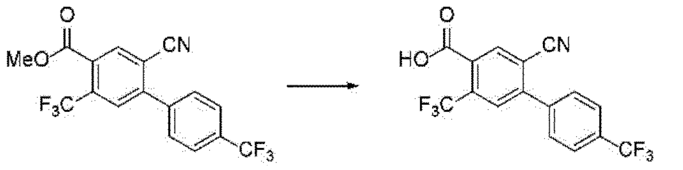

2.58 g of methyl 2-cyano-4',5-bis(trifluoromethyl)-[1',1-biphenyl]-4-carboxylate was dissolved in 12 ml of methanol, 12 ml of tetrahydrofuran, and 23 ml of water, and 0.41 g of sodium hydroxide was added thereto, followed by stirring at room temperature for 4 hours. Thereafter, dilute hydrochloric acid was added to the reaction solution, and the mixture was extracted with ethyl acetate, washed with saturated saline, and dried over anhydrous magnesium sulfate. The solvent was distilled off under reduced pressure to obtain 2.48 g of a target compound. Yield: 98%.

$^1$H-NMR ($CD_3OD$, δppm) 8.42 (1H, s), 7.97 (1H, s), 7.83 (2H, d), 7.73 (2H, d)

(Step 7)

Synthesis of 2-Cyano-N-[2,2-diethyl-4-(ethylthio)butyl]-4',5-bis(trifluoromethyl)-(1,1-biphenyl)-4-ca rboxamide

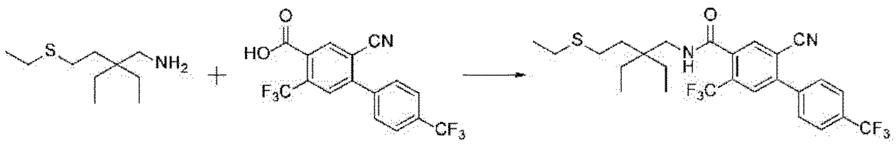

2-Cyano-4',5-bis(trifluoromethyl)-(1',1-biphenyl)-4-carboxylic acid was dissolved in 6 ml of dichloromethane, and 0.28 g of 1-(3-dimethylaminopropyl)-3-ethylcarbodiimide, 0.19 g of 1-hydroxybenzotriazole, 0.27 g of 2,2-diethyl-4-(ethylthio)butanamine, and 0.62 ml of triethylamine were added thereto, followed by stirring overnight at room temperature. Thereafter, the reaction solution was distilled off under reduced pressure and the obtained residue was purified by silica gel column chromatography (developing solvent: n-hexane/ethyl acetate) to obtain 0.47 g of a target compound. Yield: 80%.

$^1$H-NMR ($CDCl_3$, δppm) 7.95 (1H, s), 7.85 (1H, s), 7.80 (2H, d), 7.70 (2H, d), 5.83 (1H, t), 3.37 (2H, d), 2.60-2.45 (4H, m), 1.60-1.50 (2H, m), 1.34-1.20 (7H, m), 0.86 (6H, t)

Some of the compounds of the present invention produced by the same method as in the above-described Examples are shown in Table 1, Table 2, Table 3, and Table 4. Table 1 shows the combinations of Y, $R^1$, $R^{2a}$, $R^{2b}$, $X^1$, $X^2$, $X^3$, and $(X^5)_n$ in Formula (I-1) and the physical properties of the compounds exhibited by the combinations. Table 2 shows the combinations of Y, $R^1$, $R^{2a}$, $R^{2b}$, $X^1$, $X^2$, $X^3$, and $(X^5)_n$ in Formula (I-1) and the physical properties of the compounds exhibited by the combinations. Table 3 shows the combinations of Y, $R^1$, $R^{2a}$, $R^{2b}$, $X^1$, $X^2$, $X^3$, and $(X^5)_n$ in Formula (I-2) and the physical properties of the compounds exhibited by the combinations. Table 4 shows the combinations of Y, $R^1$, $R^{2a}$, $R^{2b}$, $X^1$, $X^2$, $X^3$, and $(X^5)_n$ in Formula (I-3) and the physical properties of the compounds exhibited by the combinations. n in Formulae (I-1), (I-2), and (I-3) represents the number of the substituents $X^5$'s on the phenyl group. The melting point (m.p.) or the properties are shown in the column of the physical properties. In the tables, Ph represents a phenyl group, Me represents a methyl group, Et represents an ethyl group, $^n$Pr represents a normal propyl

group, ᶜPr represents a cyclopropyl group, and ⁱPr represents an isopropyl group.

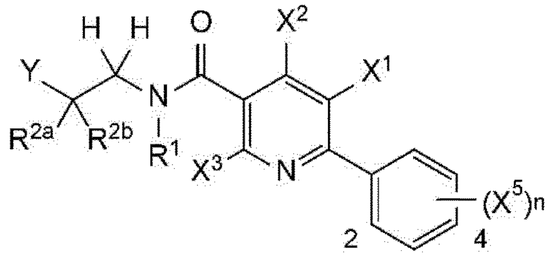

(I-1)

[Table 1]

Table 1

| No. | Y | $R^{2a}$ | $R^{2b}$ | $R^1$ | $X^1$ | $X^2$ | $X^3$ | $(X^5)n$ | Physical properties |
|---|---|---|---|---|---|---|---|---|---|
| A-1 | $EtSCH_2CH_2$ | Me | Me | Me | CN | H | $CH_2OMe$ | 2-OMe, 4-CNOEt | viscous oil |
| A-2 | $EtSCH_2CH_2$ | Me | Me | Me | CN | H | $CH_2OMe$ | 2-OMe, 4-$OCF_3$ | viscous oil |
| A-3 | $EtSCH_2CH_2$ | Me | Me | Me | CN | H | $CH_2OMe$ | 4-$OCHF_2$ | viscous oil |
| A-4 | $EtSCH_2CH_2$ | Me | Me | Me | CN | H | $CH_2OMe$ | 4-$OCF_3$ | viscous oil |
| A-5 | $EtSCH_2CH_2$ | Et | Et | H | CN | H | Me | 2-OMe, 4-CNOEt | amorphous |
| A-6 | $EtSCH_2CH_2$ | Et | Et | H | CN | H | $CH_2OMe$ | 2-OMe, 4-CNOEt | 111 to 112 |
| A-7 | $EtSCH_2CH_2$ | Et | Et | H | CN | H | $CH_2OMe$ | 2-OMe, 4-$OCF_3$ | 90 to 91 |
| A-8 | $EtSCH_2CH_2$ | Et | Et | H | CN | H | $CH_2OMe$ | 4-$OCHF_2$ | viscous oil |
| A-9 | $EtSCH_2CH_2$ | Et | Et | H | CN | H | $CH_2OMe$ | 4-$OCF_3$ | viscous oil |
| A-10 | $EtSO_2CH_2CH_2$ | $^n$Pr | $^n$Pr | H | CN | H | Me | 2-OMe, 4-$OCF_3$ | amorphous |
| A-11 | $EtS(=O)CH_2CH_2$ | $^n$Pr | $^n$Pr | H | CN | H | Me | 2-OMe, 4-$OCF_3$ | amorphous |
| A-12 | $EtSCH_2CH_2$ | $^n$Pr | $^n$Pr | H | CN | H | Me | 2-OMe, 4-$OCF_3$ | viscous oil |
| A-13 | $EtSO_2CH_2CH_2$ | $^n$Pr | $^n$Pr | H | CN | H | Me | 4-$CF_3$ | amorphous |
| A-14 | $EtS(=O)CH_2CH_2$ | $^n$Pr | $^n$Pr | H | CN | H | Me | 4-$CF_3$ | amorphous |
| A-15 | $EtSCH_2CH_2$ | $^n$Pr | $^n$Pr | H | CN | H | Me | 4-$CF_3$ | 84 to 86 |
| A-16 | $EtSO_2CH_2CH_2$ | Et | Et | H | CN | H | $CHF_2$ | 2-OMe, 4-$OCF_3$ | amorphous |
| A-17 | $EtS(=O)CH_2CH_2$ | Et | Et | H | CN | H | $CHF_2$ | 2-OMe, 4-$OCF_3$ | amorphous |
| A-18 | $EtSCH_2CH_2$ | Et | Et | H | CN | H | $CHF_2$ | 2-OMe, 4-$OCF_3$ | 68 to 70 |
| A-19 | $EtSO_2CH_2CH_2$ | Et | Et | H | CN | H | Et | 4-$OCHF_2$ | amorphous |
| A-20 | $EtS(=O)CH_2CH_2$ | Et | Et | H | CN | H | Et | 4-$OCHF_2$ | amorphous |

[Table 2]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-21 | $EtSCH_2CH_2$ | Et | Et | H | CN | H | Et | 4-$OCHF_2$ | 70 to 72 |
| A-22 | $EtSO_2CH_2CH_2$ | Et | Et | H | CN | H | Me | 4-$OCHF_2$ | amorphous |
| A-23 | $EtS(=O)CH_2CH_2$ | Et | Et | H | CN | H | Me | 4-$OCHF_2$ | amorphous |
| A-24 | $EtSCH_2CH_2$ | Et | Et | H | CN | H | Me | 4-$OCHF_2$ | 68 to 70 |
| A-25 | $EtSO_2CH_2CH_2$ | Et | Et | H | CN | H | Me | 2-OMe, 4-$OCF_3$ | amorphous |
| A-26 | $EtS(=O)CH_2CH_2$ | Et | Et | H | CN | H | Me | 2-OMe, 4-$OCF_3$ | amorphous |
| A-27 | $EtSCH_2CH_2$ | Et | Et | H | CN | H | Me | 2-OMe, 4-$OCF_3$ | amorphous |
| A-28 | $EtSO_2CH_2CH_2$ | Me | Me | Me | CN | H | Me | 2-OMe, 4-$OCF_3$ | amorphous |
| A-29 | $EtS(=O)CH_2CH_2$ | Me | Me | Me | CN | H | Me | 2-OMe, 4-$OCF_3$ | amorphous |
| A-30 | $EtSCH_2CH_2$ | Me | Me | Me | CN | H | Me | 2-OMe, 4-$OCF_3$ | 119 to 121 |
| A-31 | $EtSO_2CH_2CH_2$ | Et | Et | H | CN | H | Me | 2-OEt, 4-$CF_3$ | amorphous |
| A-32 | $EtS(=O)CH_2CH_2$ | Et | Et | H | CN | H | Me | 2-OEt, 4-$CF_3$ | amorphous |
| A-33 | $EtSCH_2CH_2$ | Et | Et | H | CN | H | Me | 2-OEt, 4-$CF_3$ | amorphous |
| A-34 | $EtSO_2CH_2CH_2$ | Et | Et | H | CN | H | Me | 2-OMe, 4-$CF_3$ | amorphous |
| A-35 | $EtS(=O)CH_2CH_2$ | Et | Et | H | CN | H | Me | 2-OMe, 4-$CF_3$ | amorphous |
| A-36 | $EtSCH_2CH_2$ | Et | Et | H | CN | H | Me | 2-OMe, 4-$CF_3$ | 110 to 111 |
| A-37 | $EtSO_2CH_2CH_2$ | Me | Me | Me | CN | H | Me | 2-OEt, 4-$CF_3$ | 193 to 195 |
| A-38 | $EtS(=O)CH_2CH_2$ | Me | Me | Me | CN | H | Me | 2-OEt, 4-$CF_3$ | 155 to 157 |
| A-39 | $EtSCH_2CH_2$ | Me | Me | Me | CN | H | Me | 2-OEt, 4-$CF_3$ | 139 to 140 |
| A-40 | $EtSO_2CH_2CH_2$ | Me | Me | Me | CN | H | Me | 2-OMe, 4-$CF_3$ | 209 to 210 |

[Table 3]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-41 | $EtS(=O)CH_2CH_2$ | Me | Me | Me | CN | H | Me | 2-OMe, 4-$CF_3$ | 175 to 177 |
| A-42 | $EtSCH_2CH_2$ | Me | Me | Me | CN | H | Me | 2-OMe, 4-$CF_3$ | 175 to 176 |
| A-43 | 3-$EtSCH_2$-Py-2-yl | Me | Me | Me | CN | H | Me | 4-$CF_3$ | amorphous |
| A-44 | 3-$EtSCH_2$-Py-2-yl | Me | Me | H | CN | H | Me | 4-$CF_3$ | 105 to 106 |
| A-45 | $EtSO_2CH_2CH_2$ | Et | Et | H | CN | H | Me | 4-$OCF_3$ | amorphous |
| A-46 | $EtSCH_2CH_2$ | Me | Me | Me | CN | H | Me | 4-$CF_3$ | viscous oil |
| A-47 | $EtSO_2CH_2CH_2$ | Et | Et | Me | CN | H | Me | 2-F, 4-$CF_3$ | 46 to 48 |
| A-48 | $EtS(=O)CH_2CH_2$ | Et | Et | Me | CN | H | Me | 2-F, 4-$CF_3$ | 38 to 40 |
| A-49 | $EtSCH_2CH_2$ | Et | Et | Me | CN | H | Me | 2-F, 4-$CF_3$ | viscous oil |

(continued)

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-50 | $EtSO_2CH_2CH_2$ | Et | Et | Me | CN | H | Me | 4-$OCF_3$ | 42 to 45 |
| A-51 | $EtS(=O)CH_2CH_2$ | Et | Et | Me | CN | H | Me | 4-$OCF_3$ | viscous oil |
| A-52 | $EtSCH_2CH_2$ | Et | Et | Me | CN | H | Me | 4-$OCF_3$ | viscous oil |
| A-53 | $EtSO_2CH_2CH_2$ | Et | Et | H | CN | H | Me | 2-F, 4-$OCF_3$ | 49 to 51 |
| A-54 | $EtS(=O)CH_2CH_2$ | Et | Et | H | CN | H | Me | 2-F, 4-$OCF_3$ | 53 to 55 |
| A-55 | $EtSCH_2CH_2$ | Et | Et | H | CN | H | Me | 2-F, 4-$OCF_3$ | viscous oil |
| A-56 | $EtSO_2CH_2CH_2$ | Me | Me | Me | CN | H | Me | 2-F, 4-$OCF_3$ | 44 to 46 |
| A-57 | $EtS(=O)CH_2CH_2$ | Me | Me | Me | CN | H | Me | 2-F, 4-$OCF_3$ | 36 to 38 |
| A-58 | $EtSCH_2CH_2$ | Me | Me | Me | CN | H | Me | 2-F, 4-$OCF_3$ | viscous oil |
| A-59 | $EtSCH_2CH_2$ | Et | Et | Me | CN | H | $CF_3$ | 4-$CF_3$ | amorphous |
| A-60 | $EtS(=O)CH_2CH_2$ | Et | Et | H | CN | H | Me | 2-F, 4-$OCH_2CF_2CHF_2$ | 65 to 67 |

[Table 4]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-61 | $EtSCH_2CH_2$ | Et | Et | H | CN | H | Me | 2-F, 4-$OCH_2CF_2CHF_2$ | 90 to 92 |
| A-62 | $EtS(=O)CH_2CH_2$ | Et | Et | H | CN | H | Me | 4-$CF_3$ | 55 to 57 |
| A-63 | $EtS(=O)CH_2CH_2$ | Et | Et | H | CN | H | $CH_2OMe$ | 2-F, 4-$OCH_2CF_2CHF_2$ | amorphous |
| A-64 | $EtS(=O)CH_2CH_2$ | Et | Et | H | CN | H | Me | 2-F, 4-$CF_3$ | 55 to 57 |
| A-65 | $EtS(=O)CH_2CH_2$ | Et | Et | H | CN | H | $CF_3$ | 4-$CF_3$ | 61 to 63 |
| A-66 | $EtS(=O)CH_2CH_2$ | Et | Et | H | CN | H | Me | 4-$OCH_2CF_2CHF_2$ | 53 to 55 |
| A-67 | $EtS(=O)CH_2CH_2$ | Et | Et | H | CN | H | Me | 4-$OCF_3$ | amorphous |
| A-68 | $EtSCH_2CH_2$ | Et | Et | H | CN | H | $CH_2OMe$ | 2-F, 4-$OCH_2CF_2CHF_2$ | amorphous |
| A-69 | $EtSCH_2CH_2$ | Et | Et | H | CN | H | Me | 2-F, 4-$CF_3$ | amorphous |
| A-70 | $EtSCH_2CH_2$ | Et | Et | H | CN | H | $CF_3$ | 4-$CF_3$ | 113 to 114 |
| A-71 | $EtSCH_2CH_2$ | Et | Et | H | CN | H | Me | 4-$OCH_2CF_2CHF_2$ | 96 to 97 |
| A-72 | $EtSCH_2CH_2$ | Et | Et | H | CN | H | Me | 4-$OCF_3$ | 86 to 87 |
| A-73 | $EtSO_2CH_2CH_2$ | Me | Me | Me | CN | H | $CH_2OMe$ | 2-F, 4-$OCH_2CF_2CHF_2$ | amorphous |
| A-74 | $EtS(=O)CH_2CH_2$ | Me | Me | Me | CN | H | $CH_2OMe$ | 2-F, 4-$OCH_2CF_2CHF_2$ | 145 to 146 |
| A-75 | $EtSCH_2CH_2$ | Me | Me | Me | CN | H | $CH_2OMe$ | 2-F, 4-$OCH_2CF_2CHF_2$ | 78 to 79 |
| A-76 | 3-EtS-Py-2-yl | Me | Me | Me | CN | H | Me | 4-$OCH_2CF_2CHF_2$ | amorphous |
| A-77 | 3-EtS-Py-2-yl | Me | Me | Me | CN | H | Me | 4-$CF_3$ | amorphous |
| A-78 | $EtSCH_2CH_2$ | Et | Et | H | CN | H | Me | 4-$CF_3$ | 97 to 99 |

(continued)

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-79 | $EtSO_2CH_2CH_2$ | Me | Me | Me | CN | H | Me | 2-F, 4-$OCH_2CF_2CHF_2$ | 46 to 48 |
| A-80 | $EtS(=O)CH_2CH_2$ | Me | Me | Me | CN | H | Me | 2-F, 4-$OCH_2CF_2CHF_2$ | 153 to 154 |

[Table 5]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-81 | $EtSCH_2CH_2$ | Me | Me | Me | CN | H | Me | 2-F, 4-$OCH_2CF_2CHF_2$ | viscous oil |
| A-82 | 3-F-Py-2-yl | Me | Me | Me | CN | H | Me | 4-$CF_3$ | 187 to 189 |
| A-83 | 3-EtS-Py-2-yl | Me | Me | H | CN | H | Me | 4-$CF_3$ | 147 to 148 |
| A-84 | (4-F-Ph)-C(=N-OEt) | Me | Me | H | CN | H | Me | 4-$CF_3$ | 175 to 177 |
| A-85 | (4-F-Ph)-C(=N-OMe) | Me | Me | H | CN | H | Me | 4-$CF_3$ | 201 to 204 |
| A-86 | (4-F-Ph)-C(=O) | Me | Me | H | CN | H | Me | 4-$CF_3$ | 132 to 133 |
| A-87 | (4-F-Ph)-CH(OH) | Me | Me | H | CN | H | Me | 4-$CF_3$ | 80 to 82 |
| A-88 | $EtSO_2CH_2CH_2$ | Me | Me | Me | CN | H | Me | 4-$OCH_2CF_2CHF_2$ | viscous oil |
| A-89 | $EtS(=O)CH_2CH_2$ | Me | Me | Me | CN | H | Me | 4-$OCH_2CF_2CHF_2$ | viscous oil |
| A-90 | 3-F-Py-2-yl | Me | Me | H | CN | H | Me | 4-$CF_3$ | 189 to 190 |
| A-91 | $EtSO_2CH_2CH_2$ | Et | Et | Me | CN | H | Me | 4-$OCHF_2$ | amorphous |
| A-92 | $EtS(=O)CH_2CH_2$ | Et | Et | Me | CN | H | Me | 4-$OCHF_2$ | viscous oil |
| A-93 | $EtSCH_2CH_2$ | Et | Et | Me | CN | H | Me | 4-$OCHF_2$ | viscous oil |
| A-94 | $EtSO_2CH_2CH_2$ | $-(CH_2)_5-$ | | Me | CN | H | Me | 4-$OCHF_2$ | amorphous |
| A-95 | $EtS(=O)CH_2CH_2$ | $-(CH_2)_5-$ | | Me | CN | H | Me | 4-$OCHF_2$ | amorphous |
| A-96 | $EtSCH_2CH_2$ | $-(CH_2)_5-$ | | Me | CN | H | Me | 4-$OCHF_2$ | amorphous |
| A-97 | $EtSCH_2CH_2$ | Me | Me | Me | CN | H | $CF_2CF_3$ | 4-$CF_3$ | viscous oil |
| A-98 | $EtSCH_2CH_2$ | Me | Me | Me | CN | H | $CH_2OMe$ | 2-F, 4-$CF_3$ | viscous oil |
| A-99 | $EtSCH_2CH_2$ | Me | Me | Me | CN | H | $CF_3$ | 4-$OCH_2CF_2CHF_2$ | viscous oil |
| A-100 | $EtSCH_2CH_2$ | Me | Me | Me | CN | H | $CF_3$ | 4-$OCF_2CHF_2$ | viscous oil |

[Table 6]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | X3 | $(X^5)n$ | Physical properties |
| A-101 | $EtSCH_2CH_2$ | Me | Me | Me | CN | H | $CF_3$ | 4-$OCF_3$ | viscous oil |
| A-102 | $EtSCH_2CH_2$ | Me | Me | Me | CN | H | Me | 4-$SCF_3$ | viscous oil |
| A-103 | $EtSCH_2CH_2$ | Me | Me | Me | CN | H | Me | 2-SH, 4-$CF_3$ | 168 to 169 |
| A-104 | $EtSCH_2CH_2$ | Me | Me | Me | CN | H | Me | 2-F, 4-$CF_3$ | 92 to 94 |
| A-105 | $EtSCH_2CH_2$ | Me | Me | Me | CN | H | Me | 4-$CF(CF_3)_2$ | viscous oil |
| A-106 | $EtSCH_2CH_2$ | Me | Me | Me | CN | H | $CF_3$ | 4-$OCHF_2$ | viscous oil |
| A-107 | $EtSCH_2CH_2$ | Me | Me | Me | CN | H | Et | 4-$OCHF_2$ | viscous oil |
| A-108 | $EtSCH_2CH_2$ | Me | Me | Me | CN | H | Me | 4-$OCH_2CF_2CHF_2$ | viscous oil |
| A-109 | $EtSCH_2CH_2$ | Me | Me | Me | CN | H | Me | 4-$OCF_2CHF_2$ | viscous oil |
| A-110 | $EtSCH_2CH_2$ | Me | Me | Me | CN | H | $CF_3$ | 4-$CF_3$ | amorphous |
| A-111 | $EtSO_2CH_2CH_2$ | $-(CH_2)_2-$ | | Me | CN | H | Me | 4-$OCHF_2$ | amorphous |
| A-112 | $EtS(=O)CH_2CH_2$ | $-(CH_2)_2-$ | | Me | CN | H | Me | 4-$OCHF_2$ | amorphous |
| A-113 | $EtSCH_2CH_2$ | $-(CH_2)_2-$ | | Me | CN | H | Me | 4-$OCHF_2$ | 98 to 100 |
| A-114 | (4-F-Ph)$CH_2$ | Me | Me | Me | CN | H | Me | 4-$OCHF_2$ | viscous oil |
| A-115 | $EtSO_2CH_2CH_2$ | Me | Me | Me | CN | H | Me | 4-$OCHF_2$ | amorphous |
| A-116 | $EtS(=O)CH_2CH_2$ | Me | Me | Me | CN | H | Me | 4-$OCHF_2$ | amorphous |
| A-117 | (4-F-Ph)$CH_2$ | Me | Me | Me | CN | H | Me | 4-$CF_3$ | amorphous |
| A-118 | $EtSCH_2CH_2$ | Me | Me | Me | CN | H | Me | 4-$OCF_3$ | viscous oil |
| A-119 | (Py-2-yl)$CH_2$ | Me | Me | Me | CN | H | Me | 4-$OCHF_2$ | viscous oil |
| A-120 | (Py-2-yl)$CH_2$ | Me | Me | Me | CN | H | Me | 4-$CF_3$ | viscous oil |

[Table 7]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-121 | $EtSCH_2CH_2$ | Me | Me | Me | CN | H | Me | 4-$OCHF_2$ | amorphous |
| A-122 | $EtSCH_2CH_2$ | Me | Me | Me | CN | H | Me | 4-$CF_3$ | amorphous |
| A-123 | $^iPrSCH_2$ | Me | Me | Me | CN | H | Me | 4-$CF_3$ | viscous oil |
| A-124 | $^nPrSCH_2$ | Me | Me | Me | CN | H | Me | 4-$CF_3$ | viscous oil |
| A-125 | $MeSCH_2$ | Me | Me | Me | CN | H | Me | 4-$CF_3$ | viscous oil |
| A-126 | $EtS(=O)CH_2$ | Me | Me | Me | CN | H | Me | 4-$OCF_2CHF_2$ | viscous oil |
| A-127 | $EtSCH_2$ | Me | Me | Me | CN | H | Me | 4-$OCF_2CHF_2$ | viscous oil |
| A-128 | $EtS(=O)CH_2$ | Me | Me | Me | CN | H | Me | 4-$OCH_2CF_2CHF_2$ | viscous oil |
| A-129 | $EtSCH_2$ | Me | Me | Me | CN | H | Me | 4-$OCH_2CF_2CHF_2$ | viscous oil |

(continued)

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-130 | $EtS(=O)CH_2$ | Me | Me | Me | CN | H | Me | $4\text{-}OCF_2CHFCF_3$ | viscous oil |
| A-131 | $EtSCH_2$ | Me | Me | Me | CN | H | Me | $4\text{-}OCF_2CHFCF_3$ | viscous oil |
| A-132 | $EtSO_2CH_2$ | Me | Me | Me | CN | H | Me | $4\text{-}OCHF_2$ | viscous oil |
| A-133 | $EtS(=O)CH_2$ | Me | Me | Me | CN | H | Me | $4\text{-}OCHF_2$ | viscous oil |
| A-134 | 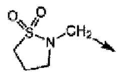 | Me | Me | H | CN | H | Me | $4\text{-}CF_3$ | 168 to 169 |
| A-135 | $EtSCH_2$ | Me | Me | Me | CN | H | $CF_3$ | $4\text{-}CF_3$ | viscous oil |
| A-136 | $EtSCH_2$ | Me | Me | Me | CN | H | Me | $4\text{-}OCHF_2$ | viscous oil |
| A-137 | $EtSCH_2$ | Me | Me | Me | CN | H | Me | $4\text{-}OCF_3$ | viscous oil |
| A-138 | (5-Me-1,2,4-oxadiazol-3-yl)$CH_2$ | Me | Me | Me | CN | H | Me | $4\text{-}CF_3$ | viscous oil |
| A-139 | (5-Me-1,2,4-oxadiazol-3-yl)$CH_2$ | Me | Me | H | CN | H | Me | $4\text{-}CF_3$ | 141 to 142 |
| A-140 | $EtSO_2CH_2$ | Me | Me | Me | CN | H | Me | $4\text{-}CF_3$ | viscous oil |

[Table 8]

| Table 1 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| A-141 | $EtS(=O)CH_2$ | Me | Me | Me | CN | H | Me | $4\text{-}CF_3$ | viscous oil |
| A-142 | $EtSCH_2$ | Me | Me | $CH_2C{\equiv}CH$ | CN | H | Me | $4\text{-}CF_3$ | amorphous |
| A-143 | $EtSCH_2$ | Me | Me | $CH_2{}^cPr$ | CN | H | Me | $4\text{-}CF_3$ | viscous oil |
| A-144 | $EtSCH_2$ | Me | Me | $^nPr$ | CN | H | Me | $4\text{-}CF_3$ | viscous oil |
| A-145 | $EtSCH_2$ | Me | Me | Et | CN | H | Me | $4\text{-}CF_3$ | viscous oil |
| A-146 | $EtSCH_2$ | Me | Me | Me | CN | H | Me | $4\text{-}CF_3$ | amorphous |
| A-147 | $EtSCH_2$ | Me | Me | H | CN | H | Me | $4\text{-}CF_3$ | 172 to 173 |

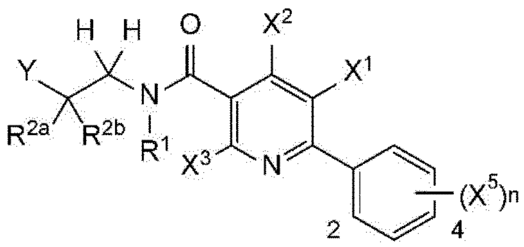

(I－1)

[Table 9]

| Table 2 | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| B-1 | $EtS(=O)CH_2CH_2$ | Me | Me | Me | C(=S) $NH_2$ | H | $CH_2OMe$ | 2-F, 4-$OCH_2CF_2CHF_2$ | 60 to 63 |
| B-2 | $EtSCH_2CH_2$ | Me | Me | Me | C(=S) $NH_2$ | H | $CH_2OMe$ | 2-F, 4-$OCH_2CF_2CHF_2$ | 160 to 161 |
| B-3 | $EtSO_2CH_2CH_2$ | Me | Me | Me | C(=S) $NH_2$ | H | Me | 4-$OCH_2CF_2CHF_2$ | 83 to 85 |
| B-4 | $EtS(=O)CH_2CH_2$ | Me | Me | Me | C(=S) $NH_2$ | H | Me | 4-$OCH_2CF_2CHF_2$ | 88 to 90 |
| B-5 | $EtS(=O)CH_2CH_2$ | Et | Et | Me | C(=S) $NH_2$ | H | Me | 4-$OCHF_2$ | 121 to 122 |
| B-6 | $EtSO_2CH_2CH_2$ | $-(CH_2)_5-$ | | Me | C(=S) $NH_2$ | H | Me | 4-$OCHF_2$ | 92 to 95 |
| B-7 | $EtS(=O)CH_2CH_2$ | $-(CH_2)_5-$ | | Me | C(=S) $NH_2$ | H | Me | 4-$OCHF_2$ | 80 to 83 |
| B-8 | $EtSCH_2CH_2$ | $-(CH_2)_5-$ | | Me | C(=S) $NH_2$ | H | Me | 4-$OCHF_2$ | 73 to 75 |
| B-9 | $EtSCH_2CH_2$ | Me | Me | Me | C(=S) $NH_2$ | H | $CF_2CF_3$ | 4-$CF_3$ | 208 to 209 |
| B-10 | $EtSCH_2CH_2$ | Me | Me | Me | C(=S) $NH_2$ | H | $CH_2OMe$ | 2-F, 4-$CF_3$ | 112 to 115 |
| B-11 | $EtSCH_2CH_2$ | Me | Me | Me | C(=S) $NH_2$ | H | $CF_3$ | 4-$OCH_2CF_2CHF_2$ | 61 to 63 |
| B-12 | $EtSCH_2CH_2$ | Me | Me | Me | C(=S) $NH_2$ | H | $CF_3$ | 4-$OCF_2CHF_2$ | 191 to 192 |
| B-13 | $EtSCH_2CH_2$ | Me | Me | Me | C(=S) $NH_2$ | H | $CF_3$ | 4-$OCF_3$ | 174 to 175 |
| B-14 | $EtSCH_2CH_2$ | Me | Me | Me | C(=S) $NH_2$ | H | Me | 4-$SCF_3$ | 136 to 137 |
| B-15 | $EtSCH_2CH_2$ | Me | Me | Me | C(=S) $NH_2$ | H | Me | 2-F, 4-$CF_3$ | 179 to 181 |
| B-16 | $EtSCH_2CH_2$ | Me | Me | Me | C(=S) $NH_2$ | H | Me | 4-$CF(CF_3)_2$ | 166 to 169 |
| B-17 | $EtSCH_2CH_2$ | Me | Me | Me | C(=S) $NH_2$ | H | $CF_3$ | 4-$OCHF_2$ | 151 to 152 |
| B-18 | $EtSCH_2CH_2$ | Me | Me | Me | C(=S) $NH_2$ | H | Et | 4-$OCHF_2$ | 140 to 142 |
| B-19 | $EtSCH_2CH_2$ | Me | Me | Me | C(=S) $NH_2$ | H | Me | 4-$OCH_2CF_2CHF_2$ | 68 to 70 |
| B-20 | $EtSCH_2CH_2$ | Me | Me | Me | C(=S) $NH_2$ | H | Me | 4-$OCF_2CHF_2$ | 72 to 74 |

[Table 10]

| Table 2 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| B-21 | $EtSCH_2CH_2$ | Me | Me | Me | $C(=S)NH_2$ | H | $CF_3$ | $4\text{-}CF_3$ | 190 to 191 |
| B-22 | $EtSO_2CH_2CH_2$ | $-(CH_2)_2-$ | | Me | $C(=S)NH_2$ | H | Me | $4\text{-}OCHF_2$ | 62 to 64 |
| B-23 | $EtS(=O)CH_2CH_2$ | $-(CH_2)_2-$ | | Me | $C(=S)NH_2$ | H | Me | $4\text{-}OCHF_2$ | 88 to 91 |
| B-24 | $EtSCH_2CH_2$ | $-(CH_2)_2-$ | | Me | $C(=S)NH_2$ | H | Me | $4\text{-}OCHF_2$ | 70 to 72 |
| B-25 | $(4\text{-}F\text{-}Ph)CH_2$ | Me | Me | Me | $C(=S)NH_2$ | H | Me | $4\text{-}OCHF_2$ | 91 to 95 |
| B-26 | $EtSO_2CH_2CH_2$ | Me | Me | Me | $C(=S)NH_2$ | H | Me | $4\text{-}OCHF_2$ | amorphous |
| B-27 | $EtS(=O)CH_2CH_2$ | Me | Me | Me | $C(=S)NH_2$ | H | Me | $4\text{-}OCHF_2$ | amorphous |
| B-28 | $(4\text{-}F\text{-}Ph)CH_2$ | Me | Me | Me | $C(=S)NH_2$ | H | Me | $4\text{-}CF_3$ | amorphous |
| B-29 | $EtSCH_2CH_2$ | Me | Me | Me | $C(=S)NH_2$ | H | Me | $4\text{-}OCF_3$ | 140 to 142 |
| B-30 | $(Py\text{-}2\text{-}yl)CH_2$ | Me | Me | Me | $C(=S)NH_2$ | H | Me | $4\text{-}CF_3$ | 223 to 225 |
| B-31 | $(Py\text{-}2\text{-}yl)CH_2$ | Me | Me | Me | $C(=S)NH_2$ | H | Me | $4\text{-}OCHF_2$ | 209 to 212 |
| B-32 | $EtSCH_2CH_2$ | Me | Me | Me | $C(=S)NH_2$ | H | Me | $4\text{-}OCHF_2$ | 49 to 52 |
| B-33 | $EtSCH_2CH_2$ | Me | Me | Me | $C(=S)NH_2$ | H | Me | $4\text{-}CF_3$ | 84 to 87 |
| B-34 | $^iPrSCH_2$ | Me | Me | Me | $C(=S)NH_2$ | H | Me | $4\text{-}CF_3$ | 81 to 83 |
| B-35 | $^nPrSCH_2$ | Me | Me | Me | $C(=S)NH_2$ | H | Me | $4\text{-}CF_3$ | 72 to 75 |
| B-36 | $MeSCH_2$ | Me | Me | Me | $C(=S)NH_2$ | H | Me | $4\text{-}CF_3$ | 74 to 77 |
| B-37 | $EtS(=O)CH_2$ | Me | Me | Me | $C(=S)NH_2$ | H | Me | $4\text{-}OCF_2CHF_2$ | 48 to 52 |
| B-38 | $EtSCH_2$ | Me | Me | Me | $C(=S)NH_2$ | H | Me | $4\text{-}OCF_2CHF_2$ | 66 to 68 |
| B-39 | $EtS(=O)CH_2$ | Me | Me | Me | $C(=S)NH_2$ | H | Me | $4\text{-}OCH_2CF_2CHF_2$ | 46 to 48 |
| B-40 | $EtSCH_2$ | Me | Me | Me | $C(=S)NH_2$ | H | Me | $4\text{-}OCH_2CF_2CHF_2$ | 67 to 70 |

[Table 11]

| Table 2 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properti es |
| B-41 | $EtS(=O)CH_2$ | Me | Me | Me | $C(=S)NH_2$ | H | Me | $4\text{-}OCF_2CHFCF_3$ | 69 to 72 |
| B-42 | $EtSCH_2$ | Me | Me | Me | $C(=S)NH_2$ | H | Me | $4\text{-}OCF_2CHFCF_3$ | 61 to 64 |
| B-43 | $EtSO_2CH_2$ | Me | Me | Me | $C(=S)NH_2$ | H | Me | $4\text{-}OCHF_2$ | amorph ous |
| B-44 | $EtS(=O)CH_2$ | Me | Me | Me | $C(=S)NH_2$ | H | Me | $4\text{-}OCHF_2$ | amorph ous |
| B-45 | | Me | Me | Me | $C(=S)NH_2$ | H | Me | $4\text{-}CF_3$ | 58 to 60 |

(continued)

| Table 2 (Continued) | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properti es |
| B-46 | | Me | Me | H | C(=S) $NH_2$ | H | Me | 4-$CF_3$ | 116 to 119 |
| B-47 | $EtSCH_2$ | Me | Me | Me | C(=S) $NH_2$ | H | $CF_3$ | 4-$CF_3$ | 134 to 135 |
| B-48 | $EtSCH_2$ | Me | Me | Me | C(=S) $NH_2$ | H | Me | 4-$OCHF_2$ | 57 to 59 |
| B-49 | $EtSCH_2$ | Me | Me | Me | C(=S) $NH_2$ | H | Me | 4-$OCF_3$ | 56 to 58 |
| B-50 | (5-Me-1,2,4-oxadiazol-3-yl)$CH_2$ | Me | Me | Me | C(=S) $NH_2$ | H | Me | 4-$CF_3$ | 94 to 96 |
| B-51 | (5-Me-1,2,4-oxadiazol-3-yl)$CH_2$ | Me | Me | H | C(=S) $NH_2$ | H | Me | 4-$CF_3$ | 97 to 99 |
| B-52 | $EtSCH_2$ | Me | Me | Me | C(=S) $NH_2$ | H | Me | 4-$CF_3$ | 76 to 79 |

[Table 12]

| Table 3 | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| C-1 | $EtS(=O)CH_2CH_2$ | Me | Me | Me | CN | H | $CH_2OMe$ | 4-$CF_3$ | viscous oil |
| C-2 | $EtSO_2CH_2CH_2$ | Me | Me | Me | CN | H | $CH_2OMe$ | 4-$CF_3$ | viscous oil |
| C-3 | $EtSCH_2CH_2$ | Me | Me | Me | CN | H | $CH_2OMe$ | 4-$CF_3$ | viscous oil |
| C-4 | $EtS(=O)CH_2CH_2$ | Et | Et | Me | CN | H | $CF_3$ | 4-$CF_3$ | amorphous |
| C-5 | $EtSCH_2CH_2$ | Et | Et | Me | CN | H | $CF_3$ | 4-$CF_3$ | amorphous |
| C-6 | $EtS(=O)CH_2CH_2$ | Et | Et | H | CN | H | $CF_3$ | 4-$CF_3$ | 40 to 42 |
| C-7 | $EtSCH_2CH_2$ | Et | Et | H | CN | H | $CF_3$ | 4-$CF_3$ | 153 to 155 |
| C-8 | $EtSO_2CH_2CH_2$ | Me | Me | Me | CN | H | $CF_3$ | 4-$CF_3$ | 63 to 65 |
| C-9 | $EtS(=O)CH_2CH_2$ | Me | Me | Me | CN | H | $CF_3$ | 4-$CF_3$ | amorphous |
| C-10 | $EtSCH_2CH_2$ | Me | Me | Me | CN | H | $CF_3$ | 4-$CF_3$ | viscous oil |

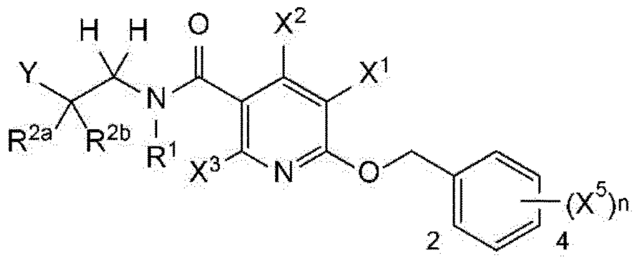

(I-3)

[Table 13]

| Table 4 | | | | | | | | | |
|---|---|---|---|---|---|---|---|---|---|
| No. | Y | $R^{2a}$ | $R^{2b}$ | $R^1$ | $X^1$ | $X^2$ | $X^3$ | $(X^5)n$ | Physical properties |
| D-1 | $EtSCH_2CH_2$ | Et | Et | H | CN | H | Me | 4-$CF_3$ | viscous oil |
| D-2 | $EtSCH_2$ | Me | Me | Me | CN | H | Me | 4-$CF_3$ | 42 to 45 |

Among the compounds shown in the table, $^1$H-NMR ($CDCl_3$) was measured for the compounds with physical properties of a viscous oil or amorphous substance. The measured values are shown below.

Compound No. (A-1): 1H-NMR (400 MHz, CDCl3/TMS, δ (ppm)): 8.10 (s, 1H), 7.80 (s, 1H), 7.45 (d, 1H), 7.32 (s, 1H), 7.22 (s, 1H), 4.70 (s, 2H), 4.25 (q, 2H), 3.90 (s, 3H), 3.50 to 3.30 (br, 2H), 3.45 (s, 3H), 2.95 (s, 3H), 2.63 to 2.57 (m, 4H), 1.68 to 1.60 (m, 2H), 1.35 (t, 3H), 1.25 (t, 3H), 1.05 (s, 6H)
Compound No. (A-2): 1H-NMR (400 MHz, CDCl3/TMS, δ (ppm)): 7.80 (s, 1H), 7.48 (d, 1H), 6.98 (d, 1H), 6.87 (s, 1H), 4.70 (s, 2H), 3.90 (s, 3H), 3.50 to 3.30 (br, 2H), 3.45 (s, 3H), 2.95 (s, 3H), 2.63 to 2.57 (m, 4H), 1.68 to 1.60 (m, 2H), 1.25 (t, 3H), 1.05 (s, 6H)
Compound No. (A-3): 1H-NMR (400 MHz, CDCl3/TMS, δ (ppm)): 8.00 (d, 1H), 7.89 (s, 1H), 7.26 (d, 2H), 6.60 (t, 1H), 4.70 (s, 2H), 3.50 to 3.30 (br, 2H), 3.45 (s, 3H), 2.95 (s, 3H), 2.63 to 2.57 (m, 4H), 1.68 to 1.60 (m, 2H), 1.25 (t, 3H), 1.05 (s, 6H)
Compound No. (A-4): 1H-NMR (400 MHz, CDCl3/TMS, δ (ppm)): 8.00 (d, 2H), 7.89 (s, 1H), 7.37 (d, 2H), 4.70 (s, 2H), 3.50 to 3.30 (br, 2H), 3.45 (s, 3H), 2.95 (s, 3H), 2.63 to 2.57 (m, 4H), 1.68 to 1.60 (m, 2H), 1.25 (t, 3H), 1.05 (s, 6H)
Compound No. (A-5): 1H-NMR (400 MHz, CDCl3/TMS, δ (ppm)): 8.10 (s, 1H), 7.95 (s, 1H), 7.41 (d, 1H), 7.32 (s, 1H), 7.26 (d, 2H), 5.87 (t, 1H), 4.26 (q, 2H), 3.90 (s, 3H), 3.38 (d, 2H), 2.78 (s, 3H), 2.60 to 2.48 (m, 4H), 1.60 to 1.52 (m, 2H), 1.36 to 1.25 (m, 10H), 1.88 (t, 6H)
Compound No. (A-8): 1H-NMR (400 MHz, CDCl3/TMS, δ (ppm)): 8.60 (s, 1H), 8.05 (d, 2H), 7.90 (t, 1H), 7.30 (d, 2H), 6.60 (t, 1H), 4.79 (s, 2H), 3.51 (s, 3H), 3.38 (d, 2H), 2.60 to 2.48 (m, 4H), 1.60 to 1.52 (m, 2H), 1.36 to 1.25 (m, 7H), 1.86 (t, 6H)
Compound No. (A-9): 1H-NMR (400 MHz, CDCl3/TMS, δ (ppm)): 8.60 (s, 1H), 8.05 (d, 2H), 7.90 (t, 1H), 7.37 (d, 2H), 4.79 (s, 2H), 3.50 (s, 3H), 3.38 (d, 2H), 2.60 to 2.48 (m, 4H), 1.60 to 1.52 (m, 2H), 1.36 to 1.25 (m, 7H), 1.86 (t, 6H)
Compound No. (A-10): 1H-NMR (400 MHz, CDCl3/TMS, δ (ppm)): 8.00 (s, 1H), 7.46 (d, 1H), 6.98 (d, 1H), 6.87 (s, 1H), 6.20 (t, 1H), 3.90 (s, 3H), 3.38 (d, 2H), 3.10 to 3.00 (m, 4H), 2.80 (s, 3H), 1.86 to 1.76 (m, 2H), 1.43 to 1.20 (m, 13H), 1.95 (t, 6H)
Compound No. (A-11): 1H-NMR (400 MHz, CDCl3/TMS, δ (ppm)): 7.95 (s, 1H), 7.46 (d, 1H), 7.37 (t, 1H), 6.98 (d, 1H), 6.87 (s, 1H), 3.90 (s, 3H), 3.40 (dd, 2H), 2.80 (s, 3H), 2.80 to 2.57 (m, 4H), 1.90 to 1.68 (m, 2H), 1.50 to 1.15 (m, 13H), 1.92 (t, 6H)
Compound No. (A-12): 1H-NMR (400 MHz, CDCl3/TMS, δ (ppm)): 7.95 (s, 1H), 7.46 (d, 1H), 6.98 (d, 1H), 6.87 (s, 1H), 5.90 (t, 1H), 3.90 (s, 3H), 3.38 (d, 2H), 2.78 (s, 3H), 2.60 to 2.50 (m, 4H), 1.60 to 1.50 (m, 2H), 1.40 to 1.20 (m, 13H), 1.93 (t, 6H)
Compound No. (A-13): 1H-NMR (400 MHz, CDCl3/TMS, δ (ppm)): 8.10 (s, 1H), 8.06 (d, 2H), 7.80 (d, 1H), 6.38 (t, 1H), 3.38 (d, 2H), 3.10 to 3.00 (m, 4H), 2.80 (s, 3H), 1.86 to 1.76 (m, 2H), 1.43 to 1.18 (m, 13H), 1.95 (t, 6H)
Compound No. (A-14): 1H-NMR (400 MHz, CDCl3/TMS, δ (ppm)): 8.12 (s, 1H), 8.05 (t, 1H), 7.79 (d, 1H), 7.65 (t, 1H), 3.40 (dd, 2H), 2.80 (s, 3H), 2.80 to 2.57 (m, 4H), 1.90 to 1.68 (m, 2H), 1.50 to 1.15 (m, 13H), 1.92 (t, 6H)
Compound No. (A-16): 1H-NMR (400 MHz, CDCl3/TMS, δ (ppm)): 8.30 (s, 1H), 7.52 (d, 1H), 6.99 (d, 1H), 6.97 (t, 1H), 6.89 (s, 1H), 6.43 (t, 1H), 3.90 (s, 3H), 3.38 (d, 2H), 3.10 to 3.00 (m, 4H), 1.86 to 1.76 (m, 2H), 1.43 to 1.22 (m, 7H), 1.90 (t, 6H)
Compound No. (A-17): 1H-NMR (400 MHz, CDCl3/TMS, δ (ppm)): 8.35 (s, 1H), 7.70 (t, 1H), 7.52 (d, 1H), 7.16 (t, 1H), 6.98 (d, 1H), 6.87 (s, 1H), 3.90 (s, 3H), 3.40 to (dd, 2H), 2.80 to 2.57 (m, 4H), 1.90 to 1.68 (m, 2H), 1.43 to 1.22 (m, 7H), 1.90 (t, 6H)
Compound No. (A-19): 1H-NMR (400 MHz, CDCl3/TMS, δ (ppm)): 8.05 (s, 1H), 8.04 (d, 2H), 7.25 (d, 2H), 6.60 (t, 1H), 6.20 (t, 1H), 3.35 (d, 2H), 3.10 to 3.00 (m, 6H), 1.85 to 1.78 (m, 2H), 1.43 to 1.22 (m, 7H), 1.90 (t, 6H)
Compound No. (A-20): 1H-NMR (400 MHz, CDCl3/TMS, δ (ppm)): 8.05 (s, 1H), 8.00 (d, 2H), 7.41 (t, 1H), 7.25 (d, 2H), 6.60 (t, 1H), 3.40 (dd, 2H), 3.10 (q, 2H), 2.80 to 2.54 (m, 4H), 1.90 to 1.70 (m, 2H), 1.43 to 1.20 (m, 7H), 1.93 to 1.88 (m, 6H)
Compound No. (A-22): 1H-NMR (400 MHz, CDCl3/TMS, δ (ppm)): 8.12 (s, 1H), 7.96 (d, 2H), 7.25 (d, 2H), 6.60 (t, 1H), 6.25 (t, 1H), 3.35 (d, 2H), 3.06 to 3.00 (m, 4H), 2.80 (s, 3H), 1.85 to 1.78 (m, 2H), 1.43 to 1.22 (m, 7H), 1.90 (t, 6H)

Compound No. (A-23): 1H-NMR (400 MHz, CDCl3/TMS, δ (ppm)): 8.12 (s, 1H), 7.96 (d, 2H), 7.52 (t, 1H), 7.25 (d, 2H), 6.60 (t, 1H), 3.37 (dd, 2H), 2.80 (s, 3H), 2.80 to 2.54 (m, 4H), 1.90 to 1.78 (m, 2H), 1.43 to 1.20 (m, 7H), 1.93 to 1.88 (m, 6H)

Compound No. (A-25): 1H-NMR (400 MHz, CDCl3/TMS, δ (ppm)): 8.00 (s, 1H), 7.49 (d, 1H), 6.98 (d, 1H), 6.87 (s, 1H), 6.20 (t, 1H), 3.90 (s, 3H), 3.34 (d, 2H), 3.05 to 3.00 (m, 4H), 2.78 (s, 3H), 1.81 to 1.77 (m, 2H), 1.40 to 1.20 (m, 7H), 1.90 (t, 6H)

Compound No. (A-26): 1H-NMR (400 MHz, CDCl3/TMS, δ (ppm)): 8.00 (s, 1H), 7.47 (d, 1H), 7.33 (t, 1H), 6.98 (d, 1H), 6.87 (s, 1H), 3.90 (s, 3H), 3.40 (dd, 2H), 2.85 to 2.55 (m, 7H), 1.90 to 1.70 (m, 2H), 1.41 to 1.20 (m, 7H), 1.95 to 1.80 (m, 6H)

Compound No. (A-27): 1H-NMR (400 MHz, CDCl3/TMS, δ (ppm)): 7.96 (s, 1H), 7.49 (d, 1H), 6.98 (d, 1H), 6.87 (s, 1H), 5.90 (t, 1H), 3.90 (s, 3H), 3.38 (d, 2H), 2.80 (s, 3H), 2.60 to 2.50 (m, 4H), 1.55 (t, 3H), 1.36 to 1.25 (m, 7H), 1.91 to 1.80 (m, 6H)

Compound No. (A-28): 1H-NMR (400 MHz, CDCl3/TMS, δ (ppm)): 7.81 (s, 1H), 7.45 (d, 1H), 6.96 (d, 1H), 6.87 (s, 1H), 3.90 (s, 3H), 3.50 (s, 2H), 3.21 to 3.18 (m, 2H) 3.05 to 3.00 (m, 5H), 2.68 (s, 3H), 1.90 to 1.80 (m, 2H), 1.41 (t, 3H), 1.13 (s, 6H)

Compound No. (A-29): 1H-NMR (400 MHz, CDCl3/TMS, δ (ppm)): 7.81 (s, 1H), 7.45 (d, 1H), 6.96 (d, 1H), 6.87 (s, 1H), 3.90 (s, 3H), 3.65 (d, 1H), 3.32 (d, 1H), 3.05 (s, 3H), 2.96 to 2.67 (m, 7H), 1.90 to 1.70 (m, 2H), 1.40 (t, 3H), 1.16 (s, 3H), 1.10 (s, 3H)

Compound No. (A-31): 1H-NMR (400 MHz, CDCl3/TMS, δ (ppm)): 7.96 (s, 1H), 7.53 (d, 1H), 7.32 (d, 1H), 7.21 (s, 1H), 6.23 (t, 1H), 4.16 (q, 2H), 3.34 (d, 2H), 3.05 to 3.00 (m, 4H) 2.78 (s, 3H), 1.81 to 1.77 (m, 2H), 1.40 to 1.20 (m, 7H), 1.91 to 1.80 (m, 6H)

Compound No. (A-32): 1H-NMR (400 MHz, CDCl3/TMS, δ (ppm)): 7.96 (s, 1H), 7.53 (d, 1H), 7.41 (t, 1H), 7.32 (dd, 1H), 7.21 (d, 1H), 4.16 (q, 2H), 3.50 to 3.29 (m, 2H), 2.80 (s, 3H), 2.80 to 2.52 (m, 4H), 1.90 to 1.70 (m, 2H), 1.41 to 1.20 (m, 7H), 1.91 to 1.80 (m, 6H)

Compound No. (A-33): 1H-NMR (400 MHz, CDCl3/TMS, δ (ppm)): 7.96 (s, 1H), 7.53 (d, 1H), 7.32 (d, 1H), 7.21 (s, 1H), 5.97 (t, 1H), 4.16 (q, 2H), 3.34 (d, 2H), 2.76 (s, 3H), 2.57 to 2.50 (m, 4H), 1.58 to 1.50 (m, 2H), 1.40 to 1.20 (m, 7H), 1.91 to 1.80 (m, 6H)

Compound No. (A-34): 1H-NMR (400 MHz, CDCl3/TMS, δ (ppm)): 8.01 (s, 1H), 7.53 (d, 1H), 7.32 (dd, 1H), 7.21 (d, 1H), 6.25 (t, 1H), 3.90 (s, 3H), 3.34 (d, 2H), 3.05 to 3.00 (m, 4H), 2.76 (s, 3H), 1.80 to 1.75 (m, 2H), 1.40 to 1.20 (m, 7H), 1.91 to 1.86 (m, 6H)

Compound No. (A-35): 1H-NMR (400 MHz, CDCl3/TMS, δ (ppm)): 8.06 (s, 1H), 7.53 to 7.20 (m, 4H), 3.90 (s, 3H), 3.43 to 3.28 (m, 2H), 2.81 (s, 3H), 2.80 to 2.55 (m, 4H), 1.90 to 1.68 (m, 2H), 1.40 to 1.20 (m, 7H), 1.91 to 1.86 (m, 6H)

Compound No. (A-43): 1H-NMR (400 MHz, CDCl3/TMS, δ (ppm)): 8.47 to 7.04 (m, 8H), 4.14 (s, 3H), 4.06 (s, 2H), 2.80 to 2.53 (m, 7H), 1.67 (s, 6H), 1.40 to 1.26 (m, 3H)

Compound No. (A-45): 1H-NMR (400 MHz, CDCl3/TMS, δ (ppm)): 8.12 (s, 1H), 7.98 (d, 2H), 7.35 (d, 2H), 6.29 (t, 1H), 3.34 (d, 2H), 3.08 to 3.00 (m, 4H), 2.80 (s, 3H), 1.80 to 1.76 (m, 2H), 1.41 to 1.20 (m, 7H), 1.92 to 1.86 (m, 6H)

Compound No. (A-46): 1H-NMR (400 MHz, CDCl3/TMS, δ (ppm)): 8.05 (d, 2H), 7.88 (s, 1H), 7.77 (d, 2H), 3.69 to 2.45 (m, 12H), 1.71 to 1.20 (m, 7H), 1.03 (s, 6H)

Compound No. (A-49): 1H-NMR (400 MHz, CDCl3/TMS, δ (ppm)): 7.87 (s, 1H), 7.71 (t, 1H), 7.60 (dd, 2H), 7.52 (dd, 1H), 3.50 to 2.52 (m, 9H), 1.62 to 1.20 (m, 9H), 0.93 to 0.85 (m, 6H)

Compound No. (A-51): 1H-NMR (400 MHz, CDCl3/TMS, δ (ppm)): 8.00 (d, 2H), 7.87 (s, 1H), 7.37 (d, 2H), 3.65 (d, 1H), 3.28 (d, 1H), 2.95 (s, 3H), 2.95 to 2.90 (m, 2H), 2.78 to 2.70 (m, 2H), 2.65 (s, 3H), 1.90 to 1.30 (m, 9H), 0.93 to 0.86 (m, 6H)

Compound No. (A-52): 1H-NMR (400 MHz, CDCl3/TMS, δ (ppm)): 8.00 (d, 2H), 7.87 (s, 1H), 7.37 (d, 2H), 3.50 to 2.52 (m, 9H), 1.62 to 1.20 (m, 9H), 0.93 to 0.85 (m, 6H)

Compound No. (A-55): 1H-NMR (400 MHz, CDCl3/TMS, δ (ppm)): 8.00 (s, 1H), 7.63 (t, 1H), 7.20 (dd, 1H), 7.11 (dd, 1H), 5.92 (t, 1H), 3.37 (d, 2H), 2.78 (s, 3H), 2.60 to 2.47 (m, 4H), 1.60 to 1.50 (m, 2H), 1.35 to 1.20 (m, 7H), 0.90 to 0.80 (m, 6H)

Compound No. (A-58): 1H-NMR (400 MHz, CDCl3/TMS, δ (ppm)): 7.85 (s, 1H), 7.61 (t, 1H), 7.20 (dd, 1H), 7.11 (dd, 1H), 3.47 to 2.30 (m, 12H), 1.68 to 1.60 (m, 2H), 1.25 (t, 3H), 1.05 (s, 6H)

Compound No. (A-59): 1H-NMR (400 MHz, CDCl3/TMS, δ (ppm)): 8.15 (d, 2H), 8.13 (s, 1H), 7.83 (d, 2H), 3.73 (d, 1H), 3.22 (d, 1H), 2.95 (s, 3H), 2.62 to 2.52 (m, 4H), 1.65 to 1.20 (m, 9H), 0.93 to 0.85 (m, 6H)

Compound No. (A-63): 1H-NMR (400 MHz, CDCl3/TMS, δ (ppm)): 8.49 (s, 1H), 8.04 (t, 1H), 7.59 (t, 1H), 6.90 (dd, 1H), 6.81 (dd, 1H), 6.05 (dt, 1H), 4.79 (s, 2H), 4.41 (t, 2H), 3.49 (s, 3H), 3.47 to 3.29 (m, 2H), 2.73 to 2.58 (m, 4H), 1.80 to 1.60 (m, 2H), 1.40 to 1.28 (m, 7H), 0.90 to 0.80 (m, 6H)

Compound No. (A-67): 1H-NMR (400 MHz, CDCl3/TMS, δ (ppm)): 8.12 (s, 1H), 7.98 (d, 2H), 7.56 (t, 1H), 7.35 (d, 2H), 3.34 (d, 1H), 3.31 (d, 1H), 2.80 (s, 3H), 2.80 to 2.52 (m, 4H), 1.90 to 1.69 (m, 2H), 1.41 to 1.20 (m, 7H), 1.92 to 1.86 (m, 6H)

Compound No. (A-68): 1H-NMR (400 MHz, CDCl3/TMS, δ (ppm)): 8.55 (s, 1H), 7.87 (t, 1H), 7.59 (t, 1H), 6.90 (dd, 1H), 6.81 (dd, 1H), 6.05 (dt, 1H), 4.76 (s, 2H), 4.41 (t, 2H), 3.49 (s, 3H), 3.37 (d, 2H), 2.60 to 2.47 (m, 4H), 1.60 to 1.50 (m, 2H), 1.35 to 1.20 (m, 7H), 0.90 to 0.80 (m, 6H)

Compound No. (A-69): 1H-NMR (400 MHz, CDCl3/TMS, δ (ppm)): 8.00 (s, 1H), 7.70 (t, 1H), 7.59 (dd, 1H), 7.50 (dd, 1H), 5.99 (t, 1H), 3.37 (d, 2H), 2.79 (s, 3H), 2.60 to 2.47 (m, 4H), 1.60 to 1.50 (m, 2H), 1.35 to 1.20 (m, 7H), 0.90 to 0.80 (m, 6H)

Compound No. (A-73): 1H-NMR (400 MHz, CDCl3/TMS, δ (ppm)): 7.90 (s, 1H), 7.57 (t, 1H), 6.89 (dd, 1H), 6.80 (dd, 1H), 6.05 (dt, 1H), 4.70 (s, 2H), 4.40 (t, 2H), 3.45 (s, 2H), 3.21 to 3.16 (m, 2H), 3.05 to 2.97 (m, 4H), 1.90 to 1.80 (m, 2H), 1.40 (t, 3H), 1.10 (s, 6H)

Compound No. (A-76): 1H-NMR (400 MHz, CDCl3/TMS, δ (ppm)): 8.39 to 7.04 (m, 8H), 6.08 (tt, 1H), 4.41 (dt, 2H), 4.35 (s, 2H), 3.06 to 2.41 (m, 8H), 1.67 (s, 6H), 1.47 to 1.26 (m, 3H)

Compound No. (A-77): 1H-NMR (400 MHz, CDCl3/TMS, δ (ppm)): 8.39 to 7.12 (m, 8H), 4.35 (s, 2H), 3.06 to 2.41 (m, 8H), 1.67 (s, 6H), 1.47 to 1.26 (m, 3H)

Compound No. (A-81): 1H-NMR (400 MHz, CDCl3/TMS, δ (ppm)): 7.82 (s, 1H), 7.56 (t, 1H), 6.83 (dd, 1H), 6.80 (d, 1H), 6.05 (tt, 1H), 4. 40 (t, 2H), 3.47 to 2.30 (m, 12H), 1.68 to 1.60 (m, 2H), 1.25 (t, 3H), 1.05 (s, 6H)

Compound No. (A-88): 1H-NMR (400 MHz, CDCl3/TMS, δ (ppm)): 7.98 (d, 2H), 7.81 (s, 1H), 7.16 (d, 2H), 6.07 (dt, 1H), 4.42 (t, 2H), 3.45 (s, 2H), 3.21 to 3.16 (m, 2H), 3.05 to 2.97 (m, 4H), 2.67 (s, 3H), 1.90 to 1.80 (m, 2H), 1.40 (t, 3H), 1.13 (s, 6H)

Compound No. (A-89): 1H-NMR (400 MHz, CDCl3/TMS, δ (ppm)): 7.98 (d, 2H), 7.81 (s, 1H), 7.16 (d, 2H), 6.07 (dt, 1H), 4.42 (t, 2H), 3.62 (d, 1H), 3.33 (d, 1H), 2.98 (s, 3H), 2.91 to 2.69 (m, 4H), 2.66 (s, 3H), 1.90 to 1.70 (m, 2H), 1.35 (t, 3H), 1.11 (s, 3H), 1.09 (s, 3H)

Compound No. (A-91): 1H-NMR (400 MHz, CDCl3/TMS, δ (ppm)): 7.98 (d, 2H), 7.80 (s, 1H), 7.26 (d, 2H), 6.58 (t, 1H), 3.45 (s, 2H), 3.28 to 3.19 (m, 2H), 3.02 to 2.90 (m, 2H), 2.95 (s, 3H), 2.66 (s, 3H), 1.83 to 1.77 (m, 2H), 1.50 to 1.31 (m, 7H), 0.90 (t, 6H)

Compound No. (A-92): 1H-NMR (400 MHz, CDCl3/TMS, δ (ppm)): 7.98 (d, 2H), 7.80 (s, 1H), 7.26 (d, 2H), 6.58 (t, 1H), 3.65 (d, 1H), 3.28 (d, 1H), 2.95 (s, 3H), 2.95 to 2.90 (m, 2H), 2.78 to 2.70 (m, 2H), 2.65 (s, 3H), 1.90 to 1.30 (m, 9H), 0.90 (t, 6H)

Compound No. (A-93): 1H-NMR (400 MHz, CDCl3/TMS, δ (ppm)): 7.98 (d, 2H), 7.80 (s, 1H), 7.26 (d, 2H), 6.58 (t, 1H), 3.49 to 2.53 (m, 12H), 1.63 to 1.20 (m, 9H), 0.90 (t, 6H)

Compound No. (A-94): 1H-NMR (400 MHz, CDCl3/TMS, δ (ppm)): 7.98 (d, 2H), 7.80 (s, 1H), 7.26 (d, 2H), 6.58 (t, 1H), 3.46 (s, 2H), 3.28 to 3.19 (m, 2H), 3.02 to 2.95 (m, 2H), 2.97 (s, 3H), 2.66 (s, 3H), 1.98 to 1.30 (m, 15H)

Compound No. (A-95): 1H-NMR (400 MHz, CDCl3/TMS, δ (ppm)): 7.98 (d, 2H), 7.80 (s, 1H), 7.26 (d, 2H), 6.58 (t, 1H), 3.72 (d, 1H), 3.23 (d, 1H), 2.98 (s, 3H), 2.98 to 2.70 (m, 4H), 2.67 (s, 3H), 2.03 to 1.30 (m, 15H)

Compound No. (A-96): 1H-NMR (400 MHz, CDCl3/TMS, δ (ppm)): 7.98 (d, 2H), 7.80 (s, 1H), 7.26 (d, 2H), 6.58 (t, 1H), 3.50 to 2.50 (m, 12H), 1.75 to 1.23 (m, 15H)

Compound No. (A-97): 1H-NMR (400 MHz, CDCl3/TMS, δ (ppm)): 8.18 (d, 2H), 8.10 (s, 1H), 7.84 (d, 2H), 3.58 (d, 1H), 3.30 (d, 1H), 2.95 (s, 3H), 2.62 to 2.53 (m, 4H), 1.68 to 1.60 (m, 2H), 1.25 (t, 3H), 1.06 (s, 6H)

Compound No. (A-98): 1H-NMR (400 MHz, CDCl3/TMS, δ (ppm)): 8.61 to 7.51 (m, 4H), 5.48 to 4.70 (m, 2H), 3.60 to 2.95 (m, 8H), 2.62 to 2.52 (m, 4H), 1.68 to 1.60 (m, 2H), 1.25 (t, 3H), 1.06 (s, 6H)

Compound No. (A-99): 1H-NMR (400 MHz, CDCl3/TMS, δ (ppm)): 8.19 (d, 2H), 8.05 (s, 1H), 7.10 (d, 2H), 6.07 (tt, 1H), 4.44 (t, 2H), 3.60 to 3.30 (m, 2H), 2.62 to 2.53 (m, 4H), 1.66 to 1.60 (m, 2H), 1.27 (t, 3H), 1.04 (s, 6H)

Compound No. (A-100): 1H-NMR (400 MHz, CDCl3/TMS, δ (ppm)): 8.12 (d, 2H), 8.10 (s, 1H), 7.40 (d, 2H), 5.95 (tt, 1H), 3.60 to 3.30 (m, 2H), 2.62 to 2.53 (m, 4H), 1.66 to 1.60 (m, 2H), 1.27 (t, 3H), 1.04 (s, 6H)

Compound No. (A-101): 1H-NMR (400 MHz, CDCl3/TMS, δ (ppm)): 8.12 (d, 2H), 8.10 (s, 1H), 7.40 (d, 2H), 3.60 to 3.30 (m, 2H), 2.62 to 2.53 (m, 4H), 1.66 to 1.60 (m, 2H), 1.27 (t, 3H), 1.04 (s, 6H)

Compound No. (A-102): 1H-NMR (400 MHz, CDCl3/TMS, δ (ppm)): 8.00 (d, 2H), 7.89 (s, 1H), 7.78 (d, 2H), 3.45 to 2.30 (m, 12H), 1.66 to 1.60 (m, 2H), 1.25 (t, 3H), 1.07 (s, 6H)

Compound No. (A-105): 1H-NMR (400 MHz, CDCl3/TMS, δ (ppm)): 8.00 (d, 2H), 7.89 (s, 1H), 7.78 (d, 2H), 3.47 to 2.30 (m, 12H), 1.68 to 1.60 (m, 2H), 1.25 (t, 3H), 1.05 (s, 6H)

Compound No. (A-106): 1H-NMR (400 MHz, CDCl3/TMS, δ (ppm)): 8.08 (d, 2H), 8.07 (s, 1H), 7.30 (d, 2H), 6.61 (t, 1H), 3.60 to 3.30 (m, 2H), 2.91 (s, 3H), 2.60 to 2.53 (m, 4H), 1.68 to 1.60 (m, 2H), 1.25 (t, 3H), 1.05 (s, 6H)

Compound No. (A-107): 1H-NMR (400 MHz, CDCl3/TMS, δ (ppm)): 8.63 to 7.27 (m, 5H), 6.61 (t, 1H), 3.47 to 2.53 (m, 11H), 1.68 to 1.60 (m, 2H), 1.35 to 1.20 (m, 3H), 1.05 (s, 6H)

Compound No. (A-108): 1H-NMR (400 MHz, CDCl3/TMS, δ (ppm)): 7.98 (d, 2H), 7.81 (s, 1H), 7.16 (d, 2H), 6.07 (dt, 1H), 4.42 (t, 2H), 3.47 to 2.30 (m, 12H), 1.68 to 1.60 (m, 2H), 1.25 (t, 3H), 1.07 (s, 6H)

Compound No. (A-109): 1H-NMR (400 MHz, CDCl3/TMS, δ (ppm)): 8.00 (d, 2H), 7.88 (s, 1H), 7.37 (d, 2H), 5.93 (tt, 1H), 3.47 to 2.30 (m, 12H), 1.68 to 1.60 (m, 2H), 1.25 (t, 3H), 1.07 (s, 6H)

Compound No. (A-110): 1H-NMR (400 MHz, CDCl3/TMS, δ (ppm)): 8.16 (s, 1H), 8.13 (d, 2H), 7.81 (d, 2H), 3.60 to

3.30 (m, 2H), 2.95 (s, 3H), 2.61 to 2.53 (m, 4H), 1.66 to 1.60 (m, 2H), 1.27 (t, 3H), 1.05 (s, 6H)

Compound No. (A-111): 1H-NMR (400 MHz, CDCl3/TMS, δ (ppm)): 7.98 (d, 2H), 7.90 (s, 1H), 7.25 (d, 2H), 6.58 (t, 1H), 3.50 (s, 2H), 3.40 to 3.30 (m, 2H), 3.02 to 2.90 (m, 4H), 2.66 (s, 3H), 1.82 to 1.76 (m, 2H), 1.43 (t, 3H), 0.61 (dt, 4H)

Compound No. (A-112): 1H-NMR (400 MHz, CDCl3/TMS, δ (ppm)): 7.98 (d, 2H), 7.90 (s, 1H), 7.25 (d, 2H), 6.58 (t, 1H), 3.61 to 2.59 (m, 12H), 1.92 to 1.70 (m, 2H), 1.30 (t, 3H), 0.70 to 0.50 (m, 4H)

Compound No. (A-114): 1H-NMR (400 MHz, CDCl3/TMS, δ (ppm)): 8.00 to 7.72 (m, 3H), 7.30 to 6.86 (m, 6H), 6.61 (t, 1H), 3.53 to 2.38 (m, 10H), 1.03 (s, 6H)

Compound No. (A-115): 1H-NMR (400 MHz, CDCl3/TMS, δ (ppm)): 7.98 (d, 2H), 7.86 (s, 1H), 7.26 (d, 2H), 6.58 (t, 1H), 3.45 (s, 2H), 3.21 to 3.16 (m, 2H), 3.05 to 2.97 (m, 4H), 2.67 (s, 3H), 1.90 to 1.80 (m, 2H), 1.40 (t, 3H), 1.13 (s, 6H)

Compound No. (A-116): 1H-NMR (400 MHz, CDCl3/TMS, δ (ppm)): 7.98 (d, 2H), 7.86 (s, 1H), 7.26 (d, 2H), 6.58 (t, 1H), 3.62 (d, 1H), 3.30 (d, 1H), 2.99 (s, 3H), 2.90 to 2.70 (m, 4H), 2.67 (s, 3H), 1.90 to 1.71 (m, 2H), 1.35 (t, 3H), 1.13 (s, 6H)

Compound No. (A-117): 1H-NMR (400 MHz, CDCl3/TMS, δ (ppm)): 8.06 (d, 2H), 7.89 (s, 1H), 7.80 (d, 2H), 7.16 to 6.86 (m, 4H), 3.52 to 2.39 (m, 10H), 1.03 (s, 6H)

Compound No. (A-118): 1H-NMR (400 MHz, CDCl3/TMS, δ (ppm)): 8.00 (d, 2H), 7.88 (s, 1H), 7.36 (d, 2H), 3.47 to 2.30 (m, 12H), 1.66 to 1.50 (m, 2H), 1.27 (t, 3H), 1.05 (s, 6H)

Compound No. (A-119): 1H-NMR (400 MHz, CDCl3/TMS, δ (ppm)): 8.58 to 6.88 (m, 9H), 6.60 (t, 1H), 3.61 to 2.58 (m, 10H), 1.08 (s, 6H)

Compound No. (A-120): 1H-NMR (400 MHz, CDCl3/TMS, δ (ppm)): 8.57 to 6.88 (m, 9H), 3.61 to 2.58 (m, 10H), 1.08 (s, 6H)

Compound No. (A-121): 1H-NMR (400 MHz, CDCl3/TMS, δ (ppm)): 7.98 (d, 2H), 7.86 (s, 1H), 7.26 (d, 2H), 6.58 (t, 1H), 3.62 (d, 1H), 3.30 (d, 1H), 2.92 (s, 3H), 2.62 to 2.53 (m, 4H), 1.68 to 1.60 (m, 2H), 1.27 (t, 3H), 1.13 (s, 6H)

Compound No. (A-122): 1H-NMR (400 MHz, CDCl3/TMS, δ (ppm)): 8.05 (d, 2H), 7.90 (s, 1H), 7.80 (d, 2H), 3.62 (d, 1H), 3.30 (d, 1H), 2.92 (s, 3H), 2.62 to 2.53 (m, 4H), 1.68 to 1.60 (m, 2H), 1.27 (t, 3H), 1.13 (s, 6H)

Compound No. (A-123): 1H-NMR (400 MHz, CDCl3/TMS, δ (ppm)): 8.05 (d, 2H), 7.90 (s, 1H), 7.80 (d, 2H), 3.57 to 2.60 (m, 11H), 1.30 (d, 6H), 1.13 (s, 6H)

Compound No. (A-124): 1H-NMR (400 MHz, CDCl3/TMS, δ (ppm)): 8.05 (d, 2H), 7.90 (s, 1H), 7.80 (d, 2H), 3.57 to 2.33 (m, 12H), 1.61 (q, 2H), 1.15 (s, 6H), 0.99 (t, 3H)

Compound No. (A-125): 1H-NMR (400 MHz, CDCl3/TMS, δ (ppm)): 8.05 (d, 2H), 7.90 (s, 1H), 7.80 (d, 2H), 3.69 to 2.16 (m, 13H), 1.15 (s, 6H)

Compound No. (A-126): 1H-NMR (400 MHz, CDCl3/TMS, δ (ppm)): 8.00 (d, 2H), 7.88 (s, 1H), 7.37 (d, 2H), 5.93 (tt, 1H), 3.84 (d, 1H), 3.41 (d, 1H), 3.01 (s, 3H), 2.80 to 2.60 (m, 7H), 1.39 (t, 3H), 1.30 (s, 6H)

Compound No. (A-127): 1H-NMR (400 MHz, CDCl3/TMS, δ (ppm)): 8.00 (d, 2H), 7.88 (s, 1H), 7.37 (d, 2H), 5.93 (tt, 1H), 3.56 to 2.35 (m, 12H), 1.28 (t, 3H), 1.14 (s, 6H)

Compound No. (A-128): 1H-NMR (400 MHz, CDCl3/TMS, δ (ppm)): 7.98 (d, 2H), 7.81 (s, 1H), 7.16 (d, 2H), 6.07 (dt, 1H), 4.42 (t, 2H), 3.84 (d, 1H), 3.41 (d, 1H), 3.01 (s, 3H), 2.80 to 2.60 (m, 7H), 1.39 (t, 3H), 1.30 (s, 6H)

Compound No. (A-129): 1H-NMR (400 MHz, CDCl3/TMS, δ (ppm)): 7.98 (d, 2H), 7.81 (s, 1H), 7.16 (d, 2H), 6.07 (dt, 1H), 4.42 (t, 2H), 3.56 to 2.35 (m, 12H), 1.28 (t, 3H), 1.14 (s, 6H)

Compound No. (A-130): 1H-NMR (400 MHz, CDCl3/TMS, δ (ppm)): 7.98 (d, 2H), 7.88 (s, 1H), 7.37 (d, 2H), 5.10 to 4.95 (m, 1H), 3.84 (d, 1H), 3.41 (d, 1H), 3.01 (s, 3H), 2.80 to 2.60 (m, 7H), 1.39 (t, 3H), 1.30 (s, 6H)

Compound No. (A-131): 1H-NMR (400 MHz, CDCl3/TMS, δ (ppm)): 7.98 (d, 2H), 7.88 (s, 1H), 7.37 (d, 2H), 5.10 to 4.95 (m, 1H), 3.56 to 2.35 (m, 12H), 1.28 (t, 3H), 1.14 (s, 6H)

Compound No. (A-132): 1H-NMR (400 MHz, CDCl3/TMS, δ (ppm)): 7.97 (d, 2H), 7.87 (s, 1H), 7.27 (d, 2H), 6.58 (t, 1H), 3.72 (s, 2H), 3.12 (s, 2H), 3.01 (s, 3H), 3.01 (q, 2H), 2.65 (s, 3H), 1.41 (t, 3H), 1.39 (s, 6H)

Compound No. (A-133): 1H-NMR (400 MHz, CDCl3/TMS, δ (ppm)): 7.98 (d, 2H), 7.86 (s, 1H), 7.26 (d, 2H), 6.58 (t, 1H), 3.87 (d, 1H), 3.41 (d, 1H), 3.00 (s, 3H), 2.80 to 2.61 (m, 7H), 1.40 to 1.29 (m, 9H)

Compound No. (A-135): 1H-NMR (400 MHz, CDCl3/TMS, δ (ppm)): 8.16 (s, 1H), 8.13 (d, 2H), 7.81 (d, 2H), 3.70 to 3.40 (m, 2H), 2.98 (s, 3H), 2.65 to 2.54 (m, 4H), 1.25 (t, 3H), 1.14 (s, 6H)

Compound No. (A-136): 1H-NMR (400 MHz, CDCl3/TMS, δ (ppm)): 7.97 (d, 2H), 7.87 (s, 1H), 7.27 (d, 2H), 6.58 (t, 1H), 3.56 to 2.35 (m, 12H), 1.30 to 1.11 (m, 9H)

Compound No. (A-137): 1H-NMR (400 MHz, CDCl3/TMS, δ (ppm)): 8.00 (d, 2H), 7.88 (s, 1H), 7.36 (d, 2H), 3.56 to 2.35 (m, 12H), 1.30 to 1.11 (m, 9H)

Compound No. (A-138): 1H-NMR (400 MHz, CDCl3/TMS, δ (ppm)): 8.05 (d, 2H), 7.90 (s, 1H), 7.80 (d, 2H), 3.64 to 2.40 (m, 13H), 1.15 (s, 6H)

Compound No. (A-140): 1H-NMR (400 MHz, CDCl3/TMS, δ (ppm)): 8.05 (d, 2H), 7.90 (s, 1H), 7.80 (d, 2H), 3.73 to 2.60 (m, 12H), 1.41 (t, 3H), 1.39 (s, 6H)

Compound No. (A-141): 1H-NMR (400 MHz, CDCl3/TMS, δ (ppm)): 8.05 (d, 2H), 7.90 (s, 1H), 7.80 (d, 2H), 3.87 (d, 1H), 3.41 (d, 1H), 3.00 (s, 3H), 2.80 to 2.61 (m, 7H), 1.40 to 1.29 (m, 9H)

Compound No. (A-142): 1H-NMR (400 MHz, CDCl3/TMS, δ (ppm)): 8.05 (d, 2H), 8.00 (s, 1H), 7.80 (d, 2H), 4.02 (s, 1H), 3.63 (s, 1H), 2.70 to 2.35 (m, 10H), 1.25 (t, 3H), 1.15 (s, 3H)
Compound No. (A-143): 1H-NMR (400 MHz, CDCl3/TMS, δ (ppm)): 8.05 (d, 2H), 8.00 (s, 1H), 7.80 (d, 2H), 3.69 to 2.31 (m, 11H), 1.30 to 0.07 (m, 14H)
Compound No. (A-144): 1H-NMR (400 MHz, CDCl3/TMS, δ (ppm)): 8.05 (d, 2H), 8.00 (s, 1H), 7.80 (d, 2H), 3.53 to 2.35 (m, 11H), 1.54 to 0.73 (m, 14H)
Compound No. (A-145): 1H-NMR (400 MHz, CDCl3/TMS, δ (ppm)): 8.05 (d, 2H), 8.00 (s, 1H), 7.80 (d, 2H), 3.59 to 2.34 (m, 11H), 1.32 to 0.90 (m, 12H)
Compound No. (A-146): 1H-NMR (400 MHz, CDCl3/TMS, δ (ppm)): 8.05 (d, 2H), 8.00 (s, 1H), 7.80 (d, 2H), 3.57 to 2.35 (m, 12H), 1.30 to 1.11 (m, 9H)
Compound No. (B-26): 1H-NMR (400 MHz, CDCl3/TMS, δ (ppm)): 7.92 (s, 1H), 7.80 (d, 2H), 7.60 (br, 1H), 7.19 (d, 2H), 6.85 (br, 1H), 6.55 (t, 1H), 3.42 to 2.88 (m, 9H), 2.60 (s, 3H), 1.90 to 1.80 (m, 2H), 1.40 (t, 3H), 1.10 (s, 6H)
Compound No. (B-27): 1H-NMR (400 MHz, CDCl3/TMS, δ (ppm)): 7.92 (s, 1H), 7.80 (d, 2H), 7.60 (br, 1H), 7.19 (d, 2H), 6.85 (br, 1H), 6.55 (t, 1H), 3.60 to 2.75 (m, 9H), 2.60 (s, 3H), 1.90 to 1.68 (m, 2H), 1.35 (t, 3H), 1.12 (s, 3H), 1.08 (s, 3H)
Compound No. (B-28): 1H-NMR (400 MHz, CDCl3/TMS, δ (ppm)): 7.93 (d, 1H), 7.94 (d, 2H), 7.71 (d, 1H), 7.54 (d, 1H), 7.26 to 6.86 (m, 5H), 3.51 to 2.99 (m, 5H), 2.65 to 2.39 (m, 5H), 1.01 (s, 6H)
Compound No. (B-43): 1H-NMR (400 MHz, CDCl3/TMS, δ (ppm)): 7.95 (s, 1H), 7.80 (d, 2H), 7.62 (br, 1H), 7.20 (d, 2H), 6.78 (br, 1H), 6.54 (t, 1H), 3.67 (s, 2H), 3.23 to 3.00 (m, 7H), 2.60 (s, 3H), 1.41 (t, 3H), 1.40 (s, 6H)
Compound No. (B-44): 1H-NMR (400 MHz, CDCl3/TMS, δ (ppm)): 7.95 (s, 1H), 7.80 (d, 2H), 7.62 (br, 1H), 7.20 (d, 2H), 6.78 (br, 1H), 6.54 (t, 1H), 3.89 (d, 1H), 3.32 (d, 1H), 3.23 to 2.60 (m, 7H), 2.58 (s, 3H), 1.36 (t, 3H), 1.32 (s, 3H), 1.27 (s, 3H)
Compound No. (C-1): 1H-NMR (400 MHz, CDCl3/TMS, δ (ppm)): 7.77 (d, 2H), 7.68 (d, 2H), 7.65 (s, 1H), 7.61 (s, 1H), 4.55 (s, 2H), 3.70 to 3.60 (m, 1H), 3.45 (s, 3H), 3.33 to 3.27 (m, 1H), 2.99 (s, 3H), 2.98 to 2.67 (m, 4H), 1.89 to 1.70 (m, 2H), 1.36 (t, 3H), 1.13 (s, 3H), 1.10 (s, 3H)
Compound No. (C-2): 1H-NMR (400 MHz, CDCl3/TMS, δ (ppm)): 7.77 (d, 2H), 7.70 (d, 2H), 7.64 (s, 1H), 7.62 (s, 1H), 4.56 (s, 2H), 3.45 (s, 3H), 3.44 (s, 2H), 3.23 to 3.19 (m, 2H), 3.05 to 2.97 (m, 2H), 2.98 (s, 3H), 1.90 to 1.86 (m, 2H), 1.40 (t, 3H), 1.12 (s, 6H)
Compound No. (C-3): 1H-NMR (400 MHz, CDCl3/TMS, δ (ppm)): 7.77 (d, 2H), 7.73 to 7.60 (m, 4H), 4.56 (s, 2H), 3.48 to 3.45 (m, 5H), 2.97 (s, 3H), 2.64 to 2.55 (m, 4H), 1.68 to 1.64 (m, 2H), 1.28 (t, 3H), 1.08 (s, 6H)
Compound No. (C-4): 1H-NMR (400 MHz, CDCl3/TMS, δ (ppm)): 7.89 to 7.66 (m, 6H), 3.47 to 2.61 (m, 9H), 1.90 to 1.30 (m, 9H), 0.91 (t, 6H)
Compound No. (C-5): 1H-NMR (400 MHz, CDCl3/TMS, δ (ppm)): 7.89 to 7.66 (m, 6H), 3.76 (d, 1H), 3.20 (dd, 1H), 2.90 (s, 3H), 2.62 to 2.55 (m, 4H), 1.63 to 1.20 (m, 9H), 0.91 (t, 6H)
Compound No. (C-9): 1H-NMR (400 MHz, CDCl3/TMS, δ (ppm)): 7.85 to 7.65 (m, 6H), 3.66 to 3.20 (m, 2H), 2.95 (s, 3H), 2.95 to 2.67 (m, 4H), 1.90 to 1.70 (m, 2H), 1.34 (t, 3H), 1.13 (s, 3H), 1.09 (s, 3H)
Compound No. (C-10): 1H-NMR (400 MHz, CDCl3/TMS, δ (ppm)): 7.85 to 7.65 (m, 6H), 3.62 (d, 1H), 3.30 (d, 1H), 2.92 (s, 3H), 2.62 to 2.53 (m, 4H), 1.68 to 1.60 (m, 2H), 1.27 (t, 3H), 1.13 (s, 6H)
Compound No. (D-1): 1H-NMR (400 MHz, CDCl3/TMS, δ (ppm)): 7.89 (s, 1H), 7.61 (dd, 4H), 5.78 (t, 1H), 5.57 (s, 2H), 3.31 (d, 2H), 2.61 (s, 3H), 2.58 to 2.48 (m, 4H), 1.55 to 1.50 (m, 2H), 1.36 to 1.22 (m, 7H), 1.85 (t, 6H)

[Bioassay Test]

The following Test Examples show that the compound of the present invention is useful as an active ingredient of an insecticidal agent, an acaricidal agent, or an ectoparasite control agent.

(Preparation of Test Emulsion)

5 parts by mass of the compound of the present invention, 93.6 parts by mass of dimethylformamide, and 1.4 parts by mass of polyoxyethylene alkylaryl ether were mixed and dissolved to prepare an emulsion (I) containing 5% of an active ingredient.
In addition, 98.6 parts by mass of dimethylformamide and 1.4 parts by mass of polyoxyethylene alkylaryl ether were mixed to prepare a solvent control solution.

(Test Example 1) Efficacy Test against Mythimna separata

The emulsion (I) was diluted with water so that the concentration of the compound of the present invention reached 125 ppm by mass. Maize leaf pieces were immersed in the diluent solution for 30 seconds. These maize leaf

pieces were placed in a petri dish, and five second-instar larvae of Mythimna separata were released to prepare an emulsion-treated section.

Similarly, the solvent control solution was diluted with water at the same ratio as that of the emulsion (I), and maize leaf pieces were immersed therein for 30 seconds. These maize leaf pieces were placed in a petri dish, and five second-instar larvae of Mythimna separata were released to prepare a solvent control section.

The petri dishes were left to stand in a thermostatic chamber at a temperature of 25°C and a humidity of 60% during the test period. Mortality was evaluated at a time of passage of 6 days from the release of the insects, and the insecticidal rate in the emulsion-treated section was calculated, based on the following expression. At the same time, the food consumptions in the emulsion-treated section and the solvent control section were measured. The test was repeated twice.

$$\text{Insecticidal rate (\%)} = (\text{Number of dead insects}/\text{Number of test insects}) \times 100$$

The efficacy test against Mythimna separata was carried out for the compounds of Compound Nos. A-27 and A-105. In all compounds, the insecticidal rates were 90% against Mythimna separata and the food consumption was 10% or less in terms of the solvent control section ratio.

(Test Example 2) Efficacy Test against Tetranychus kanzawai

The primary leaves of Kidney bean plants were inoculated with 5 adult female Tetranychus kanzawai from Okayama Prefecture. Next, the emulsion (I) was diluted with water so that the compound concentration reached 125 ppm by mass to obtain a drug. This drug was sprayed on the Kidney bean plants and air-dried. The kidney bean seedlings were then placed in a thermostatic chamber with a temperature of 25°C and humidity of 65%. Mortality of the adult Tetranychus kanzawai was investigated after 10 days had passed from the spraying. The test was repeated twice.

The efficacy test against Tetranychus kanzawai was carried out for the compounds with Compound Nos. shown in Table 5. All of the compounds demonstrated an insecticical rate of 90% or more against Tetranychus kanzawai.

[Table 14]

| Table 5 | | | | | | | | | |
|---|---|---|---|---|---|---|---|---|---|
| Compound No. | | | | | | | | | |
| A-1 | A-15 | A-29 | A-45 | A-59 | A-73 | A-95 | A-114 | A-146 | C-4 |
| A-2 | A-16 | A-30 | A-46 | A-60 | A-74 | A-96 | A-115 | A-147 | C-5 |
| A-3 | A-17 | A-31 | A-47 | A-61 | A-75 | A-97 | A-116 | B-25 | C-6 |
| A-4 | A-18 | A-32 | A-48 | A-62 | A-76 | A-98 | A-119 | B-28 | C-7 |
| A-5 | A-19 | A-3 3 | A-49 | A-63 | A-77 | A-102 | A-120 | B-30 | C-8 |
| A-6 | A-20 | A-34 | A-50 | A-64 | A-78 | A-104 | A-123 | B-31 | C-9 |
| A-7 | A-21 | A-35 | A-51 | A-65 | A-79 | A-105 | A-124 | B-34 | C-10 |
| A-8 | A-22 | A-36 | A-52 | A-66 | A-80 | A-107 | A-125 | B-35 | D-1 |
| A-9 | A-23 | A-38 | A-53 | A-67 | A-81 | A-108 | A-132 | B-36 | D-2 |
| A-10 | A-24 | A-3 9 | A-54 | A-68 | A-82 | A-109 | A-138 | B-50 | |
| A-11 | A-25 | A-40 | A-55 | A-69 | A-83 | A-110 | A-139 | B-51 | |
| A-12 | A-26 | A-41 | A-56 | A-70 | A-88 | A-111 | A-140 | C-1 | |
| A-13 | A-27 | A-42 | A-57 | A-71 | A-89 | A-112 | A-141 | C-2 | |
| A-14 | A-28 | A-43 | A-58 | A-72 | A-94 | A-113 | A-145 | C-3 | |

(Test Example 3) Efficacy Test against Tetranychus urticae

Seedlings of green beans were grown in 3-inch pots, and 8 adult female Tetranychus urticae from Aomori Prefecture were inoculated onto the first leaves. The emulsion (I) was diluted with water so that the concentration of the

compound of the present invention reached 125 ppm by mass. The diluent solution was sprayed on the green beans. The green beans were then placed in a thermostatic chamber with a temperature of 25°C and humidity of 65%. Mortality of the adult Tetranychus kanzawai was investigated after 10 days had passed from the spraying. The test was repeated twice.

The efficacy test against Tetranychus urticae was carried out for the compounds with Compound Nos. shown in Table 6. All of the compounds demonstrated an insecticical rate of 90% or more.

[Table 15]

| Table 6 | | | | | | | | | |
|---|---|---|---|---|---|---|---|---|---|
| Compound No. | | | | | | | | | |
| A-1 | A-17 | A-36 | A-59 | A-76 | A-102 | A-124 | B-1 | B-21 | B-43 |
| A-2 | A-18 | A-3 9 | A-60 | A-77 | A-104 | A-125 | B-2 | B-22 | B-44 |
| A-3 | A-19 | A-40 | A-61 | A-78 | A-105 | A-126 | B-3 | B-23 | B-47 |
| A-4 | A-20 | A-41 | A-62 | A-79 | A-106 | A-128 | B-4 | B-24 | B-48 |
| A-5 | A-21 | A-42 | A-63 | A-80 | A-107 | A-129 | B-5 | B-26 | B-49 |
| A-6 | A-22 | A-43 | A-64 | A-81 | A-108 | A-130 | B-10 | B-27 | B-52 |
| A-7 | A-23 | A-45 | A-65 | A-83 | A-109 | A-132 | B-11 | B-29 | C-5 |
| A-8 | A-24 | A-48 | A-66 | A-88 | A-110 | A-135 | B-12 | B-32 | C-6 |
| A-9 | A-25 | A-51 | A-67 | A-89 | A-111 | A-136 | B-13 | B-33 | C-7 |
| A-10 | A-26 | A-52 | A-68 | A-91 | A-112 | A-137 | B-14 | B-35 | C-8 |
| A-11 | A-27 | A-53 | A-69 | A-92 | A-113 | A-140 | B-15 | B-37 | C-9 |
| A-12 | A-28 | A-54 | A-70 | A-93 | A-115 | A-141 | B-16 | B-38 | C-10 |
| A-13 | A-29 | A-55 | A-71 | A-98 | A-116 | A-142 | B-17 | B-39 | D-1 |
| A-14 | A-30 | A-56 | A-72 | A-99 | A-118 | A-145 | B-18 | B-40 | |
| A-15 | A-34 | A-57 | A-74 | A-100 | A-121 | A-146 | B-19 | B-41 | |
| A-16 | A-35 | A-58 | A-75 | A-101 | A-122 | A-147 | B-20 | B-42 | |

Since a compound randomly selected from the compounds of the present invention has the above-described effects, it can be understood that the compounds of the present invention, including the compounds that are not exemplary examples, are compounds having the effects of pest control, in particular, insecticidal, acaricidal, and ectoparasite control effects and the like, causing no phytotoxicity to plant bodies, with little toxicity to animals and fish and little impact on the environment.

[Industrial Applicability]

The (hetero)arylamide compound of the present invention can control pests that cause problems in terms of agricultural crops and hygiene. In particular, the (hetero)arylamide compound can effectively control agricultural pests and acari at a lower concentration. Furthermore, the (hetero)arylamide compound can effectively control ectoparasites that harm humans and livestock.

## Claims

1. A compound represented by Formula (I) or a salt of the compound,

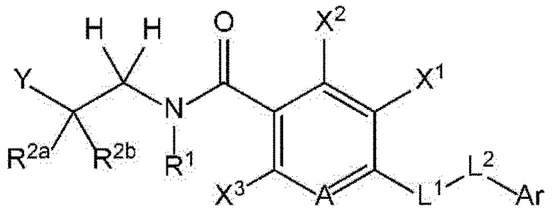
(I)

in Formula (I),

Ar represents a substituted or unsubstituted C6 to C10 aryl group, or a substituted or unsubstituted 5- or 6-membered heteroaryl group,
$L^1$ represents a single bond, an oxygen atom, or a sulfur atom,
$L^2$ represents a single bond, or a substituted or unsubstituted C1 or C2 alkylene group,
$X^1$ represents a cyano group, or a substituted or unsubstituted thiocarbamoyl group,
$X^2$ represents a hydrogen atom, a halogeno group, a substituted or unsubstituted C1 to C6 alkyl group, a hydroxyl group, or a substituted or unsubstituted C1 to C6 alkoxy group,
$X^3$ represents a halogeno group, a substituted or unsubstituted C1 to C6 alkyl group, a substituted or unsubstituted C1 to C6 alkoxy group, or a cyano group,
A represents a nitrogen atom or $CX^4$,
$X^4$ represents a hydrogen atom, a halogeno group, a substituted or unsubstituted C1 to C6 alkyl group, a substituted or unsubstituted C1 to C6 alkoxy group, or a cyano group,
$R^1$ represents a hydrogen atom, or a substituted or unsubstituted C1 to C6 alkyl group,
$R^{2a}$ represents a substituted or unsubstituted C1 to C6 alkyl group,
$R^{2b}$ represents a hydrogen atom, or a substituted or unsubstituted C1 to C6 alkyl group,
where $R^{2a}$ and $R^{2b}$ may be combined with each other to form a substituted or unsubstituted C2 to C5 alkylene group, and
Y represents a substituted or unsubstituted C1 to C6 alkyl group, a substituted or unsubstituted C6 to C10 aryl group, or a substituted or unsubstituted 5- or 6-membered heteroaryl group.

**2.** A pest control agent comprising, as an active ingredient:
at least one selected from the group consisting of the compound according to Claim 1 and a salt of the compound.

**3.** An insecticidal or acaricidal agent comprising, as an active ingredient:
at least one selected from the group consisting of the compound according to Claim 1 and a salt of the compound.

**4.** An ectoparasite control or repellent agent comprising, as an active ingredient:
at least one selected from the group consisting of the compound according to Claim 1 and a salt of the compound.

**INTERNATIONAL SEARCH REPORT**

International application No.
**PCT/JP2022/019936**

**A. CLASSIFICATION OF SUBJECT MATTER**

***C07D 213/85***(2006.01)i; ***A01N 37/34***(2006.01)i; ***A01N 41/10***(2006.01)i; ***A01N 43/40***(2006.01)i; ***A01N 43/80***(2006.01)i; ***A01N 43/836***(2006.01)i; ***A01N 47/02***(2006.01)i; ***A01P 7/02***(2006.01)i; ***A01P 7/04***(2006.01)i; ***C07C 317/28***(2006.01)i; ***C07C 323/25***(2006.01)i; ***C07D 213/83***(2006.01)i; ***C07D 401/12***(2006.01)i; ***C07D 413/12***(2006.01)i; ***C07D 417/12***(2006.01)i

FI: C07D213/85 CSP; A01N37/34 103; A01N41/10 Z; A01N43/40 101D; A01N43/80 102; A01N43/836; A01N47/02; A01P7/02; A01P7/04; C07C317/28; C07C323/25; C07D213/83; C07D401/12; C07D413/12; C07D417/12

According to International Patent Classification (IPC) or to both national classification and IPC

**B. FIELDS SEARCHED**

Minimum documentation searched (classification system followed by classification symbols)

C07D213/85; A01N37/34; A01N41/10; A01N43/40; A01N43/80; A01N43/836; A01N47/02; A01P7/02; A01P7/04; C07C317/28; C07C323/25; C07D213/83; C07D401/12; C07D413/12; C07D417/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2019-507733 A (DOW AGROSCIENCES LLC) 22 March 2019 (2019-03-22)<br>entire text, all drawings | 1-4 |
| A | JP 2015-502967 A (BASF SE) 29 January 2015 (2015-01-29)<br>entire text, all drawings | 1-4 |
| A | JP 2007-308471 A (NISSAN CHEMICAL INDUSTRIES, LTD.) 29 November 2007 (2007-11-29)<br>entire text, all drawings | 1-4 |
| A | CN 104418800 A (SINOCHEM CORP.) 18 March 2015 (2015-03-18)<br>entire text, all drawings | 1-4 |
| A | CN 105777727 A (SHENYANG SINOCHEM PESTICIDE CHEMICAL RESEARCH AND DEVELOPMENT CO., LTD.) 20 July 2016 (2016-07-20)<br>entire text, all drawings | 1-4 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 June 2022** | **28 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/019936**

| Patent document cited in search report | Publication date (day/month/year) | Patent family member(s) | Publication date (day/month/year) |
|---|---|---|---|
| JP 2019-507733 A | 22 March 2019 | US 2017/0210723 A1<br>entire text, all drawings<br>WO 2017/132014 A1<br>EP 3408262 A1<br>KR 10-2018-0109076 A<br>CN 109071484 A | |
| JP 2015-502967 A | 29 January 2015 | US 2014/0364466 A1<br>entire text, all drawings<br>WO 2013/092943 A1<br>EP 2794601 A1<br>KR 10-2014-0115329 A<br>CN 104169278 A | |
| JP 2007-308471 A | 29 November 2007 | US 2011/0144334 A1<br>entire text, all drawings<br>WO 2007/026965 A1<br>EP 1932836 A1<br>KR 10-2008-0049091 A | |
| CN 104418800 A | 18 March 2015 | WO 2015/032280 A1<br>entire text, all drawings | |
| CN 105777727 A | 20 July 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021082497 A
- WO 2015032280 A